# EUROPEAN PATENT APPLICATION

(11) **EP 2 524 968 A1**
(43) Date of publication of application: **21.11.2012**
(21) Application number: 11166976.8
(22) Date of filing: 20.05.2011
(51) Int. Cl.: C12Q 1/68

(54) **Methods and kits for diagnosing colorectal cancer**

(30) Priority: 19.05.2011 EP 11166817
(71) Applicant: Signature Diagnostics AG, 14473 Potsdam (DE)
(72) Inventor: Adams, Hans-Peter, 14478 Potsdam (DE); Hinzmann, Bernd, 13127 Berlin (DE); Mayr, Tobias, 10435 Berlin (DE); Rosenthal, André, 14194 Ludwigsfelde (DE)
(74) Representative: Hoppe, Georg Johannes

(57) **Abstract**

The invention pertains to a method for early detection and screening of colorectal cancer in human subjects based on RNA isolated from blood obtained from said subject. According to the invention, the abundance of at least 300 RNAs listed in table 1 is measured. Using the invention, an accurate and noninvasive screening and diagnosis tool for colorectal cancer is provided with a sensitivity of at least 70 % and a specificity of 75 % that has high clinical utility and the potential for broad adoption.

## Description

The invention pertains to a method for diagnosing or detecting colorectal cancer in human subjects based on ribonucleic acid (RNA), in particular based on RNA from blood.

### Background

Colorectal cancer (CRC) is the second-leading cause of cancer-related deaths in the United States. Each year, approximately 150,000 people are diagnosed with CRC and almost 60,000 people die from the disease.

CRC arises from the mucosa forming the inner lining of colon and rectum. Like any other mucosa, it needs to be regenerated and proliferates at a high rate (about one third of all fecal matter are mucosa cells), and is thus susceptible to abnormal growth, i.e., neoplasia and/or dysplasia. In fact, abnormal colonic mucosal growth can be detected in about 40 % of all persons over the age of 55 years. The development of neoplasia into cancer is a well-established concept in the biomedical sciences; and is termed adenoma-carcinoma-sequence (ACS).

Pathologists classify abnormal mucosal growth into four categories with increasing severity: 1) Low-grade intraepithelial neoplasia (LIEN) or adenoma, which occurs in more than 30 %; 2) high-grade intraepithelial neoplasia (HIEN) or advanced adenoma, occurring in more than 2 %; 3) carcinoma in situ (CIS or pTis), where the cancerous growth is still confined to the mucosa; and 4) CRC, where the cancerous growth has invaded the submucosa. CRC is diagnosed with an incidence rate of about 1 % in persons over the age of 55 years with an average risk for the disease. The lifetime risk of developing CRC is estimated to be 1 in 18 persons (Cancer Statistics 2009: A Presentation From the American Cancer Society; 2009, American Cancer Society, Inc.).

After primary diagnosis of CRC, the spread/stage of the disease is classified according to the guidelines set forth by the "Union International Contre le Cancer" (UICC). UICC-stage 0 includes CIS only. UICC-stages I and II are comprised of the localized stages, whereas UICC-stage III describes CRC where tumor cells have metastasized into regional lymph nodes. The worst case is UICC-stage IV; it describes CRC which has metastasized into other organ(s), usually liver (∼75 %), peritoneum (∼22 %), and/or lung (∼21 %).

In 2008, the cancer registry in the state of Brandenburg/Germany documented 1591 patients with newly diagnosed CRC and stage information. They were staged into UICC-stage I: 22.6 %, UICC-stage II: 29.2 %, UICC-stage III: 28.9 %, and UICC-stage IV: 19.0 %. Relative five year survival-rates by UICC-stage were: I: 90.5%, II: 78.8%, III: 60.6%, and IV: 9.3%.

The U.S. National Institutes of Health (http://seer.cancer.gov) reported for the period 1999 to 2006 216,332 patients diagnosed with CRC with localized disease (UICC-stage I and II): 39 %, regional disease (UICC stage III): 37 % and distant disease (UICC-stage IV): 19 %. Relative five year survival-rates by stage were: localized (UICC-stages I and II): 90.4 %, regional (UICC stage III): 60.5%, and distant (UICC-stage IV): 11.6 %. However, the statistics of the U.S. National Institutes of Health do not cover the U.S. population, while the data from the cancer registry in the state of Brandenburg/Germany do.

Current technologies to detect mucosal neoplasia (polyps/adenoma) and CRC can be categorized into three classes:
I) *in-vitro* diagnostics (IVDs) - a specimen/sample (blood, stool, or urine) is taken from the test person and analyzed for one or more biomarkers as surrogate markers for colorectal neoplasia/cancer. Exemplary tests include guaic fecal occult blood test (gFOBT) or immunological fecal occult blood test (iFOBT), detection of tumor DNA-chains (deoxyribonucleic acid chains) in stool samples, detection of specific methylated tumor DNA-chains in stool samples, detection of specific free methylated DNA-chains in blood plasma, detection of elevated and/or lowered amounts of specific proteins in blood samples, or detection of elevated and/or lowered amounts of specific RNA-chains in blood samples;
II) **imaging methods without interventional capabilities** such as X-ray, double contrast barium enema (DCBE), video capsule endoscopy, or computed tomographic colonography;
III) **imaging methods with interventional capabilities** such as flexible sigmoidoscopy, colonoscopy, laparoscopy, or open surgery.

To obtain a definitive diagnosis of colorectal neoplasia/cancer, an invasive procedure is typically required. The procedure requires taking a sample of the visibly abnormal tissue growth (neoplasia/cancer) and having a person of skill in the art of pathology examine this sample, who will then decide (diagnose) whether this sample was taken from a neoplasia/cancer or not (Sternberg's Diagnostic Surgical Pathology (5th Edition). Mills SE, Carter D, Greenson JK, Reuter V, Stoler MH. Lippincott Williams & Wilkins (LWW), 2009).

In response to the high incidence and mortality rate of patients with CRC, the American Cancer Society issued the following statement: "There are significant updates to the guidelines for colorectal cancer screening. Two new tests are now recommended as options for colorectal cancer screening. They are stool DNA (sDNA) and computerized tomographic colonography (also known as "virtual colonoscopy"). For the first time, screening tests are grouped into categories based on performance characteristics: those that primarily detect cancer early and those that can also detect precancerous polyps. Tests that primarily detect cancer early are fecal (stool) tests, including guaiac-based and immunochemical-based fecal occult blood tests (gFOBT & FIT), and stool DNA tests (sDNA). Tests that detect both precancerous polyps and cancer include flexible sigmoidoscopy, colonoscopy, the double contrast barium enema, and computerized tomographic colonography (also known as virtual colonoscopy). It is the strong opinion of the expert panel that colon cancer prevention should be the primary goal of colorectal cancer screening. Exams that are designed to detect both early cancer and precancerous polyps should be encouraged if resources are available and patients are willing to undergo an invasive test." (Cancer Statistics 2009: A Presentation From the American Cancer Society; ©2009, American Cancer Society, Inc.). A review of current CRC screening in Europe can be found in: Zavoral M, Suchanek S, Zavada F, Dusek L, Muzik J, Seifert B, Fric P. Colorectal cancer screening in Europe. World J Gastroenterol. 2009 Dec 21; 15: 5907-15.

However, each of the tests for the detection of mucosal neoplasia (polyps/adenomas) and CRC has limitations.

For example, the imaging methods (with or without interventional capabilities) require preparation time for the test subject, specialized equipment, and specialized medical personnel. Therefore, colonoscopy and flexible sigmoidoscopy are used only in wealthy economies such as the U.S., Switzerland, and Germany as primary screening tools for early detection of CRC. Even in the U.K., France, Italy or Spain, IVDs, in most instances gFOBT, are used as a primary screening tool for colorectal cancer. Only patients with a positive IVD test result are referred to colonoscopy.

Recently, a screening program for CRC using gFOBT was initiated in the United Kingdom. All eligible persons were contacted via mail, and a test kit was delivered. Yet, just about 50 % of all contacted people complied. The willingness of patients to undergo gFOBT testing in has dropped from 8.2 million tests in 2001 to 4.6 million tests in 2007 (Projekt wissenschaftliche Begleitung von Früherkennungs-Koloskopien in Deutschland, Berichtszeitraum 2008 - 6. Jahresbericht, im Auftrag des GKV - Spitzenverbands und der Kassenärztlichen Bundesvereinigung Version 1.1, Stand: 19. Februar 2010, Zentralinstitut für die kassenärztliche Versorgung in der Bundesrepublik Deutschland). In the U.S., about 24.01 % of all eligible patients underwent gFOBT-screening in 2000, in 2005 the rate dropped to 17.07 % (U.S. National Cancer Institute, http://progressreport.cancer.gov).

Thus, the clinical utility of all stool-based CRC-screening is limited because individuals in the CRC screening population are simply unwilling to take the test repeatedly, unless they have no other choice.

The U.s. National Institutes of Health reported that compliance with endoscopy (flexible sigmoidoscopy or colonoscopy) is dependent on the education and income of the population; by 2005 37.66 % of persons with less than high school education, 46.27 % of persons with high school education, and 57.52 % of persons with higher than high school education had ever had an endoscopy (not defined to CRC screening purposes).

Colonoscopy is an invasive procedure, which is not only inconvenient but may be associated with health risks. Approximately 3 % of the individuals over 55 years undergoing colonoscopy for screening purposes have heavy breeding incidences. Additionally, in 2 of 1,000 individuals perforation of the colon occurs. Emergency operations must be performed to correct both heavy bleeding and perforation. As a result, 2 of 10,000 individuals who undergo colonoscopy will die from these complications. The relatively high rate of accidents in combination with the time consuming bowel cleaning procedure has led to a low adoption of colonoscopy as a screening tool even in those countries where colonoscopy is paid by the health insurances.

Thus, the overall clinical utility of all endoscopy-based CRC-screening is also limited, because it is only offered in a few counties, a high percentage of the CRC screening population is unwilling to take the test, and because of the complications associated with the test.

Therefore, the clinical utility of a test for detection of colorectal neoplasia depends not only on its performance characteristics, *i.e.*, sensitivity and specificity, but on acceptance by the patients, the medical community, and, of course, the private or public health care system that has to pay for the test.

A blood test would have the highest chance of acceptance by patients, at least in Europe, the U.S., and Japan, in terms of the medical community, a blood test would also have the highest chance of acceptance, in particular if sensitivity and specificity are convincingly high, if there is no need for preparation time, if the blood need not be processed immediately but can be shipped to a central laboratory, if the test is accepted by local regulatory authorities, and if the test is commercially available. A high level of acceptance of a test can only be achieved if the test is endorsed by CRC screening guidelines and by the general health care system.

Although blood-based colon cancer screening has been attempted, each previously reported test is inherently limited in its respective specificity and sensitivity.

For example, Han et al., (Clin Cancer Res 2008; 14, 455-460; also: WO 2007/048074 A1) reported the discovery and validation of a five-gene expression (messenger RNA) signature for detection of colorectal carcinoma. Basically, the 37 candidate genes for the signature were selected from microarray data of 16 CRC cases and 15 controls. These 37 candidate genes were evaluated on a second set of 115 samples (58 CRC, 57 controls) using quantitative real-time PCR, validating 17 genes as differentially expressed. A further gene selection step using the PCR-results revealed the 5 gene signature, which was validated on a third set of 102 samples. The predictive power of these five genes, which was evaluated using a fourth set of 92 samples, correctly identifying 88 % (30 of 34) of CRC samples and only 64 % (27 of 42) of non-CRC samples. The intermediate zone contained 16 samples. The performance parameters are compiled into table A.

**Table A: Estimates and Exact Confidence Limits of GeneNews ColonSentry™ Test**

| Performance Parameter | N_{C} | N | Estimate | Exact Two-Sided 95 % CI-Limits | |
|---|---|---|---|---|---|
| | | | | Lower | Upper |
| Sensitivity | 30 | 34 | 0.88 | 0.725 | 0.967 |
| Specificity | 27 | 42 | 0.64 | 0.480 | 0.784 |
| Positive Predictive Value | 30 | 45 | 0.67 | 0.510 | 0.800 |
| Negative Predictive Value | 27 | 31 | 0.87 | 0.702 | 0.964 |
| Correct Classification Rate | 57 | 76 | 0.75 | 0.637 | 0.842 |

| | | | | | |
|---|---|---|---|---|---|
| N_{c} = Number of correctly classified cases; CI = Confidence interval; Exact confidence limits were computed using the proc FREQ of the statistics program SAS. | | | | | |

Provided that patients of the last validation set were a random selection of the screening population, applying the performance on a hypothetical set of 10,000 patients with an incidence of one percent and computing the performance parameters of this test yields the results shown in table B.

**Table B: Estimates and Exact Confidence Limits of GeneNews ColonSentry™ Test applied to a Hypothetical Set of 10000 Persons**

| Performance Parameter | N_{C} | N | Estimate | Exact Two-Sided 95 % CI-Limits | |
|---|---|---|---|---|---|
| | | | | Lower | Upper |
| Sensitivity | 73 | 100 | 0.73 | 0.632 | 0.814 |
| Specificity | 6979 | 9900 | 0.70 | 0.696 | 0.714 |
| Positive Predictive Value | 73 | 2994 | 0.02 | 0.019 | 0.031 |
| Negative Predictive Value | 6979 | 7006 | 1.00 | 0.994 | 0.997 |
| Correct Classification Rate | 7052 | 10000 | 0.71 | 0.696 | 0.714 |

| | | | | | |
|---|---|---|---|---|---|
| Nc = Number of correctly classified cases; CI = Confidence interval; Exact confidence limits were computed using the proc FREQ of the statistics program SAS. | | | | | |

However, based on the data provided by Han, 1,739 of 10,000 patients would have an "intermediate result". In clinical practice, this would mean that these 1,739 patients would have to undergo colonoscopy to clarify their state. However, in this computation, these 1,739 patients were regarded as having been predicted as low risk. The main disadvantage of the ColonSentry test is its relatively low sensitivity of 73 % and its low specificity of 70 %. Applied to a screening population of 1 million individuals this means that 2,700 individuals with undetected CRC will not be detected by the test. In addition, 300,000 individuals (30 %) are diagnosed as false positive, which need to be followed up by colonoscopy. The combination of a relatively high false negative rate of 27 % with a high false positive rate of 30 % reduces the clinical utility of this test and impedes acceptance by the medical community and the screening population itself.

Epigenomics AG, Germany, has a CE-marked test, Epi proColon^{®}, in the market that measures the methylation status of the Septin-9 gene and is based on detection of free somatic tumor DNA in blood serum.

**Table C: Estimates and Exact Confidence Limits of Epi proColon^{®} Test**

| Performance Parameter | N_{c} | N | Estimate | Exact Two-Sided 95% CI-Limits | |
|---|---|---|---|---|---|
| | | | | Lower | Upper |
| Sensitivity | 69 | 103 | 0.67 | 0.570 | 0.759 |
| Specificity | 135 | 154 | 0.88 | 0.814 | 0.924 |
| Positive Predictive Value | 69 | 88 | 0.78 | 0.684 | 0.865 |
| Negative Predictive Value | 135 | 169 | 0.80 | 0.730 | 0.856 |
| Correct Classification Rate | 204 | 257 | 0.79 | 0.739 | 0.842 |

| | | | | | |
|---|---|---|---|---|---|
| N_{c} = Number of correctly classified cases; CI = Confidence interval; Exact confidence limits were computed using the proc FREQ of the statistics program SAS. | | | | | |

The product performance figures of Epi proColon^{®} displayed in table C are cited from the companies' website (www.epigenomics.com). Table D shows the figures when the performance of the test is applied to a hypothetical screening population. Though the Epi proColon^{®} test performs better than GeneNews' test in some performance parameters its overall sensitivity for all four stages of CRC is only 67 %. This means that if 10,000 individuals are screened and the prevalence of CRC in the screening population is approximately 1 %, so that 33 individuals with CRC will be missed by the test. Applied to a screening population of 1 million individuals (3.7 % of the German screening population or 1.3% of the US screening population) 3,300 individuals with CRC will not be detected by the test. This high false negative rate limits significantly the clinical utility of the Epi proColon^{®} test. The false negative rate of patients with early stage CRC (UICC I and II) that will be missed is even higher.

**Table D: Estimates and Exact Confidence Limits of Epi proColon^{®} Test applied to a Hypothetical Set of 10,000 Persons**

| Performance Parameter | N_{c} | N | Estimate | Exact Two-Sided 95% CI-Limits | |
|---|---|---|---|---|---|
| | | | | Lower | Upper |
| Sensitivity | 67 | 100 | 0.67 | 0.569 | 0.761 |
| Specificity | 8679 | 9900 | 0.88 | 0.870 | 0.883 |
| Positive Predictive Value | 67 | 1288 | 0.05 | 0.041 | 0.066 |
| Negative Predictive Value | 8679 | 8712 | 1.00 | 0.995 | 0.997 |
| Correct Classification Rate | 8746 | 10000 | 0.87 | 0.868 | 0.881 |

| | | | | | |
|---|---|---|---|---|---|
| N_{c} = Number of correctly classified cases; CI = Confidence interval; Exact confidence limits were computed using the proc FREQ of the statistics program SAS. | | | | | |

Another blood-based test developed by OncoMethylome Science (Liege, Belgium) measures the methylation status of two Genes FOXE1 and SYNE1. The sensitivity of this two-marker test for all four stages of CRC is 56 %, while the specificity is 91 % (ESMO meeting, Berlin, Germany, September 2009)

All three blood tests have a significant false negative rate and do not detect a significant number of patients with CRC. The ColonSentry test has a low specificity of 70 % and burdens colonoscopy facilities with a high number of false-positive test results.

Thus, there is a clear clinical need for an improved blood-based test for screening, detecting, or diagnosing colorectal cancer with high sensitivity and high specificity, which is minimally invasive so as to permit more widespread testing of the population to indicate the presence of colorectal cancer with high accuracy and therefore with a high clinical utility, and to ensure greater adherence to recommended protocols. Further, the identification of biomarkers, such as RNAs for use in such a minimally-invasive test would fulfill a longstanding need in the art.

### Brief description of the invention

The present invention provides methods and kits for diagnosing, detecting, and screening for colorectal cancer. Particularly, the invention provides for preparing RNA expression profiles of patient blood samples, the RNA expression profiles being indicative of the presence or absence of colorectal cancer. The invention further provides for evaluating the patient RNA expression profiles for the presence or absence of one or more RNA expression signatures that are indicative of colorectal cancer.

The inventors have surprisingly found that a sensitivity of at least 80 %, and a specificity of at least 90 % is reached if and only if at least 300 RNAs are measured that are chosen from the RNAs listed in table 1. In other words, measuring 300 RNAs is necessary and sufficient for the detection of CRC in a human subject based on RNA from a blood sample obtained from said subject by measuring the abundance of at least 300 RNAs in the sample, that are chosen from the RNAs listed in table 1, and concluding based on the measured abundance whether the subject has CRC or not.

In one aspect, the invention provides a method for preparing RNA expression profiles that are indicative of the presence or absence of colorectal cancer. The RNA expression profiles are prepared from patient blood samples. The number of transcripts in the RNA expression profile may be selected so as to offer a convenient and cost effective means for screening samples for the presence or absence of colorectal cancer with high sensitivity and high specificity. Generally, the RNA expression profile includes the expression level or "abundance" of from 300 to about 2500 transcripts. In certain embodiments, the expression profile includes the RNA levels of 2500 transcripts or less, 2000 transcripts or less, 1500 transcripts or less, 1000 transcripts or less.

In such embodiments, the profile may contain the expression level of at least 300 RNAs that are indicative of the presence or absence of colorectal cancer, and specifically, as selected from table 1, or may contain the expression level of at least 600, at least 1000 RNAs selected from table 1. Where larger profiles are desired, the profile may contain the expression level or abundance of at least about 1200 RNAs that are indicative of the presence or absence of colorectal cancer, and such RNAs may be selected from table 1. The identities and/or combinations of genes and/or transcripts that make up or are included in expression profiles are disclosed in tables 1 to 4. In particular embodiments, the genes or transcripts include those listed in tables 2 to 4.

Such RNA expression profiles in accordance with this aspect may be evaluated for the presence or absence of an RNA expression signature indicative of colorectal cancer. Generally, the sequential addition of transcripts from table 1 to the expression profile provides for higher sensitivity and/or specificity for the detection of colorectal cancer. For example, the sensitivity of the methods provided herein may be at least 70 %, at least 75 %, or at least 80 %, or at least 85 %, or at least 90 %. The specificity of the method may be at least 75 %, at least 80 %, at least 85 %, or at least 90 %.

In a second aspect, the invention provides a method for detecting, diagnosing, or screening for colorectal cancer. In this aspect the method comprises preparing an RNA expression profile by measuring the abundance of at least 300, at least 600, or at least 1000 RNAs in a patient blood sample, where the abundance of such RNAs are indicative of the presence or absence of colorectal cancer. The RNAs may be selected from the RNAs listed in table 1, and exemplary sets of such RNAs are disclosed in tables 2 to 4. The method further comprises evaluating the profile for the presence or absence of an RNA expression signature indicative of colorectal cancer, to thereby conclude whether the patient has or does not have colorectal cancer. The method generally provides a sensitivity for the detection of colorectal cancer of at least about 70 %, while providing a specificity of at least about 75 %.

In various embodiments, the method comprises determining the abundance of at least 300 RNAs, at least 600 RNAs, at least 1000 RNAs chosen from the RNAs listed in table 1, and as exemplified in tables 2 to 4, or all 1200 RNAs of table 1, and classifying the sample as being indicative of colorectal cancer, or not being indicative of colorectal cancer.

In other aspects, the invention provides kits and custom arrays for preparing the gene expression profiles, and for determining the presence or absence of colorectal cancer.

### Detailed description of the invention

The invention provides methods and kits for screening, diagnosing, and detecting colorectal cancer in human patients (subjects). "Colorectal cancer" (CRC) refers to both colorectal adenoma and colorectal carcinoma.

A colorectal adenoma is characterized by atypical growth of epithelial cells in mucosal tissue, i.e. neoplasia. Hypercellularity with enlarged, hyperchromatic nuclei, varying degrees of nuclear stratification, nuclear polymorphisms, and loss of polarity are the histologically defining features. In colorectal adenoma, this abnormal growth is confined to the mucosa; a synonym of adenoma is intraepithelial neoplasia (IEN). If this atypical growth of epithelial cells extends/invades through the muscularis mucosae, the muscle layer under the mucosa, with destruction of the usual anatomical wall, the pathologist terms this atypical growth a colorectal carcinoma.

The distinction between high- (HIEN) and low-grade (LIEN) intraepithelial neoplasia refers to the extent of the defining features.

A patient with CRC is traditionally defined as having undergone surgery/resection of colon and/or rectum for CRC and whose resection specimen has undergone examination by a board certified pathologist, who has diagnosed a colorectal carcinoma as defined above. A patient with CRC may have undergone complete colonoscopy of colon and rectum during which the examinating physician has taken a sample of suspect tissue, which in turn has undergone examination by a board-certified pathologist, who has diagnosed a colorectal carcinoma as defined above. A synonym for a patient with CRC is "CRC-case" or simply "case."

A patient without CRC is traditionally a person that has undergone complete colonoscopy during which the examining physician, an endoscopist, has noted no abnormal tissue growth. A synonym for a patient without CRC is "non-CRC-case" or "control." This however does not exclude that this person has any other carcinoma.

A patient with HIEN is traditionally a person that has undergone surgery/resection of colon and/or rectum for suspected CRC and whose resection specimen has undergone examination by a board certified pathologist, who has diagnosed a high-grade intraepithelial neoplasia as defined above. Alternatively, a patient with HIEN may be a person that has undergone complete colonoscopy of colon and rectum during which the examinating physician has taken a sample of suspect tissue, which in turn has undergone examination by a board certified pathologist, who has diagnosed a high-grade intraepithelial neoplasia as defined above. A synonym for a person with HIEN is "HIEN-case" or "HIEN."

A patient with LIEN is traditionally a person that has undergone surgery/resection of colon and/or rectum for suspected CRC and whose resection specimen has undergone examination by a board certified pathologist, who has diagnosed a high-grade intraepithelial neoplasia. Alternatively, a patient with LIEN is a person that has undergone complete colonoscopy of colon and rectum during which the examinating physician has taken a sample of suspect tissue, which in turn has undergone examination by a board certified pathologist, who has diagnosed a low-grade intraepithelial neoplasia as defined above. A synonym for a person with LIEN "is LIEN-case" or "LIEN."

As disclosed herein, the present invention provides methods and kits for screening patient samples for those that are positive for CRC, that is, in the absence of colonoscopy and/or surgery or resection of colon or rectum with pathologist's examination.

The invention relates to the determination of the abundance of RNAs to detect a colorectal cancer in a human subject, wherein the determination of the abundance is based on RNA obtained (or isolated) from whole blood of the subject or from blood cells of the subject. For example, the sample may be obtained using PAXgene (QIAGEN) or an equivalent RNA isolation system. The measurement of the abundance of RNAs in the sample is preferably performed together, i.e. sequentially or preferably simultaneously. The blood sample preferably does not contain cancer cells.

In various aspects, the invention involves preparing an RNA expression profile from a patient sample. The method may comprise isolating RNA from whole blood, and detecting the abundance or relative abundance of selected transcripts. As used herein, the terms RNA "abundance" and RNA "expression" are used interchangeably. The "RNAs" may be defined by reference to an expressed gene, or by reference to a transcript, or by reference to a particular oligonucleotide probe for detecting the RNA (or cDNA derived therefrom), each of which is listed in table 1 for 1200 RNAs that are indicative of the presence or absence of colorectal cancer. Specifically, table 1 lists such RNAs by SEQ. ID. NO., probe ID, gene symbol, and Primary Transcript ID, and such nucleotide sequences are publicly accessible. Table 1A gives the fold change in expression levels for the genes/transcripts listed in table 1, when measured in 64 control cases, and 52 CRC positive cases.

The number of transcripts in the RNA expression profile may be selected so as to offer a convenient and cost effective means for screening samples for the presence or absence of colorectal cancer with high sensitivity and high specificity. For example, the RNA expression profile may include the expression level or "abundance" of from 300 to about 2500 transcripts. In certain embodiments, the expression profile includes the RNA levels of 2500 transcripts or less, 2000 transcripts or less, 1500 transcripts or less, or 1000 transcripts or less. Such profiles may be prepared, for example, using custom microarrays or multiplex gene expression assays as described in detail herein.

In such embodiments, the profile may contain the expression level of at least 600 RNAs that are indicative of the presence or absence of colorectal cancer, and specifically, as selected from table 1. Where larger profiles are desired, the profile may contain the expression level or abundance of at least 1000 RNAs that are indicative of the presence or absence of colorectal cancer, and such RNAs may be selected from table 1. Such RNAs may be defined by gene, or by transcript ID, or by probe ID, as set forth in table 1.

The identities of genes and/or transcripts that make up, or are included in exemplary expression profiles are disclosed in tables 1 to 4. As shown herein, profiles selected from the RNAs of table 1 support the detection of colorectal cancer with high sensitivity and high specificity. Exemplary selection of RNAs for the RNA expression profile are shown in tables 2 to 4.

In various embodiments, the abundance of at least 300 or at least 600 distinct RNAs are measured, in order to arrive at a reliable diagnosis of colon cancer. The set of RNAs may comprise, consist essentially of, or consist of, a set or subset of RNAs exemplified in any one of tables 1 to 4. The term "consists essentially of" in this context allows for the expression level of additional transcripts to be determined that are not differentially expressed in colorectal cancer subjects, and which may therefore be used as positive or negative expression level controls or for normalization of expression levels between samples.

Such RNA expression profiles may be evaluated for the presence or absence of an RNA expression signature indicative of colorectal cancer. Generally, the sequential addition of transcripts from table 1 to the expression profile provides for higher sensitivity and/or specificity for the detection of colorectal cancer. For example, the sensitivity of the methods provided herein may be at least 70 %, at least 75 %, or at least 80 %, or at least 85 %, or at least 90 %. The specificity of the method may be at least 75 %, at least 80 %, at least 85 %, or at least 90 %.

The present invention provides an in-vitro diagnostic test system (IVD) that is trained (as described further below) for the detection of a colorectal cancer. For example, in order to determine whether a patient has colorectal cancer, reference RNA abundance values for colon cancer positive and negative samples are determined. The RNAs can be quantitatively measured on an adequate set of training samples comprising cases and controls, and with adequate clinical information on carcinoma status, applying adequate quality control measures, and on an adequate set of test samples, for which the detection is yet to be made. With such quantitative values for the RNAs and the clinical data for the training samples, a classifier can be trained and applied to the test samples to calculate the probability of the presence or non-presence of the colorectal carcinoma.

Various classification schemes are known for classifying samples between two or more classes or groups, and these include, without limitation: Naive Bayes, Support Vector Machines, Nearest Neighbors, Decision Trees, Logistics, Articifial Neural Networks, and Rule-based schemes. In addition, the predictions from multiple models can be combined to generate an overall prediction. Thus, a classification algorithm or "class predictor" may be constructed to classify samples. The process for preparing a suitable class predictor is reviewed in R. Simon, Diagnostic and prognostic prediction using gene expression profiles in high-dimensional microarray data, British Journal of Cancer (2003) 89, 1599-1604, which review is hereby incorporated by reference.

In this context, the invention teaches an in-vitro diagnostic test system (IVD) that is trained in the detection of a colorectal cancer referred to above, comprising at least 300 RNAs, which can be quantitatively measured on an adequate set of training samples comprising cases and controls, with adequate clinical information on carcinoma status, applying adequate quality control measures, and on an adequate set of test samples, for which the detection yet has to be made. Given the quantitative values for the RNAs and the clinical data for the training samples, a classifier can be trained and applied to the test samples to calculate the probability of the presence or absence of the colorectal carcinoma.

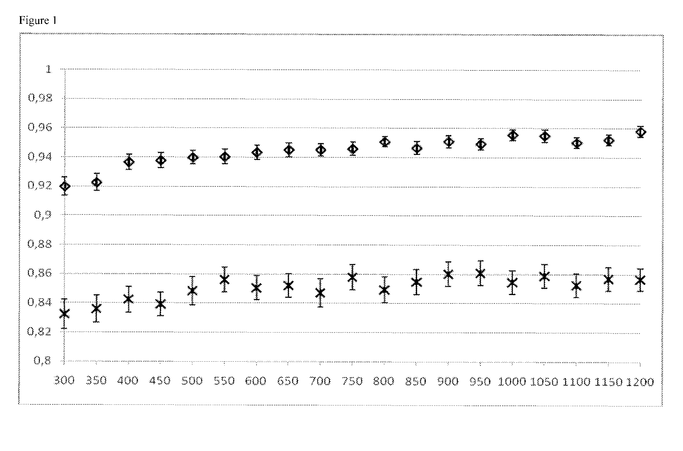

The present invention provides methods for detecting, diagnosing, or screening for colorectal cancer in a human subject with a sensitivity and specificity not previously described for a blood-based method (see Fig. 1). Specifically, the sensitivity of the methods provided herein are at least 70 %, at least 75 %, at least 80 %, at least 85 %, or at least 90 %. The specificity of the methods is at least 75 %, at least 80 %, at least 85 %, or at least 90 %, for example, when determined with samples of at least 119 patients with CRC and adequate samples (e.g., at least 109) of normal individuals without CRC are tested.

Without wishing to be bound by any particular theory, the above finding may be due to the fact that an organism such as a human systemically reacts to the development of a colorectal tumor by altering the expression levels of genes in different pathways. The formation of cancerous tumor cells from a nonmalignant adenoma or nonmalignant polyps, the formation of high-grade intraepithelial neoplasias and the further growth and development of cancer of different stages may trigger differential expression of genes in white blood cells that are involved in both adaptive and innate immune responses, for example wound healing, inflammatory response and antibody production pathways. Although the change in expression (abundance) might be small for each gene in a particular signature, measuring a set of at least 300 genes, preferably even larger numbers such as 1000, 2000 or even more RNAs, for example at least 1000, or at least 2000 RNAs at the same time allows for the detection of colorectal cancer in a human with high sensitivity and high specificity.

In this context, an RNA obtained from a subject's blood sample, i.e. an RNA biomarker, is an RNA molecule with a particular base sequence whose presence within a blood sample from a human subject can be quantitatively measured. The measurement can be based on a part of the RNA molecule, namely a part of the RNA molecule that has a certain base sequence, which allows for its detection and thereby allows for the measurement of its abundance in a sample. The measurement can be by methods known in the art, for example analysis on a solid phase device, or in solution (for example, by RT-PCR). Probes for the particular RNAs can either be bought commercially, or designed based on the respective RNA sequence.

In the method of the invention, the abundance of several RNA molecules (e.g. mRNA or prespliced RNA, intron-lariat RNA, micro RNA, small nuclear RNA, or fragments thereof) is determined in a relative or an absolute manner, wherein an absolute measurement of RNA abundance is preferred. The RNA abundance is, if applicable, compared with that of other individuals, or with multivariate quantitative thresholds.

The determination of the abundance of the RNAs described herein is performed from blood samples using quantitative methods. In particular, RNA is isolated from a blood sample obtained from a human subject that is to undergo CRC testing. Although the examples described herein use microarray-based methods, the invention is not limited thereto. For example, RNA abundance can be measured by in situ hybridization, amplification assays such as the polymerase chain reaction (PCR), sequencing, or microarray-based methods. Other methods that can be used include polymerase-based assays, such as RT-PCR (e.g., TAQMAN), hybridization-based assays, such as DNA microarray analysis, as well as direct mRNA capture with branched DNA (QUANTIGENE) or HYBRID CAPTURE (DIGENE).

In certain embodiments, the invention employs a microarray. A "microarray" includes a specific set of probes, such as oligonucleotides and/or cDNAs (*e.g.*, expressed sequence tags, "ESTs") corresponding in whole or in part, and/or continuously or discontinuously, to regions of RNAs that can be extracted from a blood sample of a human subject. The probes are bound to a solid support. The support may be selected from beads (magnetic, paramagnetic, etc.), glass slides, and silicon wafers. The probes can correspond in sequence to the RNAs of the invention such that hybridization between the RNA from the subject sample (or cDNA derived therefrom) and the probe occurs. In the microarray embodiments, the sample RNA can optionally be amplified before hybridization to the microarray. Prior to hybridization, the sample RNA is fluorescently labeled. Upon hybridization to the array and excitation at the appropriate wavelength, fluorescence emission is quantified. Fluorescence emission for each particular RNA is directly correlated with the amount of the particular RNA in the sample. The signal can be detected and together with its location on the support can be used to determine which probe hybridized with RNA from the subject's blood sample.

Accordingly, in certain aspects, the invention is directed to a kit or microarray for detecting the level of expression or abundance of RNAs in the subject's blood sample, where this "profile" allows for the conclusion of whether the subject has colorectal cancer or not (at a level of accuracy described herein). In another aspect, the invention relates to a probe set that allows for the detection of the RNAs associated with CRC. If these particular RNAs are present in a sample, they (or corresponding cDNA) will hybridize with their respective probe (i.e, a complementary nucleic acid sequence), which will yield a detectable signal. Probes are designed to minimize cross reactivity and false positives. In one embodiment, the probes used are given e.g. in table 1 as so-called Agilent probeset ID numbers. An Agilent probeset ID number is an identifier that refers to a set of probes selected to represent expressed sequences on an array. An Agilent probe set ID number identifies each probe present on the array, as known to a person of skill in the art. From the sequence defined by an Agilent probeset ID number, the sequence of a nucleic acid, such as an RNA or a molecule derived therefrom, hybridizing with the probe can be deduced.

Thus, the invention in certain aspects provides a microarray, which generally comprises a solid support and a set of oligonucleotide probes. The set of probes generally contains from 300 to about 3,000 probes, including at least 300 probes selected from table 1. In certain embodiments, the set contains 2000 probes or less, or 1000 probes or less. The set of probes may comprise, or consist essentially of, probes listed in table 1, or subsets thereof. Alternatively, the set of probes includes probes that hybridize to a combination of RNAs exemplified in any one of table 2, table 3, or table 4. The microarray may comprise, e.g., about 100.000 probes, some of which may be probes for providing reference data. For example, the microarray may comprise about 10,000 to 100,000 probes providing reference data, together with the probes of table 1.

The conclusion whether the subject has colorectal cancer or not is preferably reached on the basis of a classification algorithm, which can be developed using e.g. a random forest method, a support vector machine (SVM), or a K-nearest neighbor method (K-NN), such as a 3-nearest neighbor method (3-NN), as known in the art.

From the cross-classification of the true disease state (Positive = patient with CRC and Negative = patient without CRC) as determined by a physician and the test result as determined by the classification algorithm, the following measures for binary tests can be derived (Sullivan MS. The Statistical Evaluation of Medical Tests for Classification and Prediction. Oxford University Press, 2003), see table E. An example is given in table F.

**Table E: Cross-Classification of True Disease State by Test Result**

| Test Result | True Disease State | | Total |
|---|---|---|---|
| | Negative | Positive | |
| Negative | n₁₁ | n₁₂ | n_{1Σ} |
| Positive | n₂₁ | n₂₂ | n_{2Σ} |
| Total | n_{Σ1} | n_{Σ2} | n_{ΣΣ} |

**Table F: Example of a Cross-Classification of True Disease State by Test Result**

| Test Result | True Disease State | | Total |
|---|---|---|---|
| | Negative | Positive | |
| Negative | 30 | 10 | 40 |
| Positive | 20 | 70 | 90 |
| Total | 50 | 80 | 130 |

"Sensitivity" (S⁺ or true positive fraction (TPF)) refers to the count of positive test results among all true positive disease states divided by the count of all true positive disease states; in terms of table E this reads: S⁺ = n₂₂ / n_{Σ2}; the result form table F would read: S⁺ = 70 / 80 = 0.875. "Specificity" (S- or true negative fraction (TNF)) refers to the count of negative test results among all true negative disease states divided by the count of all true negative disease states; in terms of table E this reads: S⁺ = n₁₁ / n_{Σ1}; the result form table F would read: S- = 30 / 50 = 0.6. "Correct Classification Rate" (CCR or true fraction (TF)) refers to the sum of the count of positive test results among all true positive disease states and count of negative test results among all true negative disease states divided by all the sum of all cases; in terms of table E this reads: CCR = (n₁₁ +n₂₂) / n_{ΣΣ}; the result form table F would read: CCR = (30 + 70)/130≈ 0.769230769. The measures S⁺, S⁻, and CCR address the question: To what degree does the test reflect the true disease state?

"Positive Predictive Value" (PV⁺ or PPV) refers to the count of true positive disease states among all positive test results dived by the count of all positive test results; in terms of table E this reads: PV⁺ = n₂₂ / n_{2Σ}; the result form table F would read: PV⁺ = 70 / 90≈ 0.777777778. "Negative Predictive Value" (PV⁻ or NPV) refers to the count of true negative disease states among all negative test results dived by the count of all negative test results; in terms of table E this reads: PV⁻ = n₁₁ / n_{1ΣΣ}; the result form table F would read: PV⁻ = 30 / 40 = 0.75. The predictive values address the question: How likely is the disease given the test results?

Exact or asymptotic confidence limits (CI) for these rates or fractions can be computed using the commercially available software package SAS (SAS Institute Inc., Cary, NC, USA; www.sas.com) or the publicly available software package R (www.r-project.org) (for literature reference see: Agresti A, Caffo B. Simple and effective confidence intervals for proportions and differences of proportions from adding two successes and two failures. The American Statistician: 54: 280-288, 2000).

The preferred RNA molecules that can be used in combinations described herein for diagnosing and detecting colorectal cancer in a subject according to the invention can be found in table 1. The inventors have shown that the selection of at least 300 or more RNAs of the markers listed in table 1 can be used to diagnose or detect colorectal cancer in a subject using a blood sample from that subject. The RNA molecules that can be used for detecting, screening and diagnosing colorectal cancer are selected from the RNAs provided in table 2, 3, or 4.

Specifically, the method of the invention comprises at least the following steps: measuring the abundance of at least 300 RNAs (preferably 600 RNAs) in the sample, that are chosen from the RNAs listed in table 1, and concluding, based on the measured abundance, whether the subject has colorectal cancer or not. Measuring the abundance of RNAs may comprise isolating RNA from blood samples as described, and hybridizing the RNA or cDNA prepared therefrom to a microarray. Alternatively, other methods for determining RNA levels may be employed.

Examples for sets of 300 RNAs that are measured together, i.e. sequentially or preferably simultaneously, are shown in table 2. The sets of 300 RNAs of table 2 are defined by a common threshold of sensitivity of at least 80 % and specificity of at least 85 %.

In a preferred embodiment of the invention as mentioned herein, the abundance of at least 600 RNAs in the sample is measured, wherein the 600 RNAs are chosen from the RNAs listed in table 1. Examples for sets of 600 RNAs that can be measured together, i.e. sequentially or preferably simultaneously, to detect colorectal cancer in a human subject are shown in table 3. The sets of 600 RNAs of table 3 are defined by a common threshold of sensitivity of at least 80 % and specificity of at least 90 %.

Similarly, the abundance of at least 300 RNAs (preferably up to 599 RNAs), of at least 600 RNAs (preferably up to 999 RNAs), of at least 1000 RNAs (preferably up to 1199 RNAs), of at least 1200 RNAs that are chosen from the RNAs listed in table 1 can be measured in the method of the invention.

Tables 2 through 4 show examples of combinations from table 1, varying in size from 250 to 800 sequences. Examples (signatures) 1, 2, and 3 each consist of 300 sequences. Examples (signatures) 4, 5, and 6 each consist of 600 sequences. Examples (signatures) 7, 8, and 9 each consist of 1000 sequences.

The performance of the examples 1 to 9 was evaluated by applying them to the discovery set, split randomly into a learning and a test set, 1000 times. Each time, a random 85 % of the discovery set were used to train a classifier based on the expression profile of the example sequences. This classifier was then applied to the remaining 15 % as a test set. The results across all repetitions were averaged and are given in the following table.

| | Example 1 | Example 2 | Example 3 |
|---|---|---|---|
| Sensitivity | 86.3% | 82.0% | 82.8% |
| Specificity | 93.0% | 91.5% | 86.9% |

| | Example 4 | Example 5 | Example 6 |
|---|---|---|---|
| Sensitivity | 84.4% | 88.1% | 84.2% |
| Specificity | 93.3% | 94.7% | 95.5% |

| | Example 7 | Example 8 | Example 9 |
|---|---|---|---|
| Sensitivity | 84.7% | 84.3% | 84.1% |
| Specificity | 95.3% | 95.4% | 95.4% |

Examples 1, 2, 4, 5, 7, and 8 were chosen based on their performance in this retrospective evaluation. Examples 3, 6, and 9 were chosen during the original discovery as consensus over all outer loops. In that procedure, a prospective estimate for sequences of length 300 was observed of 74.5 % sensitivity and 79.0 % specificity; for length 600: 75.2 % sensitivity and 82.0 % specificity; for length 1000: 76.2 % sensitivity 83.4 % specificity.

When all 1200 sequences are evaluated in this retrospective fashion, a sensitivity of 85.3 % and a specificity of 94.9 % is seen. In the discovery procedure, prospective estimates of 76.4 % sensitivity and 84.2 % specificity were seen.

When the wording "at least a number of RNAs" is used, this refers to a minimum number of RNAs that are measured. It is possible to use up to 10,000 or 20,000 genes in the invention, a fraction of which can be RNAs listed in table 1. In preferred embodiments of the invention, abundance of up to 5,000, 2,500, 2,000, 1,000 RNAs of randomly chosen RNAs that are not listed in table 1 is measured in addition to RNAs of table 1 (or subsets thereof).

In a preferred embodiment, only RNAs that are mentioned in table 1 are measured.

The expression profile or abundance of RNA markers for colorectal cancer, for example the at least 300 RNAs described above, (or more RNAs as disclosed above and herein), is determined preferably by measuring the quantity of the transcribed RNA of the marker gene. This quantity of the mRNA of the marker gene can be determined for example through chip technology (microarray), (RT-) PCR (for example also on fixated material), Northern hybridization, dot-blotting, sequencing, or in situ hybridization.

The microarray technology, which is most preferred, allows for the simultaneous measurement of RNA abundance of up to many thousand RNAs and is therefore an important tool for determining differential expression (or differences in RNA abundance), in particular between two biological samples or groups of biological samples. In order to apply the microarray technology, the RNAs of the sample need to be amplified and labeled and the hybridization and detection procedure can be performed as known to a person of skill in the art.

As will be understood by those of ordinary skill in the art, the analysis can also be performed through single reverse transcriptase-PCR, competitive PCR, real time PCR, differential display RT-PCR, Northern blot analysis, sequencing, and other related methods. In general, the larger the number of markers is that are to be measured, the more preferred is the use of the microarray technology. However, multiplex PCR, for example, real time multiplex PCR is known in the art and is amenable for use with the present invention, in order to detect the presence of 2 or more genes or RNA simultaneously.

The RNA whose abundance is measured in the method of the invention can be mRNA, cDNA, unspliced RNA, or its fragments. Measurements can be performed using the complementary DNA (cDNA) or complementary RNA (cRNA), which is produced on the basis of the RNA to be analyzed, e.g. using microarrays. A great number of different arrays as well as their manufacture are known to a person of skill in the art and are described for example in the U.S. Patent Nos. 5,445,934; 5,532,128; 5,556,752; 5,242,974; 5,384,261; 5,405,783; 5,412,087; 5,424,186; 5,429,807; 5,436,327; 5,472,672; 5,527,681; 5,529,756; 5,545,331; 5,554,501; 5,561,071; 5,571,639; 5,593,839; 5,599,695; 5,624,711; 5,658,734; and 5,700,637, which are hereby all incorporated by reference in their entirety.

Preferably the decision whether the subject has CRC comprises the step of training a classification algorithm on an adequate training set of cases and controls and applying it to RNA abundance data that was experimentally determined based on the blood sample from the human subject to be diagnosed. The classification method can be a random forest method, a support vector machine (SVM), or a K-nearest neighbor method (K-NN), such as 3-NN.

For the development of a model that allows for the classification for a given set of biomarkers, such as RNAs, methods generally known to a person of skill in the art are sufficient, i.e., new algorithms need not be developed.

The major steps of such a model are:
1) condensation of the raw measurement data (for example combining probes of a microarray to probeset data, and/or normalizing measurement data against common controls);
2) training and applying a classifier (i.e. a mathematical model that generalizes properties of the different classes (carcinoma vs. healthy individual) from the training data and applies them to the test data resulting in a classification for each test sample.

For example, the raw data from microarray hybridizations can be.quantile-normalized after forming the median of multiple identical probes and subtracting the local background (see Bolstad,Irizarry,Anstrand, Speed: A comparison of normalization methods for high density oligonucleotide array data based on variance and bias. Bioinformatics, 19 (2):185-193). Alternative methods for condensation can be used, such as: FARMS as shown by Hochreiter (2006, Bioinformatics 22(8): 943-9), RMA, GC-RMA, see Irizarry et al (2003). Exploration, Normalization, and Summaries of High Density Oligonucleotide Array Probe Level Data. Biostatistics. 4, 249-264.). Similar to condensation, classification of the test data set through a support-vector-machine or other classification algorithms is known to a person of skill in the art, like for example classification and regression trees, penalized logistic regression, sparse linear discriminant analysis, Fisher linear discriminant analysis, K-nearest neighbors, shrunken centroids, and artificial neural networks (see Wladimir Wapnik: The Nature of Statistical Learning Theory, Springer Verlag, New York, NY, USA, 1995; Berhard Schölkopf, Alex Smola: Learning with Kernels: Support Vector Machines, Regularization, Optimization, and Beyond, MIT Press, Cambridge, MA, 2002; S. Kotsiantis, Supervised Machine Learning: A Review of Classification Techniques, Informatica Journal 31 (2007) 249-268).

The key component of these classifier training and classification techniques is the choice of RNA biomarkers that are used as input to the classification algorithm.

In a further aspect, the invention refers to the use of a method as described above and herein for the detection of colorectal cancer in a human subject, based on RNA from a blood sample.

In a further aspect, the invention also refers to the use of a microarray for the detection of colorectal cancer in a human subject based on RNA from a blood sample. According to the invention, such a use can comprise measuring the abundance of at least 300 RNAs (or more, as described above and herein) that are listed in table 1. Accordingly, the microarray comprises at least 300 probes for measuring the abundance of the at least 300 RNAs. It is preferred that the microarray has a set of 11 probes for each RNA, but 1 or probes for each RNA are also possible. Commercially available microarrays, such as from Agilent Technologies, Inc., may be used. Alternatively, at most 599, at most 999, or at most 1199 RNAs are measured in a sample, in order to detect or diagnose CRC.

In another embodiment, the abundance of the at least 300 RNAs is measured by multiplex RT-PCR. In a further embodiment, the RT-PCR includes real time detection, e.g., with fluorescent probes such as Molecular beacons or TaqMan^{®} probes.

In a preferred embodiment, the microarray comprises probes for measuring only RNAs that are listed in table 1 (or subsets thereof).

In yet a further aspect, the invention also refers to a kit for the detection of colorectal cancer in a human subject based on RNA obtained from a blood sample. Such a kit comprises a means for measuring the abundance of at least 300 RNAs that are chosen from the RNAs listed in table 1. In a further embodiment, the at least 300 RNAs are chosen from the RNAs listed in any of the tables provided herein, for example, the RNAs are chosen from table 2. The means for measuring expression can be probes that allow for the detection of RNA in the sample or primers that allow for the amplification of RNA in the sample. Ways to devise probes and primers for such a kit are known to a person of skill in the art.

Further, the invention refers to the use of a kit as described above and herein for the detection of colorectal cancer in a human subject based on RNA from a blood sample comprising means for measuring the abundance of at least 300 RNAs that are chosen from the RNAs listed in table 1. Such a use may comprise the following steps: contacting at least one component of the kit with RNA from a blood sample from a human subject, measuring the abundance of at least 300 RNAs (or more as described above and herein) that are chosen from the RNAs listed in table 1 using the means for measuring the abundance of at least 300 RNAs, and concluding, based on the measured abundance, whether the subject has colorectal cancer.

In yet a further aspect, the invention also refers to a method for preparing an RNA expression profile that is indicative of the presence or absence of colorectal cancer, comprising: isolating RNA from a whole blood sample, and determining the level or abundance of from 300 to about 3000 RNAs, including at least 300 RNAs selected from table 1.

Preferably, the expression profile contains the level or abundance of 600 RNAs or less, of 1000 RNAs or less. Further, it is preferred that at least 300 RNAs, at least 600 RNAs, at least 1000 RNAs are listed in table 1.

Another preferred embodiment of the method comprises determining the presence or absence of an RNA expression signature indicative of colorectal cancer.

In yet a further aspect, the invention also refers to a microarray, comprising a solid support and a set of oligonucleotide probes, the set containing from 300 to about 3,000 probes, and including at least 300 probes selected from table 1 or 2. Preferably, the set contains 2000 probes or less, 1000 probes or less. At least 300 probes can be those listed in table 1. At least 600 probes can be those listed in table 1 or table 3. In another embodiment, at least 1000 probes are listed in table 1 or table 4.

Features of the invention that were described herein in combination with a method, a microarray, a kit, or a use also refer, if applicable, to all other aspects of the invention.

### Tables and Figure

Table 1 shows a list of 1200 RNAs that are differentially expressed in several human subjects with colorectal cancer in comparison to subject without colorectal cancer. Each marker is characterized by a SEQ ID NO., by an Agilent probe set ID, and, if applicable, a HUGO ID and a Ref. Seq ID.

Table 1A (shows the changes of expression level (abundance) between cases and controls for all of 1200 RNAs listed in table 1. The data was derived from 116 samples, 52 CRC cases and 64 controls. In the second column, the log-2 fold change is shown, and in the third column, the non-log fold change is shown. The numbers in the second column represent the differences of cases (events) and control (non-envents) in log2 steps. For example, a value of 0.53 for the probe set of SEQ ID NO. 145 means an expression increase by 2^{∧}0.53-fold for events vs. non-events. In other words, the events had a 144 % expression (see third column) with respect to non-events, i.e. 44 % more RNA (0.44 = 2^0.53-1, 1.44 = 2^-0.53).

Tables 2 through 4 show examples of combinations from table 1, varying in size from 250 to 800 sequences. Examples (signatures) 1, 2, and 3 each consist of 300 sequences. Examples (signatures) 4, 5, and 6 each consist of 600 sequences. Examples (signatures) 7, 8, and 9 each consist of 1000 sequences.

**Figure 1** is a graph showing the performance of various RNA colorectal cancer signatures as a function of their length. The x-axis shows the number of RNAs in each particular signature subset. The subset length is varied along the x-axis from 300 to 1200 (the full set). The y-axis shows performance of the subset. Performance is shown in terms of sensitivity (percentage of real carcinomas that were properly classified; lower values (crosses)) and specificity (percentage of real health controls that were properly classified; upper values (diamonds)) scaled from 80% to 100%, with standard errors of mean. These are retrospective examinations of the 1200 RNA full set. As the figure shows, reduced sets compared to the full set appear to have excellent performance. For each subset size, the performance was measured for 100 randomly chosen subsets based on leave-15 %-out runs: the subset is trained on 85 % and applied to the remaining 15 %. The average performance over all the 15 % tests is shown.

### Examples

### Materials and Methods

### Study Protocol

All results described herein are based on a prospective, clinical-diagnostic study protocol entitled Früherkennung kolorektaler Karzinome mit Hilfe von RNA-basierten Expression-Signaturen aus Blut - Eine multizentrische, diagnostische Studie zur Entwicklung und Validierung von Genexpressions-Signaturen zur Früherkennung kolorektaler Karzinome - Version CRC.SCR.2.BR.1 vom 06. Januar 2009" in accordance with the Guideline for Good Clinical Practice (Directive 75/318/EEC) July 1996, version July 2002 (http://www.emea.eu.int/pdfs/human/ich/013595en.pdf). This study protocol was reviewed and approved by the local ethics authority, the "Ethik-Kommission der Landesärztekammer Brandenburg" on January 14^{th} 2009. All persons entered into this study gave written informed consent that their blood samples and associated clinical data could be used for this research endeavor. Moreover, the persons gave written, informed consent that samples and clinical data could be audited by regulatory authorities if the research results would be used in a performance evaluation study (according to German law (Gesetz über Medizinprodukte)).

This study was designed as a cohort study. One cohort, the colonoscopy cohort, included persons undergoing colonoscopy. The second cohort, the surgery cohort, included patients scheduled for surgery for suspected colorectal carcinoma.

The inclusion criteria for the colonoscopy cohort were: 1) Willingness to undergo complete colonoscopy; 2) At least 55 years of age; 3) Ability to give written, informed consent; 4) Written informed consent after information about the study was given by a physician. The exclusion criteria for the colonoscopy cohort were: 1) Rectoscopy, sigmoidoscopy, or colonoscopy during the last five years prior to inclusion into the study; 2) Treatment of a malignant disease during the last five years prior to inclusion into the study, except for malignoma with low metastatic potential such as basalioma in situ of the skin.

The inclusion criteria for the surgery cohort were: 1) Age at initial diagnosis at least 18 years of age; 2) Ability to give written, informed consent; 3) (Suspected) Diagnosis of colorectal carcinoma UICC-stage I to IV; 4) Surgery is planned in such a fashion that staging according to UICC-criteria is feasible; 5) No treatment prior to surgery; 6) No treatment for a malignant disease during the last five years prior to inclusion into the study; 7) No other disease that lowers life expectancy below one year; 8) Regular follow-up examinations have to be possible; 9) Written informed consent after information about the study was given by a physician.

### Recruitment

499 persons scheduled for colonoscopy were recruited into the study; RNA blood samples were taken prior to colonoscopy. The recruitment period for the non-CRC patients lasted from February 9, 2009 until April 3, 2009. In parallel, RNA blood samples were taken from patients with (suspected) diagnosis of CRC prior or during surgery for CRC.

### Blood Drawing and Blood Sample Storage

In the colonoscopy cohort, blood was drawn after written, informed consent and prior to bowel cleaning and change of medication if necessary. In the surgery cohort, blood was drawn after written, informed consent and prior to removal of the resected tissue from the body of the patient by the surgeon. In both cohorts, colonoscopy cohort and surgery cohort, blood was drawn into PAXgene™ blood tubes (Becton Dickinson). The tubes were stored within four hours in a freezer at -20 °C until transport to the laboratory on dry ice, where the tubes were stored again in a freezer at -20 °C until RNA-extraction.

### Sample Sizes

The study protocol initially stipulated the use of 220 blood samples from the colonoscopy cohort and 220 blood samples from the surgery cohort for the discovery of the signatures. However, the study protocol was open to changes depending on the results of the discovery process. Additionally, the study protocol initially stipulated the use 220 blood samples from the colonoscopy cohort and 220 blood samples from the surgery cohort for prospective performance evaluation purposes. Again, these samples sizes were open and amenable to change.

### Quality Control of Clinical Data

All clinical data of all included persons from both cohorts were checked directly from the patient's medical records and entered into study databases, as prescribed in the study protocol. Each item in the study databases were checked independently by two persons. Patients of both cohorts together with their blood samples were withdrawn if any violation of the inclusion of exclusion criteria cited above was detected. In particular for the colonoscopy cohort, all samples and their associated clinical data were excluded from all analyses if the colonoscopy was found to be not complete. Moreover, blood samples were destroyed and clinical data were deleted if the patient decided to withdraw his/her informed consent.

### Sample Selection for RNA-Extraction

The selection criteria of CRC-cases included availability of UICC stage information and the check that the patients did not fulfill any exclusion criteria for the MSKK-study. At this time clinical data of the non-CRC case were not available. Hence, the first 240 patients of the study were selected.

Altogether, 480 PAX-RNA samples, 240 from non-CRC persons and 240 from CRC patients, were randomized into the discovery set. Additionally, the order of processing by the laboratory was randomized. A drop-out rate of 10 %, or 20 cases in each group, was assumed.

### RNA extraction

Total RNA extraction from blood was performed using the QIAGEN PAXgene Blood miRNA Kit together with the QIAcube^{®} robot according to the manufacturer's instructions. The RNA obtained is therefore sometimes referred to as PAX-RNA.

Before starting RNA extraction, the buffers BM3 and BM4 were mixed with the specified amount of 96-100 % Ethanol. Furthermore, the DNAse I (1500 Kunitz units) was dissolved in 550 µl of RNAse free water.

After thawing, the PAX-blood tubes were turned several times to ensure proper mixing and left over night (or minimal two hours) at room temperature. Then, the tubes were centrifuged for 10 minutes at 4000 x g using a swing out bucket. Next, the supernatant was carefully removed from each tube, and the pellets were washed (vortexing until complete resuspension) with 4 ml RNAse free water. After centrifuging again for 10 minutes at 4000 x g, the pellets were resuspended in 350 µl buffer BR1 and transferred to 2 ml processing tubes. The opened tubes were then loaded into the rotor adapters of the centrifuge. Subsequently, all buffers were placed on the respective spots of the reagent bottle holder. After closing the lid, the PAXgene Blood miRNA Part A protocol was started. When it was finished, the instrument door was opened, the RNA containing tubes were closed and subsequently placed on the shaker adaptor. After closing the instrument door again, the PAXgene Blood miRNA Part B protocol was started. When the program was finished, concentration was determined by UV-absorption measurement and the samples were stored at -70 °C until use.

For understanding of underlying principles of the automatic procedure the manual protocol is briefly described below. There is no difference between the two methods until the resuspension of the pellet.

To the resupended pellet 300 µl binding buffer (BR2) and 40 µl proteinase K solution was added and mixed by vortexing for 5 seconds. Incubation follows for 10 minutes at 55 °C using a shaker-incubator at 400-1400 rpm. After incubation, the temperature of the shaker-incubator is set to 65 °C. The lysate is pipetted directly into a PAXgene Shredder spin column (lilac) placed in a 2 ml processing tube and centrifuged for 3 minutes at maximum speed (don't not to exceed 20,000 x g). The entire supernatant of the flow-through fraction was carefully transferred the to a fresh 1.5 ml microcentrifuge tube without disturbing the pellet in the processing tube. 350 µl ethanol (96-100%, purity grade p.a.) was added and mixed by vortexing. The mixture is briefly (1-2 seconds at 500-1000 x g) centrifuged to remove drops from the inside of the tube Lid. 700 µl of the sample is pipetted into the PAXgene RNA spin column (red) placed in a 2 ml processing tube, and centrifuged for 1 minute at 8,000-20,000 x g. The spin column was placed in a new 2 ml processing tube (PT), and the old processing tube containing flow-through discarded. Subsequently, the remaining sample was pipetted into the PAXgene RNA spin column, and centrifuged for 1 minute at 8,000-20,000 x g. The spin column was placed in a new 2 ml processing tube and the old processing tube containing flow-through again discarded. Subsequently, 350 µl wash buffer 1 (BR3) was pipetted into the PAXgene RNA spin column. After centrifugation for 1 minute at 8000-20,000 x g the spin column was placed in a new 2 ml processing tube and the old processing tube containing the flow-through again discarded. 10 µl DNase I stock solution is added to 70 µl DNA digestion buffer in a 1.5 ml microcentrifuge tube and mixed by gently flicking the tube followed by a brief centrifugation to collect residual liquid from the sides of the tube. The DNase I incubation mix (80 µl) was pipetted directly onto the PAXgene RNA spin column membrane, and placed on the benchtop (20-30 °C) for 15 minutes. After Incubation, 350 µl wash buffer 1 (BR3) is pipetted into the PAXgene RNA spin column and centrifuged for 1 minute at 8000-20,000 x g. The spin column was placed in a new 2 ml processing tube and the old processing tube containing flow-through again discarded. 500 µl wash buffer 2 (BR4) was pipetted into the PAXgene RNA spin column and centrifuged for 1 minute at 8,000-20,000 x g. The spin column was placed in a new 2 ml processing tube and the old processing tube containing flow-through again discarded. Another 500 µl wash buffer 2 (BR4) was added to the PAXgene RNA spin column and centrifuged for 3 minutes at 8,000-20,000 x g. The spin column is placed in a new 2 ml processing tube and the old processing tube containing flow-through again discarded. The column is centrifuged for 1 minute at 8,000-20,000 x g. The processing tube is discarded and the column was placed on a 1.5 ml microcentrifuge tube. 40 µl elution buffer (BR5) directly pipetted onto the PAXgene RNA spin column membrane and subsequntly centrifuged for 1 minute at 8,000-20,000 x g to elute the RNA. The elution step is repeated using again 40 µl elution buffer (BR5) and the same 1.5 ml microcentrifuge tube. The RNA is denatured for 5 minutes at 65 °C in the shaker-incubator (see above) without shaking.

### Results of RNA-Extraction

The quality control is performed on the Agilent Bioanalyzer.

From the RNA-extraction samples, 14 samples (2.92 %) showed RNA integrity numbers (RIN) lower than 3.5, which rendered these samples unfit for microarray hybridization.

### Selection of Samples for Microarray Hybridization

Based on the results of RNA extraction and clinical information about UICC-stages and complete colonoscopy in case of non-CRC cases, 117 samples were authorized for microarray hybridization. The vast majority of drop-outs were incomplete colonoscopies.

### Labeling and Chip Hybridization

Total RNA (100 ng) was labeled and hybridized onto Agilent Sureprint 8x60k arrays (Agilent Technologies, Inc., Santa Clara, CA, USA) according to the manufacturer's instructions (One-Color Microarray-Based Gene Expression Analysis Low Input Quick Amp Labeling, Version 6.5, May 2010).

### Spike Mix and Sample Preparation

1 The One-Color Spike Mix stock solution was vigorously mixed on a vortex mixer.
2 The stock solution was heated to 37°C for 5 minutes, and mixed on a vortex mixer once more.
3 The stock solution was briefly spun in a centrifuge to drive contents to the bottom of the tube prior to opening.
4 Three serial dilutions from One-Color Spike Mix stock (1:20 1:25 1:20) were prepared.
5 2µl of the "Spike Mix Third Dilution" were added to the RNA sample (100 ng in 1,5 µl water).

### Labeling Reaction

1 The 0,8 µl T7 Promoter Primer and 1µl water were mixed to prepare the T7 Promoter Primer Master Mix.
2 1.8 µL of T7 Promoter Primer Mix was added to the tube that contained 3.5 µL of total RNA and diluted RNA spike-in controls. Each tube now contained a total volume of 5.3 µL.
3 The primer and the template were denatured by incubating the reaction at 65°C in a circulating water bath for 10 minutes.
4 The reactions were placed on ice and incubated for 5 minutes.
5 The 5X first strand buffer was pre-warmed at 80°C for 3 to 4 minutes to ensure adequate resuspensions of the buffer components. The tube containing the 5X first strand buffer was briefly mixed on a vortex mixer and spun the tube in a microcentrifuge to drive down the contents from the tube walls. The buffer was kept at room temperature until it was used.
6 Immediately prior to use, the components were added for the cDNA Master Mix
   - 5X First Strand Buffer (green cap) 2 µl
   - 0,1 M DTT (white cap) 1 µl
   - 10 mM dNTP mix (green cap) 0.5 µl
   - AffinityScript RNase Block Mix (violet cap) 1.2 µl
   - Total Volume 4.7 µl
   A pipette was used to gently mix the components, and was kept at room temperature. The AffinityScript RNase Block mix was kept on ice and was added to the cDNA Master Mix immediately prior to use.
7 4.7 µl of cDNA Master Mix was added to each sample tube and mixed by pipetting up and down. Each tube now contained a total volume of 10 µl.
8 The samples were incubated at 40°C in a circulating water bath for 2 hours.
9 The samples were moved to a 70°C circulating water bath and incubated for 15 minutes.
10 The samples were then moved to ice and incubated for 5 minutes.
11 The samples were spun briefly in a microcentrifuge to drive down tube contents from the tube walls and lid.
12 The Transcription Master Mix was prepared and added.
   - Nuclease-free water (white cap) 0.75 µl
   - 5X Transcription Buffer (blue cap) 3.2 µl
   - 0,1 M DTT (white cap) 0.6 µl
   - NTP mix (blue cap) 1 µl
   - T7 RNA Polymerase Blend (red cap) 0.21 µl
   - Cyanine 3-CTP 0.24 µl
13 6 µL of Transcription Master Mix was added to each sample tube and gently mixed by pipetting. Each tube now contained a total volume of 16 µL.

The samples were incubated in a circulating water bath at 40°C for 2 hours.

### The Labeled/Amplified RNA was purified

Qiagen's RNeasy mini spin columns were recommended for purification of the amplified cRNA samples.
1 84 µl of nuclease-free water was added to the cRNA sample, for a total volume of 100 µl.
2 350 µl of Buffer RLT was added and mixed well by pipetting.
3 250 µl of ethanol (96% to 100% purity) was added and mixed thoroughly by pipetting. The mixture was *not* centrifuged.
4 The 700 µl of the cRNA sample was transfered to an RNeasy mini column in a 2 ml collection tube. The sample was centrifuged at 4 °C for 30 seconds at 13,000 rpm. The flow-through and collection tube were discarded.
5 The RNeasy column was transfered to a new collection tube, and 500 µl of buffer RPE (containing ethanol) was added to the column. The sample was centrifuged at 4 °C for 30 seconds at 13,000 rpm. The flow-through was discarded. The collection tube was, however, re-used.
6 Another 500 µl of buffer RPE was added to the column. The sample was centrifuged at 4 °C for 60 seconds at 13,000 rpm. The flow-through and the collection tube were discarded.
7 When any buffer RPE remains on or near the frit of the column, the RNeasy column was transfered to a new 1.5 mL collection tube and d the sample was centrifuged at 4 °C for 30 seconds at 13,000 rpm to remove any remaining traces of buffer RPE. This collection tube was discarded, and a fresh tube was used to elute the cleaned cRNA sample.
8 The sample was quantitated on a UV spectrometer and the following items were recorded:
   - Cyanine 3 dye concentration (pmol/µl)
   - RNA absorbance ratio (260 nm/280 nm)
   - cRNA concentration (ng/µl)
9 The specific activity was determined using the following formula:
   ,Concentration of Cy3 / Concentration of cRNA = pmol of Cy3 per µg cRNA The specific activity should be six. Around 1 µg cRNA was needed for an eight pack array.

### Hybridization

1 500 µL of nuclease-free water was added to the vial containing lyophilized 10X Blocking Agent supplied with the Agilent Gene Expression Hybridization Kit, or add 1250 µL of nuclease-free water to the vial containing lyophilized large volume 10X Blocking Agent (Agilent p/n 5188-5281).
2 The solution was mixed by gently vortexing. When the pellet did not go into solution completely, the mix was heated for 4 to 5 minutes at 37°C.
3 Any material adhering to the tube walls or cap was driven down by centrifuging for 5 to 10 seconds. (10X Blocking Agent was prepared in advance and stored at -20°C for up to 2 months. After thawing, the vortexing and centrifugation procedures were repeated before use.)
4 The water bath was equilibrated to 60°C.
5 For each microarray, each of the components was added as indicated to a 1.5 mL nuclease-free microfuge tube:
   - cyanine 3-labeled, linearly amplified cRNA 600 ng
   - 10X Blocking Agent 5 µl
   - Nuclease-free water µl bring volume to 24 µl
   - 25X Fragmentation Buffer 1 µl
6 The mix was incubated at 60°C for exactly 30 minutes to fragment RNA.
7 The mix was mmediately cooled on ice for one minute.
8 25 µl 2x GEx Hybridization Buffer HI-RPM was added to the 25 µl fragmentation mixture to stop the reaction.
9 The mixture was mixed well by careful pipetting. Care was taken to avoid introducing bubbles. The mixture was not mixed on a vortex mixer because mixing on a vortex mixer introduces bubbles.
10 The mixture was spun for 1 minute at room temperature at 13,000 rpm in a microcentrifuge to drive the sample off the walls and lid and to aid in bubble reduction. It was used immediately and not stored.
11 The sample was placed on ice and loaded onto the array quickly.
12 A clean gasket slide was loaded into the Agilent SureHyb chamber base with the label facing up and was aligned with the rectangular section of the chamber base. It was ensured that the gasket slide was flush with the chamber base and was not ajar.
13 The 40 µl of hybridization sample was slowly dispensed onto the gasket well in a "drag and dispense" manner.
14 An array "active side" down was slowly placed onto the SureHyb gasket slide, so that the "Agilent"-labeled barcode was facing down and the numeric barcode was facing up. The sandwich-pair was properly aligned. (The pipette tip or the hybridization solution was prevented from touching the gasket walls because when liquid touches the gasket wall, the likelihood of gasket leakage greatly increases.)
15 The SureHyb chamber cover was placed onto the sandwiched slides and the clamp assembly was slid onto both pieces.
16 The clamp was hand-tightened onto the chamber.
17 The assembled chamber was vertically rotated to wet the gasket and assess the mobility of the bubbles. The assembly was tapped on a hard surface to move stationary bubbles.
18 The assembled slide chamber was placed in rotisserie in a hybridization oven set to 65°C. The hybridization rotator was set to rotate at 10 rpm when using 2x GEx Hybridization Buffer HI-RPM.
19 Hybridization took place at 65°C for 17 hours.

### Washing

The Gene Expression Wash Buffer 2 was warmed overnight. The 0.005% Triton X-102 to the Gene Expression wash buffers was added to reduce the possibility of array wash artifacts. The Triton X-102 was added to the Gene

Expression wash buffer 1 and 2 when the cubitainer of wash buffer was first opened.
1 The cardboard box with the cubitainer of wash buffer was opened, and the outer and inner caps were carefully removed from the cubitainer.
2 A pipette was used to add 2 mL of the provided 10% Triton X-102 into the wash buffer in the cubitainer.
3 The original inner and outer caps were replaced, and the buffer was mixed carefully, but thoroughly, by inverting the container 5 to 6 times.
4 The outer and inner caps were carefully removed, and the spigot provided was installed with the wash buffer.
5 The wash buffer box was prominently labeled to indicate that Triton X-102 has been added and indicated the date of addition.
6 All dishes, racks, and stir bars were washed with Milli-Q water. (The microarray wash procedure for Agilent's one-color platform was done in an environment containing ozone levels of 50 ppb or less. Ozone levels were prevented from exceeding 50 ppb by using the Agilent Ozone Barrier Slide Cover)
7 The slide-staining dish #1 was completely filled with Gene Expression Wash Buffer 1 at room temperature.
8 A slide rack was placed into slide-staining dish #2 and a magnetic stir bar was added. The slide-staining dish #2 was filled with enough Gene Expression Wash Buffer 1 at room temperature to cover the slide rack. This dish was placed on a magnetic stir plate.
9 The empty dish #3 was placed on the stir plate and a magnetic stir bar was added. The pre-warmed (37°C) Gene Expression Wash Buffer 2 was not added until the first wash step had begun.
10 One hybridization chamber was removed from the incubator and the time recorded. It was recorded whether bubbles formed during hybridization and if all bubbles were rotating freely.
11 The hybridization chamber assembly was placed on a flat surface, and the thumbscrew was loosened by turning it counterclockwise.
12 The clamp assembly was taken off, and the chamber cover was removed.
13 The array-gasket sandwich was removed, using gloved hands, from the chamber base by grabbing the slides from their ends. The microarray slide numeric barcode of the sandwich was kept facing up as it was quickly transferred to the slide-staining dish #1.
14 Without letting go of the slides, the array-gasket sandwich was submerged into slide-staining dish #1 containing Gene Expression Wash Buffer 1.
15 With the sandwich completely submerged in Gene Expression Wash Buffer 1, the sandwich was pried open from the barcode end only:
16 One of the blunt ends of the forceps was slipped between the slides.
17 The forceps were gently turned upwards or downwards to separate the slides.
18 The gasket slide was allowed to drop to the bottom of the staining dish.
19 The microarray slide was removed and placed into slide rack in the slide-staining dish #2 containing Gene Expression Wash Buffer 1 at room temperature. Exposure of the slide to air was kept at a minimum. Only the barcode portion of the microarray slide or its edges was touched.
20 Steps 10 through 19 were repeated for up to seven additional slides in the group. For uniform washing, a maximum of eight disassembly procedures were done at a time yielding eight microarray slides.
21 When all slides in the group were placed into the slide rack in slide-staining dish #2, they were stirred using setting 4 for 1 minute.
22 During this wash step, Gene Expression Wash Buffer 2 was removed from the 37°C water bath and poured into the slide-staining dish #3.
23 The slide rack was transferred to slide-staining dish #3 containing Gene Expression Wash Buffer 2 at elevated temperature and stirred using setting 4 for 1 minute.
24 The slide rack was slowly removed minimizing droplets on the slides. 5 to 10 seconds were required to remove the slide rack.
25 Used Gene Expression Wash Buffer 1 and Gene Expression Wash Buffer 2 were discarded.
26 Step 7 through step 25 were repeated for the next group of eight slides using fresh Gene Expression Wash Buffer 1 and Gene Expression Wash Buffer 2 pre-warmed to 37°C.
27 The slides were placed in a slide holder so that the Agilent barcode faced up.
28 Slides were scanned immediately to minimize the impact of environmental oxidants on signal intensities. When necessary, the slides were stored in orange slide boxes in a nitrogen purge box, in the dark.

### Scanning and Feature Extraction

1 The assembled slide holders were placed with or without the ozone-barrier slide cover into the scanner carousel.
2 In the Scan Control main window, the slot number of the first slide was chosen for Start Slot and the slot number for the last slide for End Slot.
3 For 8x60K microarrays, the Profile AgilentG3_GX_1Color was selected.
4 The Scanner status was verified in the main window says Scanner Ready.
5 In the Scan Control main window, Scan Slot m-n was clicked where m is the slot of the first slide, and n is the slot for the last slide.
6 After generating the microarray scan images, .tif images were extracted using the Feature Extraction software.

### Microarray Quality Control

The microarray data authorized for quality control were checked. QCStats parameter according to the Agilent feature extraction software (gNonCntrlMedCVProcSignal, IsGoodGrid, AnyColorPrcntFeatNonUnifOL, gNegCtrlAveNetSig, gNegCtrlAveBGSubSig, gNegCtrlSDevBGSubSig, gSpatialDetrendRMSFilteredMinusFit, gNonCntrlMedCVProcSignal) were evaluated. Arrays with more than two parameters above the quality limit given by the manufacturer were excluded. Furthermore the spearman correlation between the raw expression values was used for quality evaluation. The threshold was set to 0.85. One sample did not pass QC.

### Discovery Procedure

In brief, all raw measurement data from microarray hybridizations scheduled for a discovery (i.e., from samples selected for discovery, and measurements that passed quality control) were background subtracted and subsequently quantile normalized. For Probes which have several copies on the array the median was used. During this discovery, the statistical software systems R (version 2.9.0) and Bioconductor (version 1.6) were used (for this software, see http://cran.r-project.org/, http://www.r-project.org/ and http://www.bioconductor.org, also Ross Ihaka and Robert Gentleman. R: A language for data analysis and graphics. Journal of Computational and Graphical Statistics, 5(3): 299-314, 1996; Gentleman et al 2005: Bioinformatics and Computational Biology Solutions Using R and Bioconductor, Springer, New York, NY).

The resulting array data were then partitioned into groups of samples in a double nested bootstrap approach (Efron (1979) Bootstrap Methods--Another Look at the Jackknifing, Ann. Statist. 7, 1-6). In the outer loop of this bootstrap, the samples were partitioned into an outer test set and an outer training set. In the inner bootstrap loop, this outer training set was partitioned again into an inner training set and an inner test set.

On the inner training set, probeset relevance was estimated through a decision-tree-analysis. The influence of each feature was determined from its contribution to the classification error: In case the error of a probe increases due to the permutation of the values of a probe, while the values of all other probesets remain unchanged, this probeset is weighted more strongly.

The relevance evaluations of the individual inner loops were combined for each external loop and the chosen probesets were used to train a support vector machine (Bernhard Schölkopf, Alex Smola: Learning with Kernels: Support Vector Machines, Regularization, Optimization, and Beyond, MIT Press, Cambridge, MA, 2002, also see citations above). This classifier was trained on the outer training set (which was already used for its feature selection) and applied to the outer test set of samples. For this classification, the same methods and parameters were use as for the intended application on external data (for example as part of validation): the details are described below as application for the RNAs of the invention. The whole external loop of the bootstrap procedure is a simulation of later classification of unknown data. Its average performance over all external loops gives a prospective estimate of the performance of the classification procedure (mainly based on the chosen biomarker set). The common mistake of overfitting, i.e., the overly optimistic evaluation of a classification method on its discovery set, is thereby avoided.

Finally, the results of all inner loops and of all external loops were combined to form a common biomarker set with prospective estimates of performance.

### Application of the Invention

A number of RNAs that result from the discovery above can be used with state of the art classification methods as described in the cited literature and as known by a person of skill in the art. As any classification, it will require a representative training set, which the inventors obtained through a clinical study fulfilling the requirements described above. Part of the training set is the necessary clinical information (carcinoma patient or healthy control, as defined by the clinical study). Similar to the clinical requirements, this description of the algorithmic part of the application of the invention also presupposes the described lab process. Quality controls were applied.

The training set of microarray raw data was treated in the same way as the discovery set in the section "Discovery Procedure" above. A standard implementation of support vector machines, such as the one contained in Bioconductor's (see references above) package for machine learning "e1071" with parameter "kernel" set to "linear" (preferential version for this package is 1.5-19).

It is important not to present the classification information on the training data as numeric data, but as categorical data. This can be ensured by passing the corresponding arguments as an R "factor" (for example as in "svm(..., as.factor(clinicalData),...)". Otherwise this svm algorithm will use the wrong type of classification.

To apply this svm, using the same software package, to any kind of new microarray data, a normalisation step is necessary just as for the discovery data. It is possible to simply reapply the method above (backgroung subtraction, normalization, median calculation for multiple probes) to each individual new sample in combination with the whole discovery set. This method is to explicitly compute a normalization model (quantile values) on the discovery data and apply it to the test data. This can be done within the R software package. The application of the described svm to the thus normalized test data produces the desired decision value.

The embodiments illustrated and discussed in this specification are intended only to teach those skilled in the art the one way to make and use the invention. Modifications and variation of the above-described embodiments of the invention are possible without departing from the invention, as appreciated by those skilled in the art in light of the above teachings. It is therefore understood that, within the scope of the claims and their equivalents, the invention may be practiced otherwise than as specifically described.

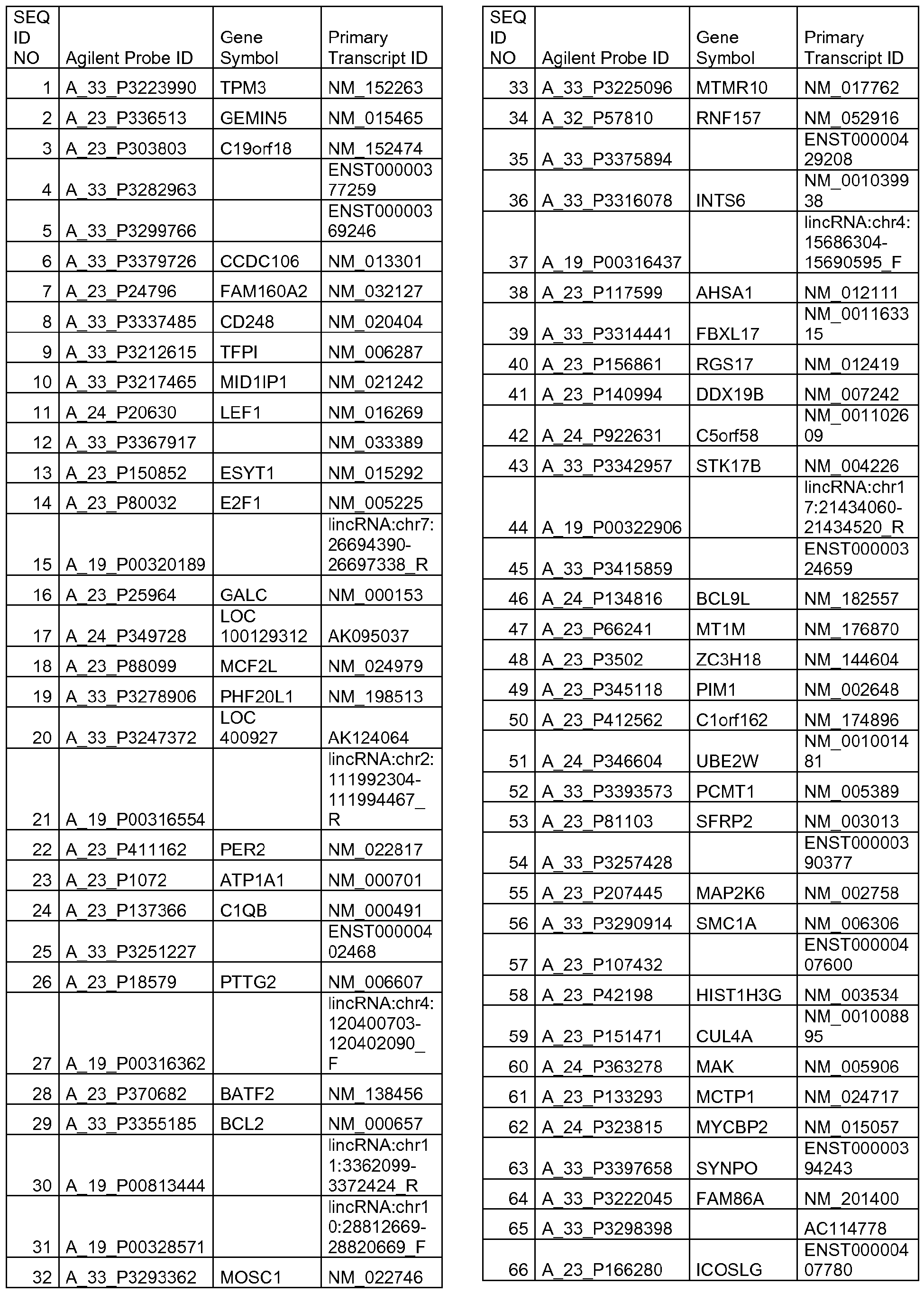

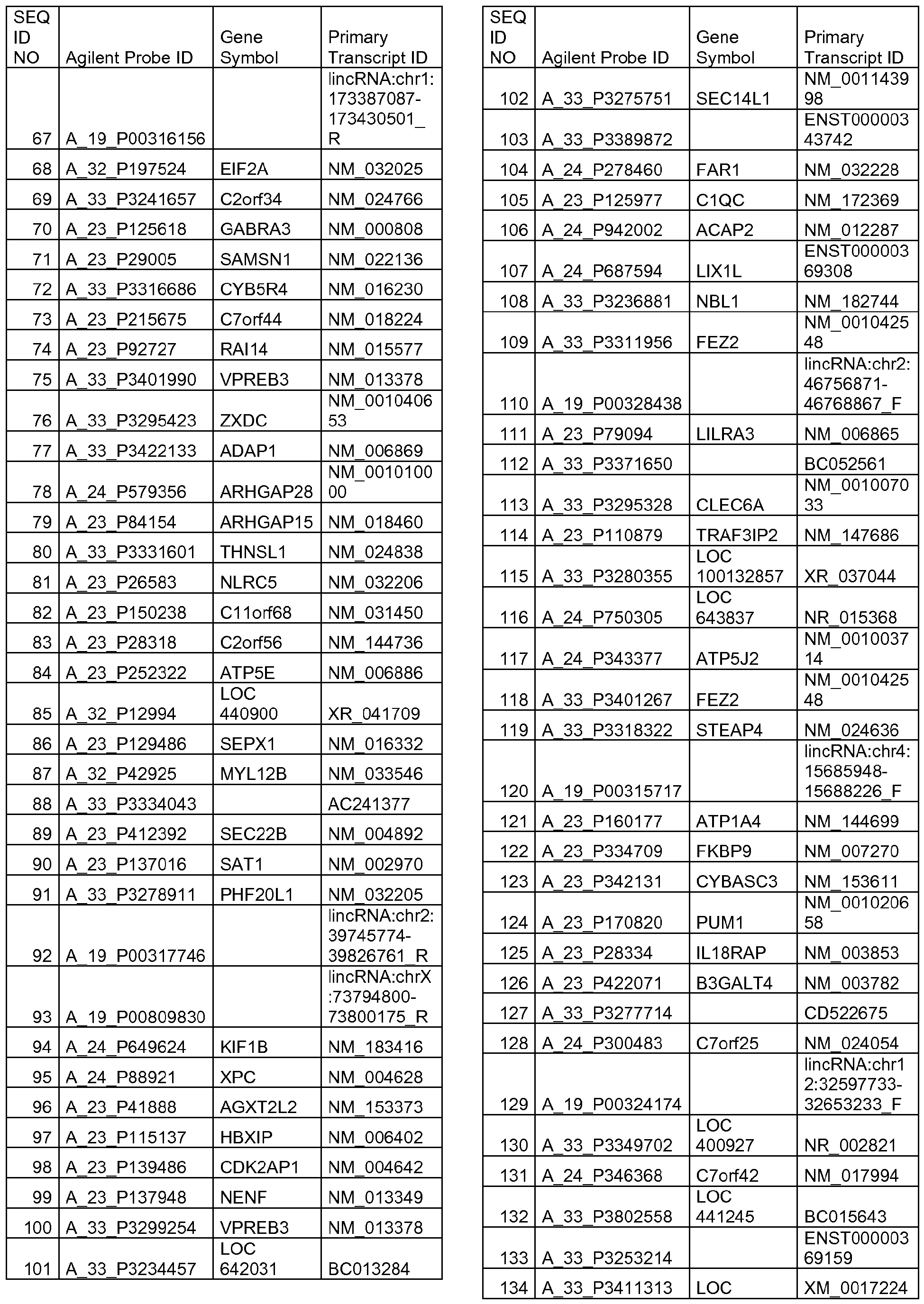

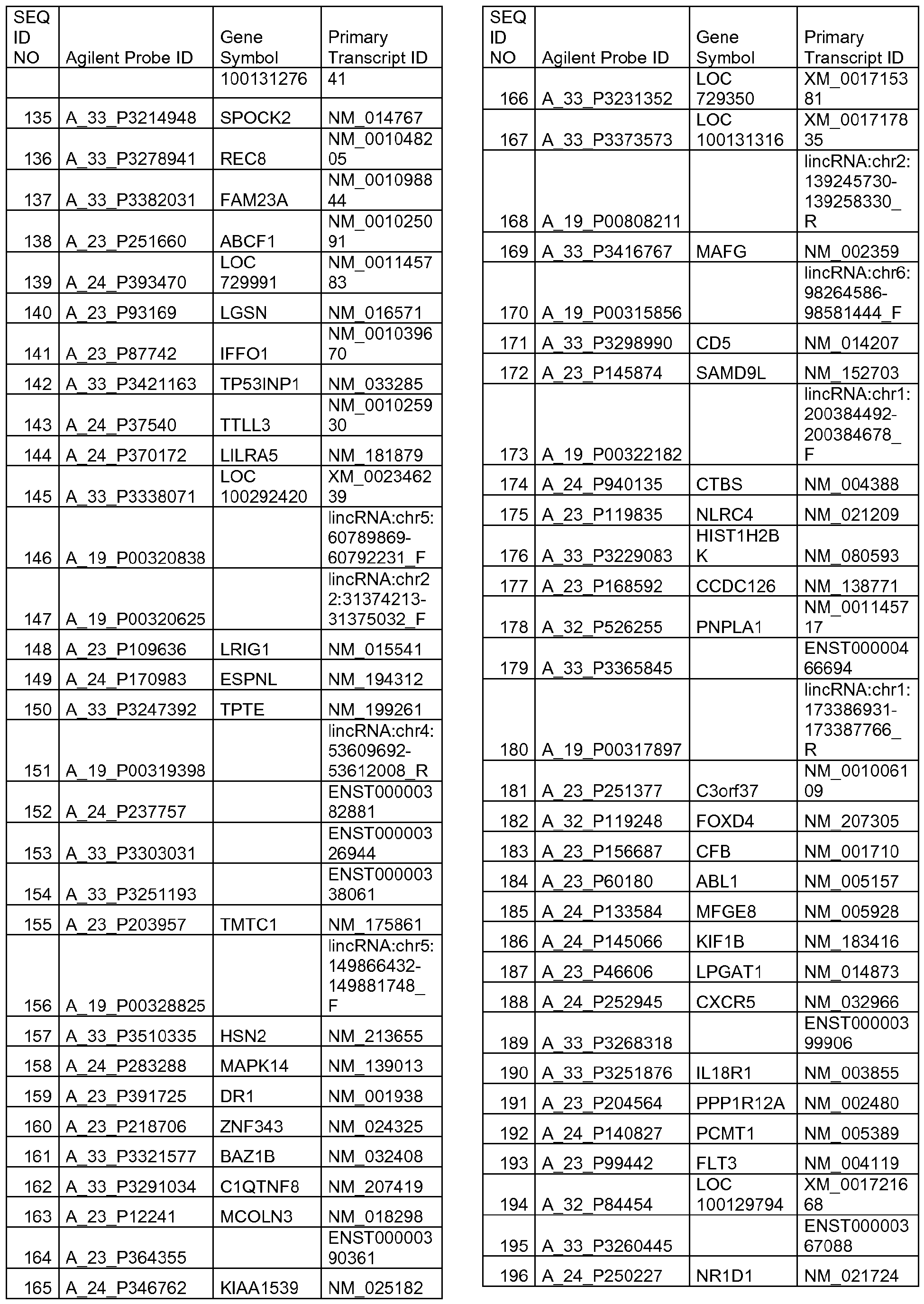

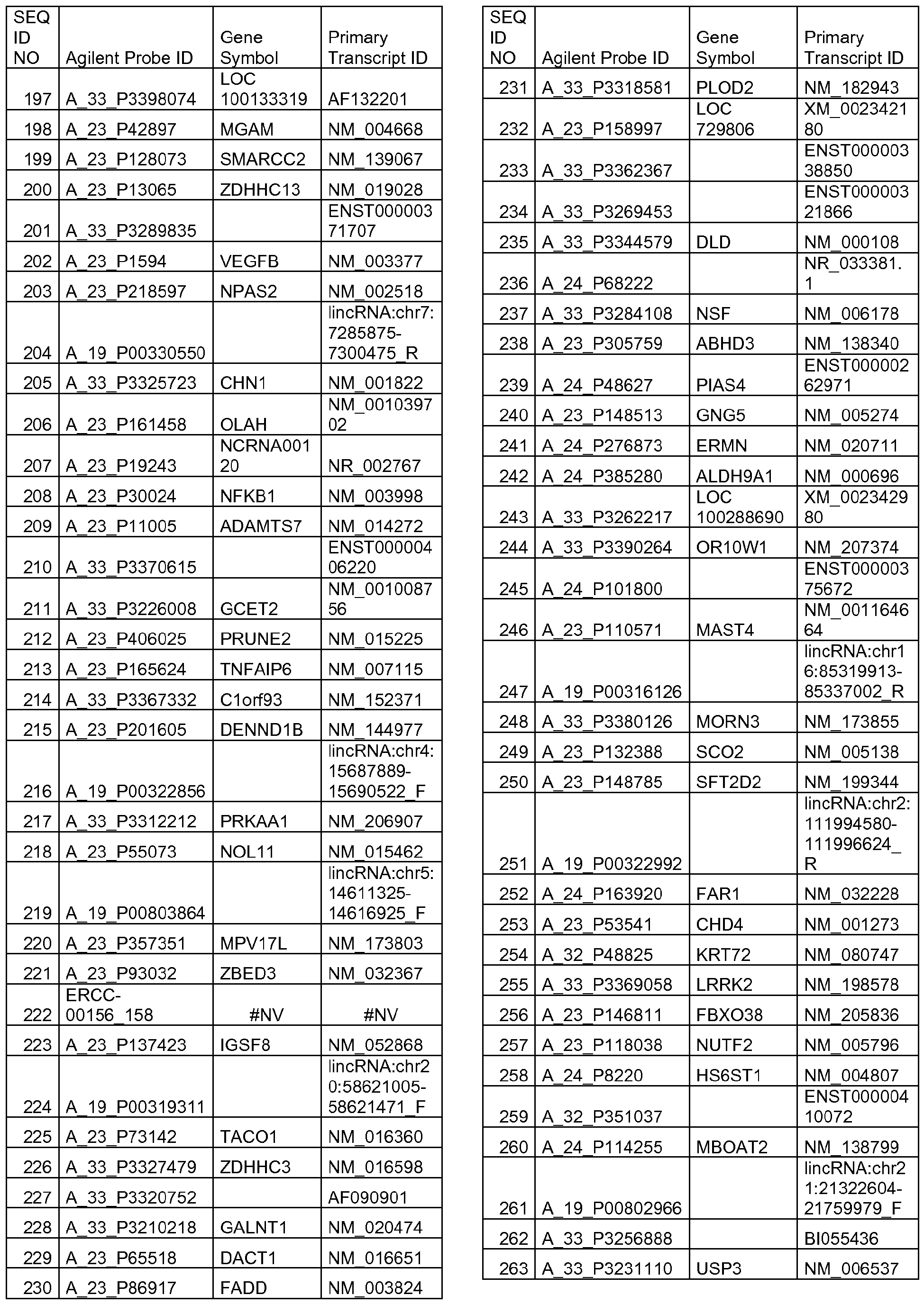

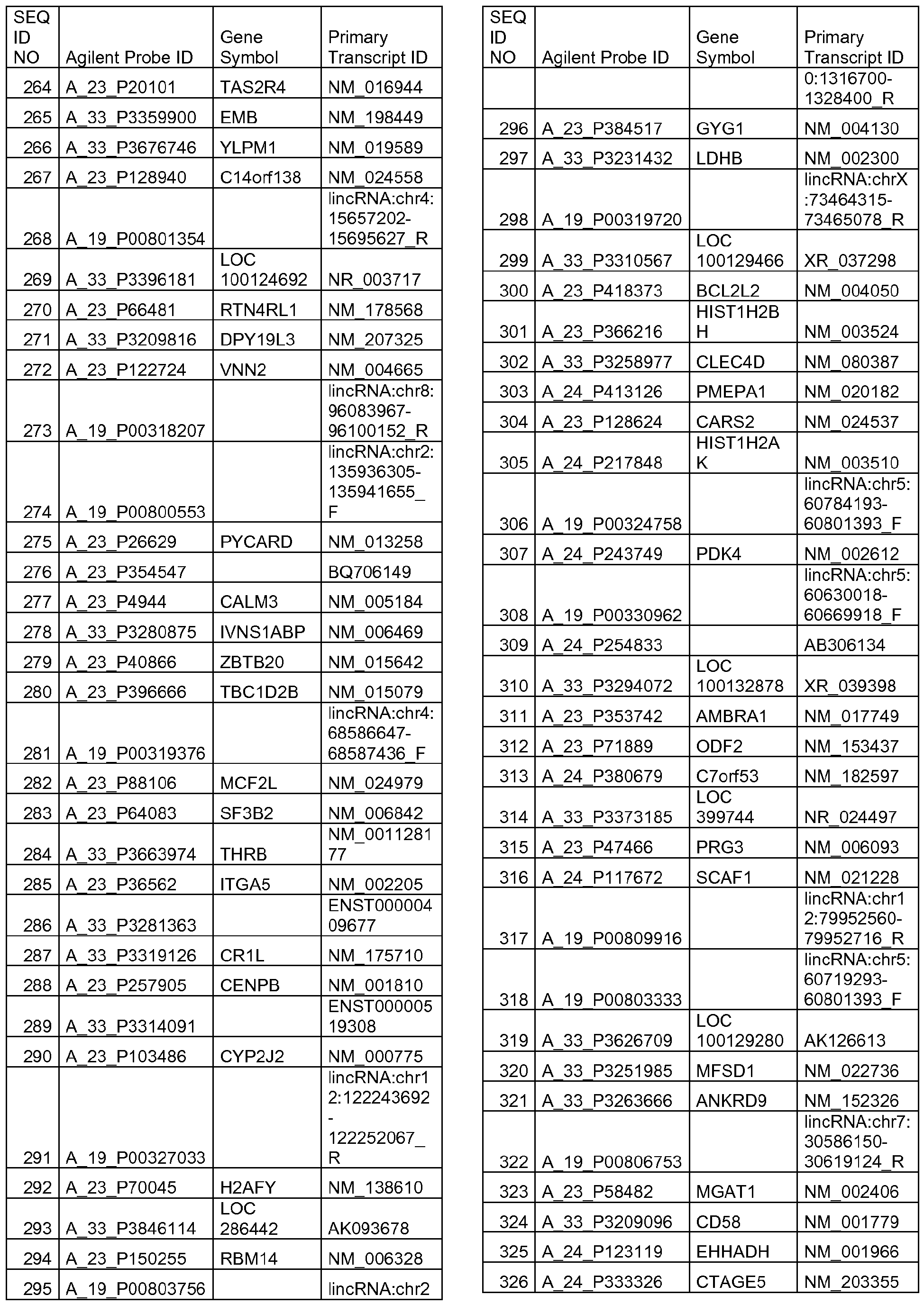

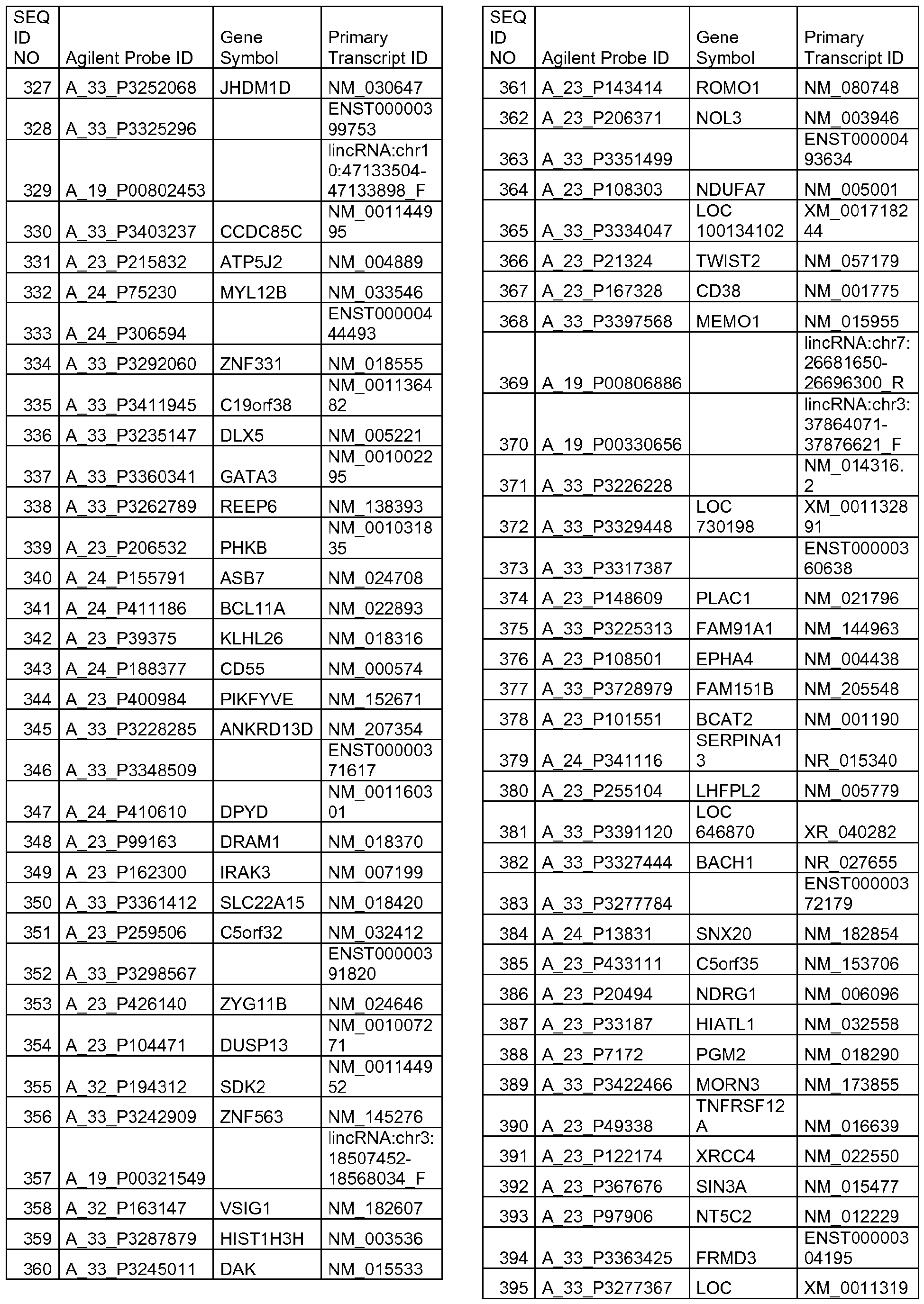

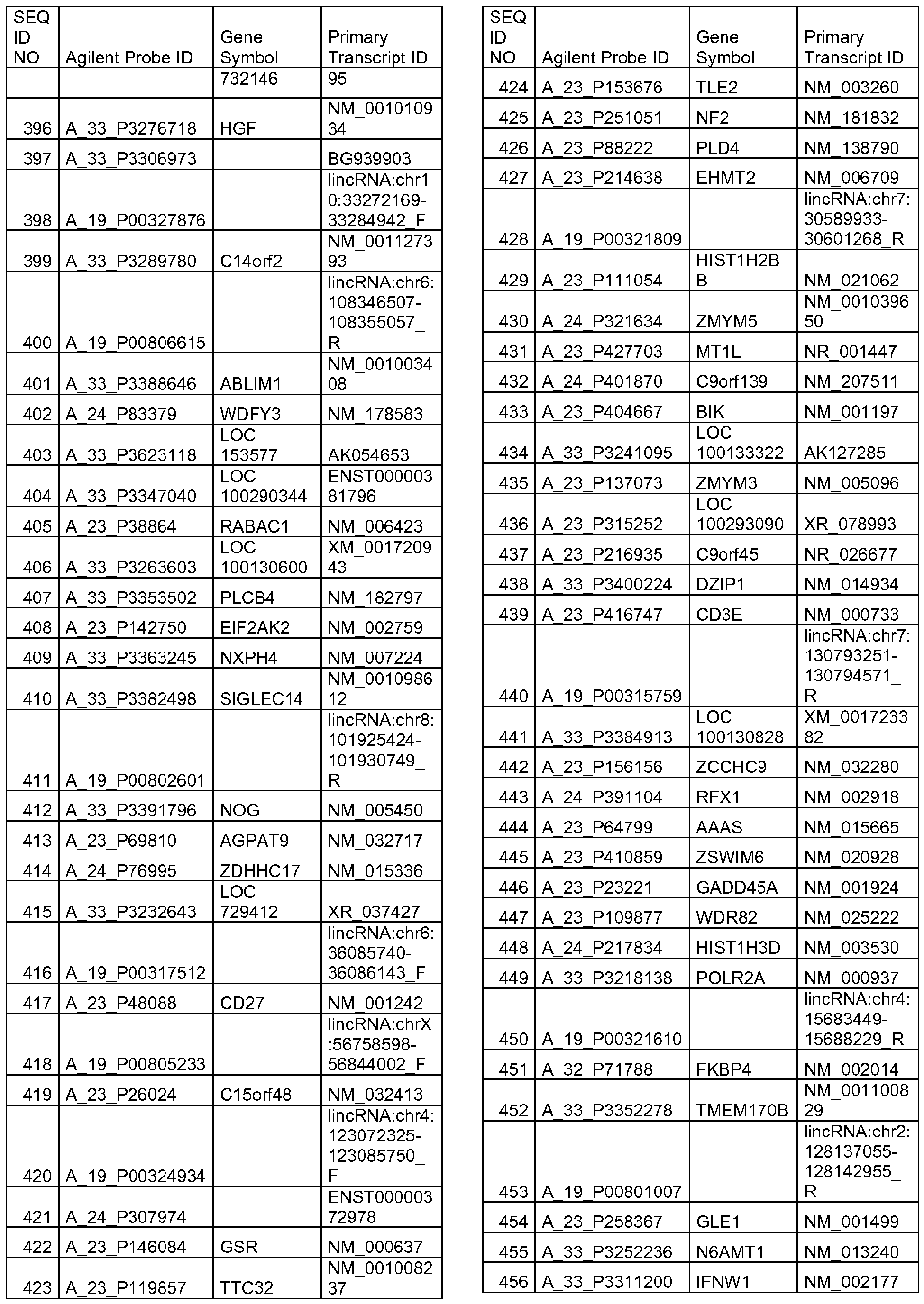

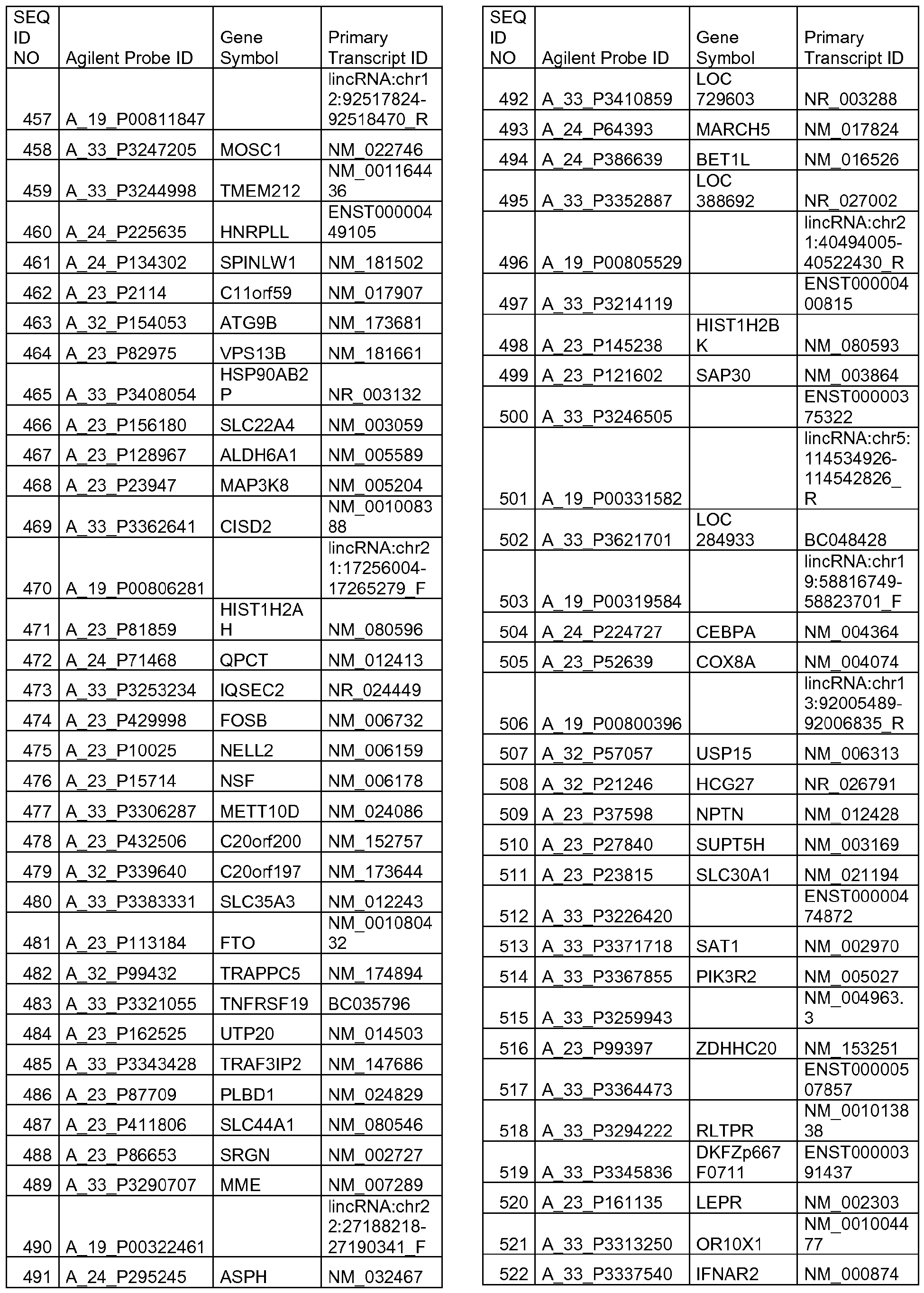

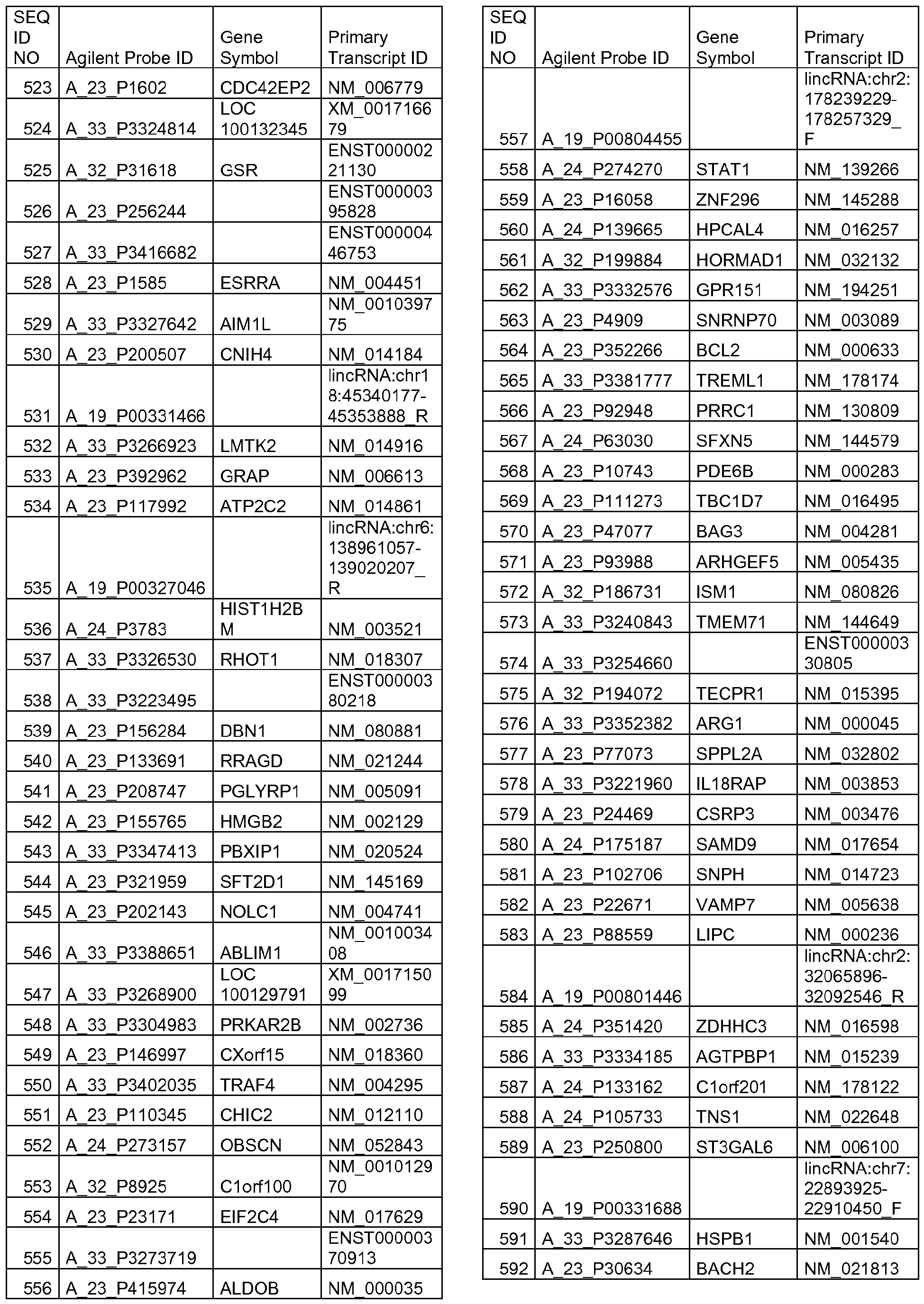

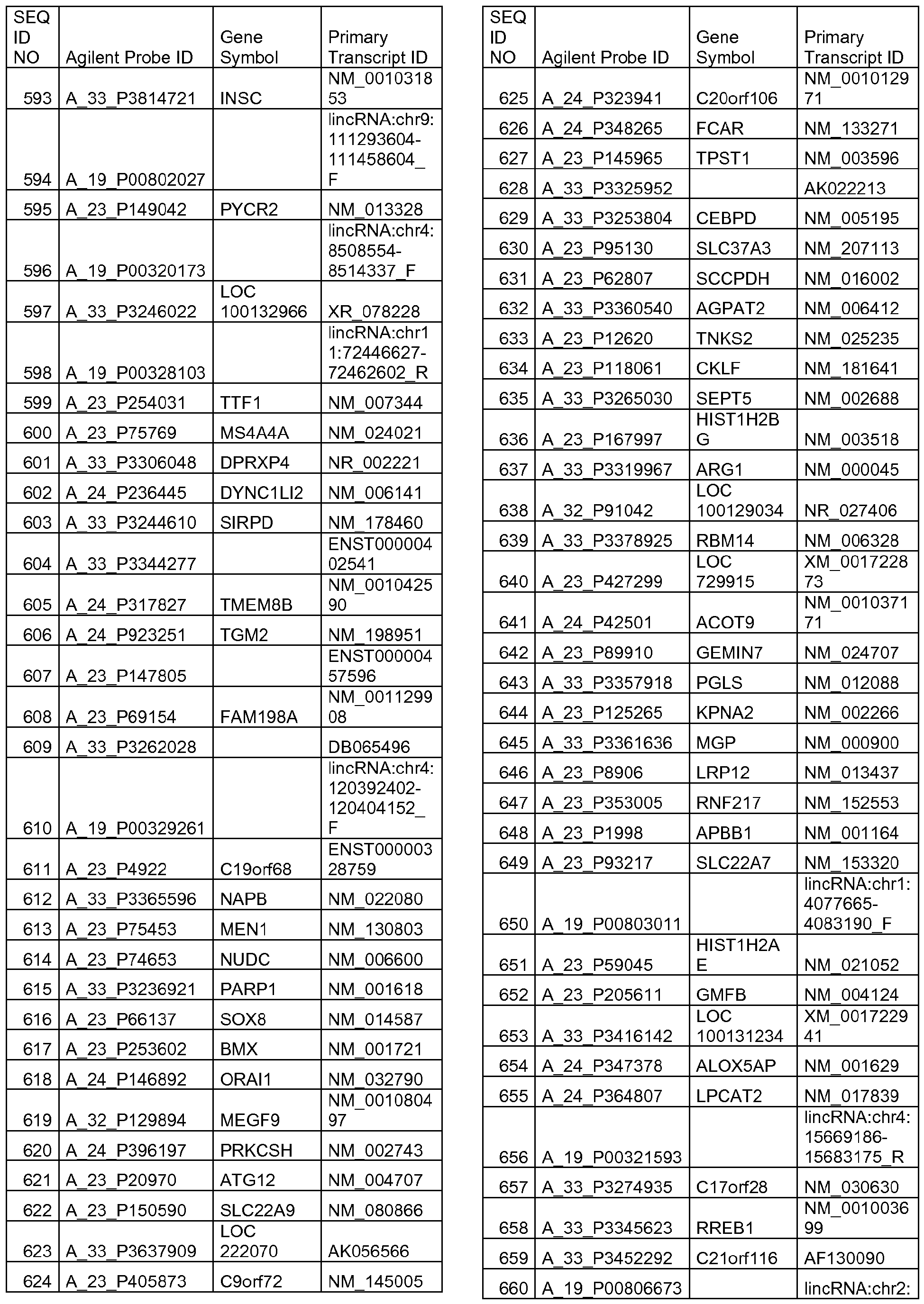

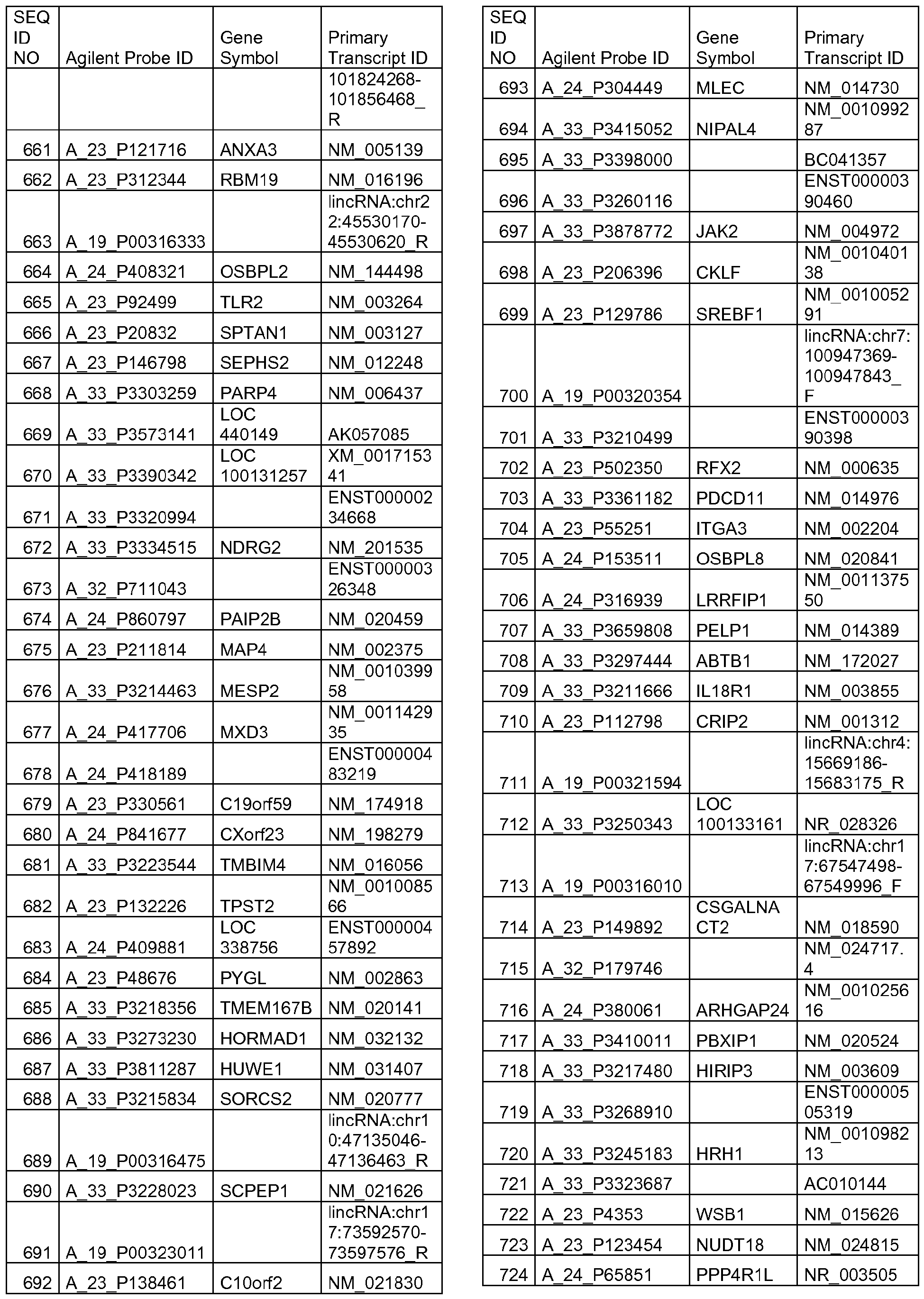

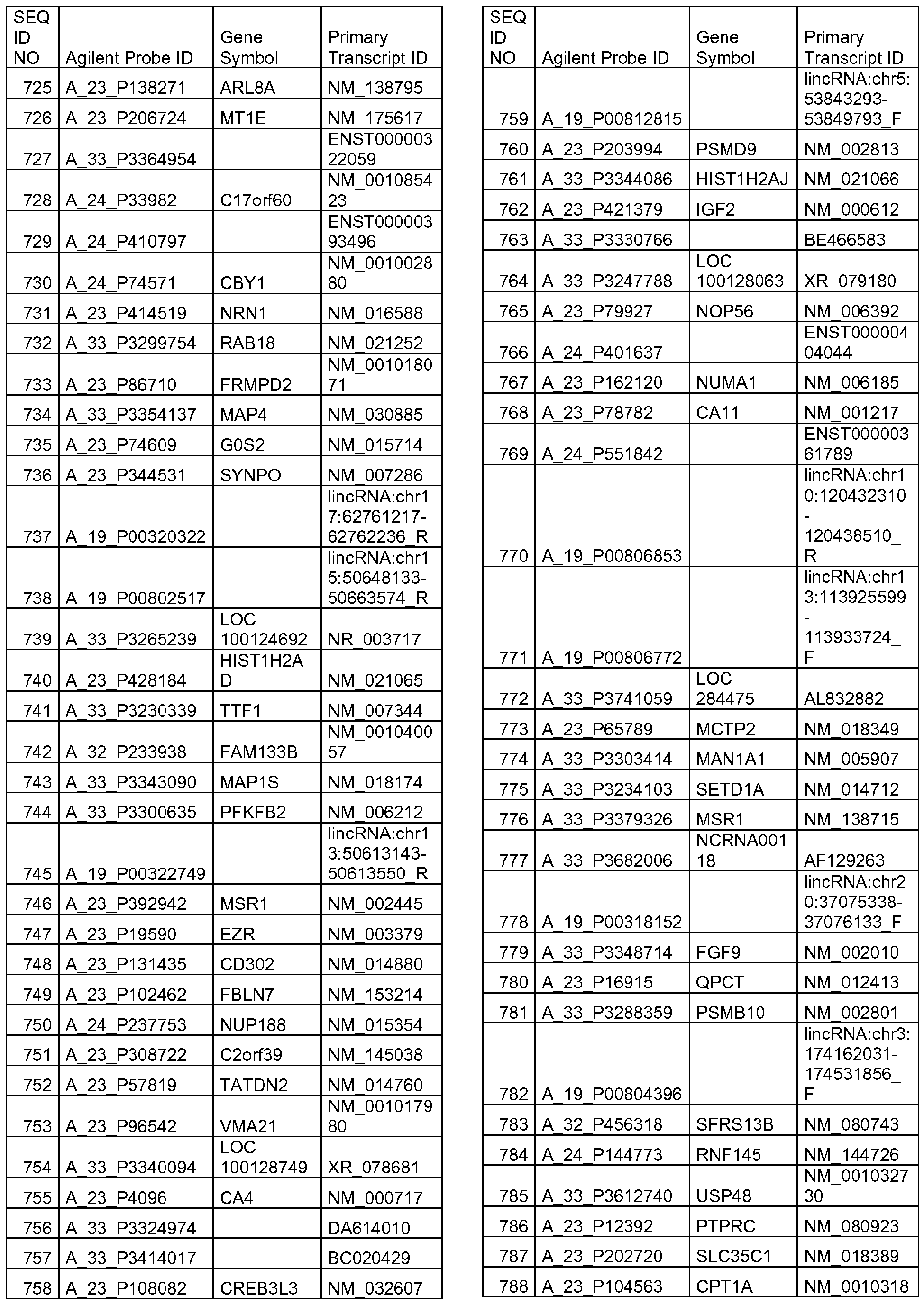

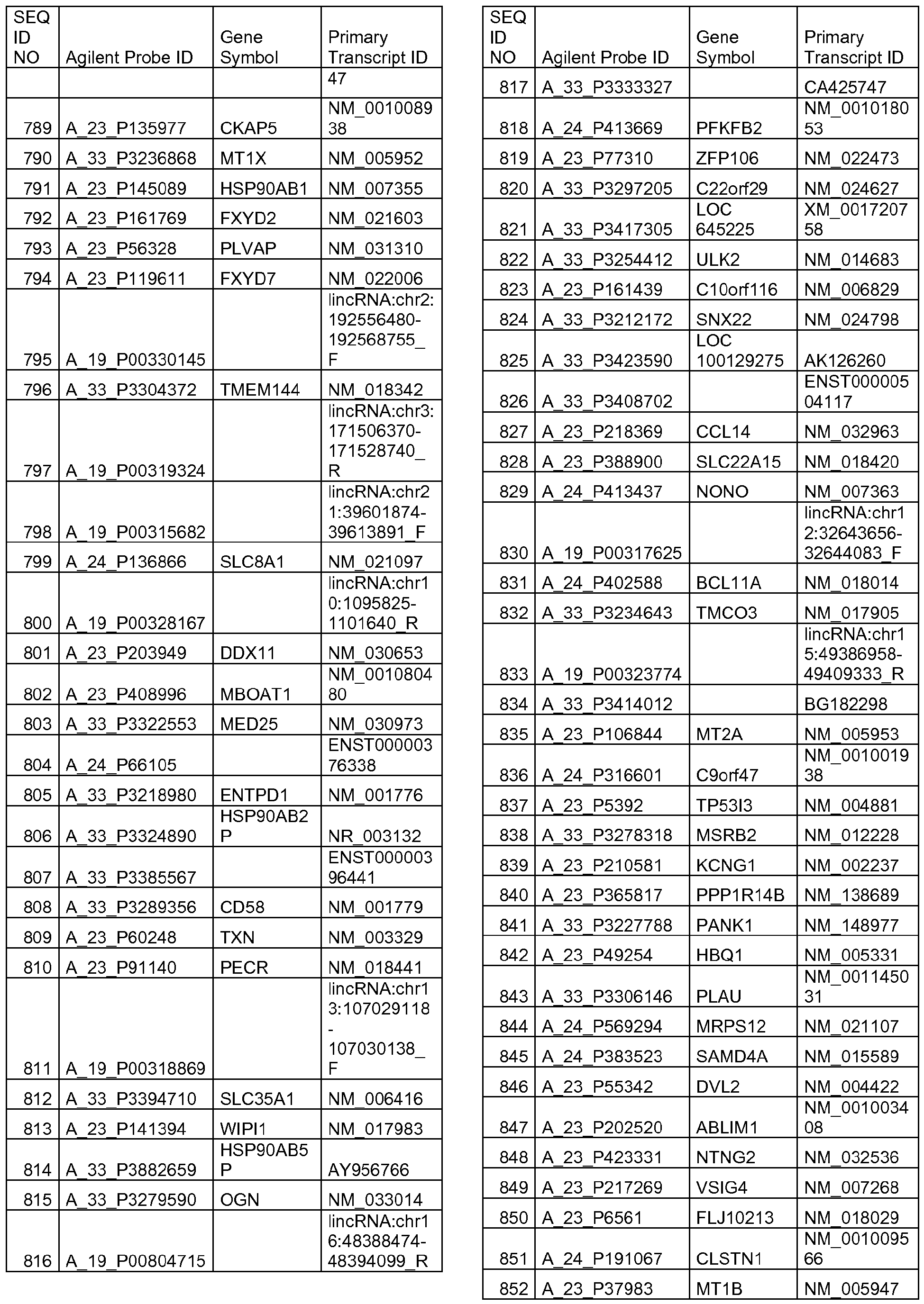

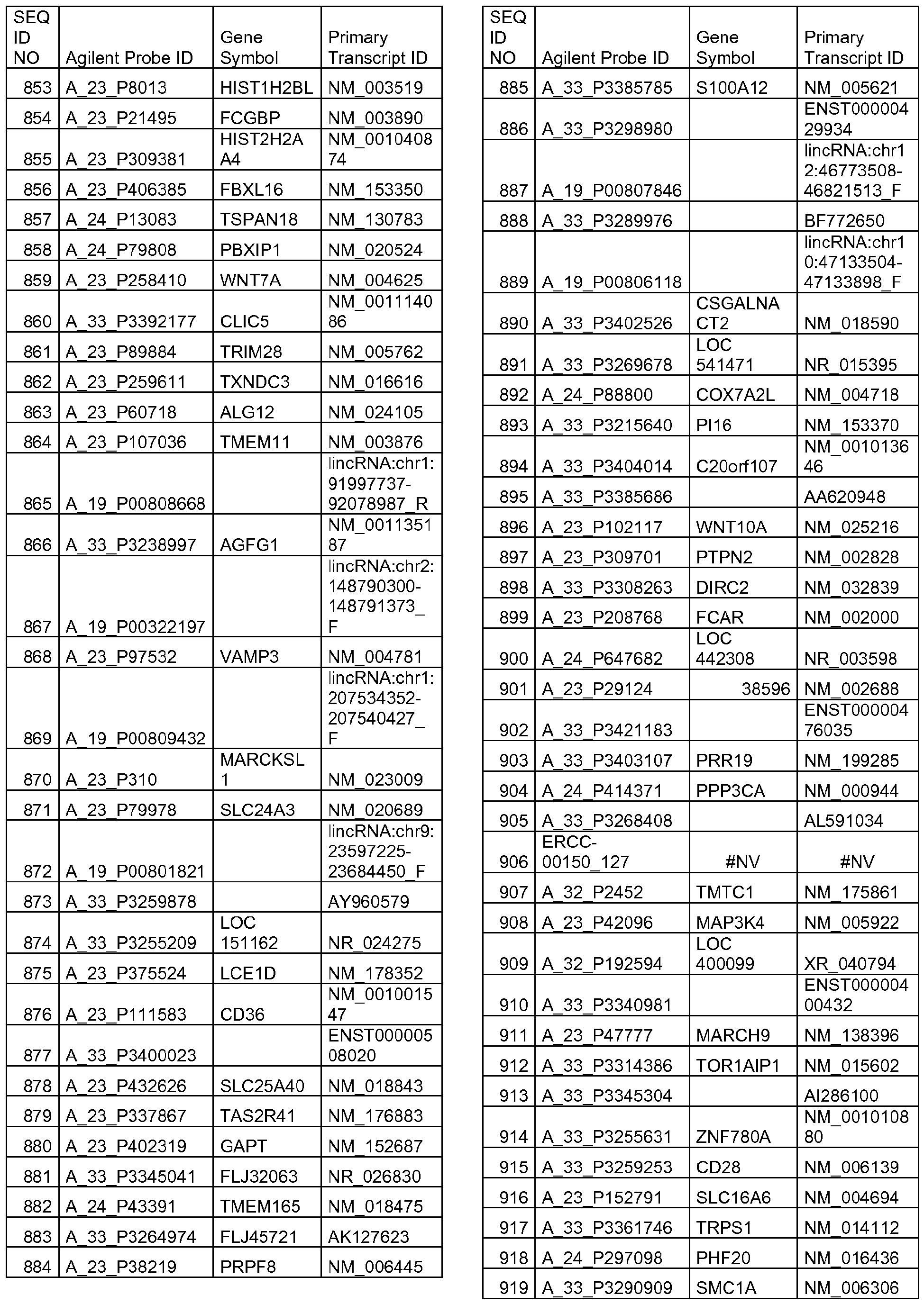

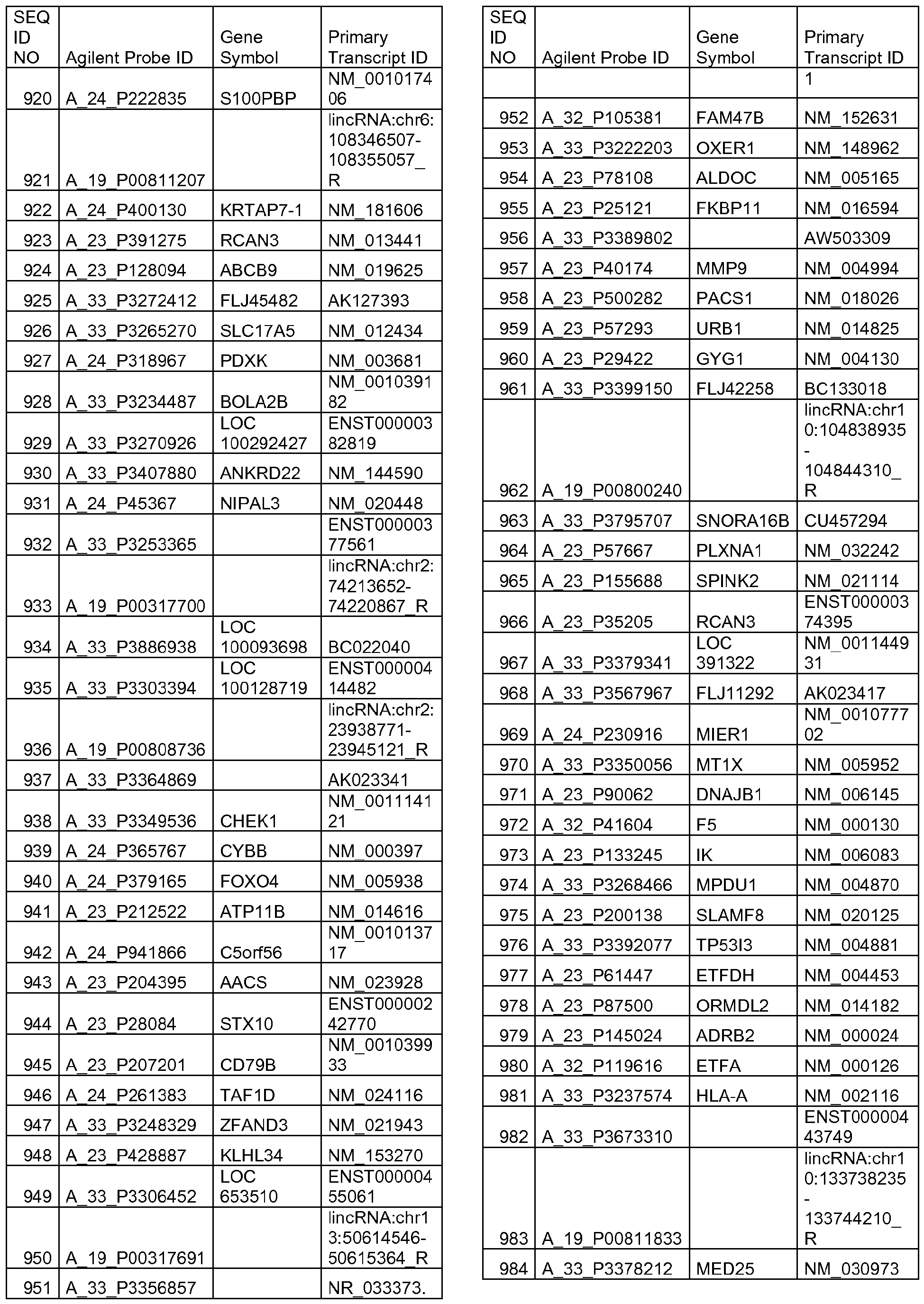

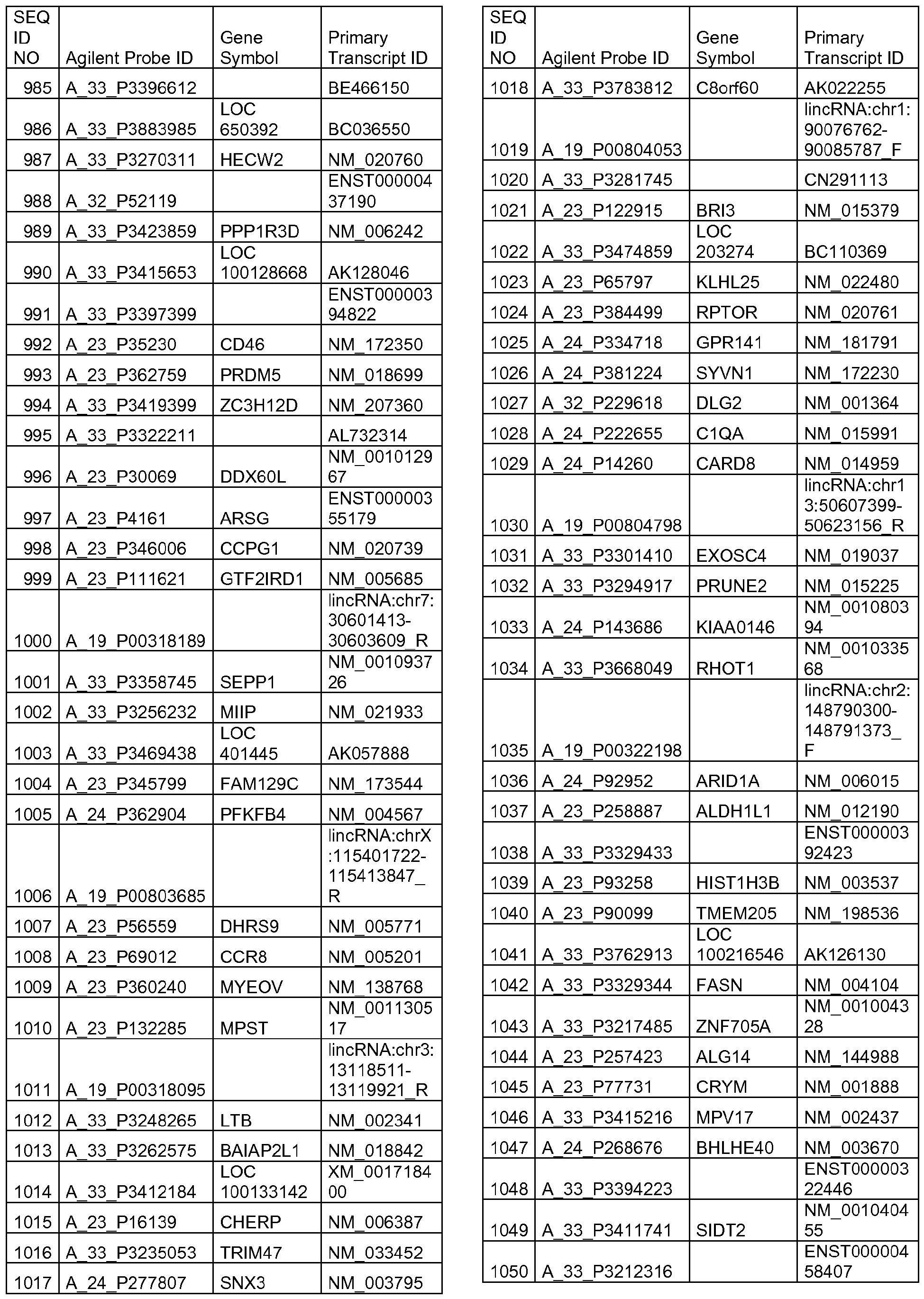

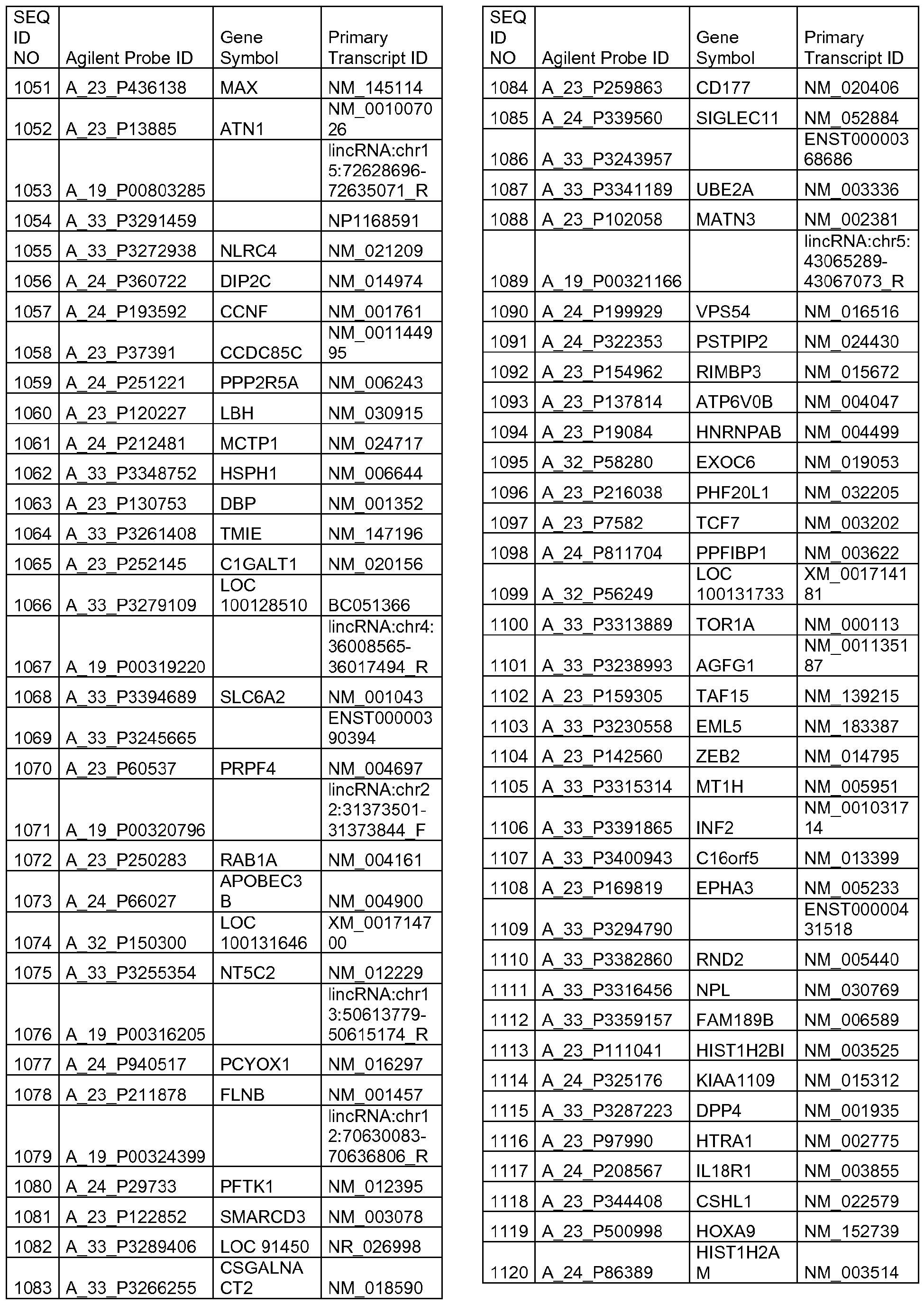

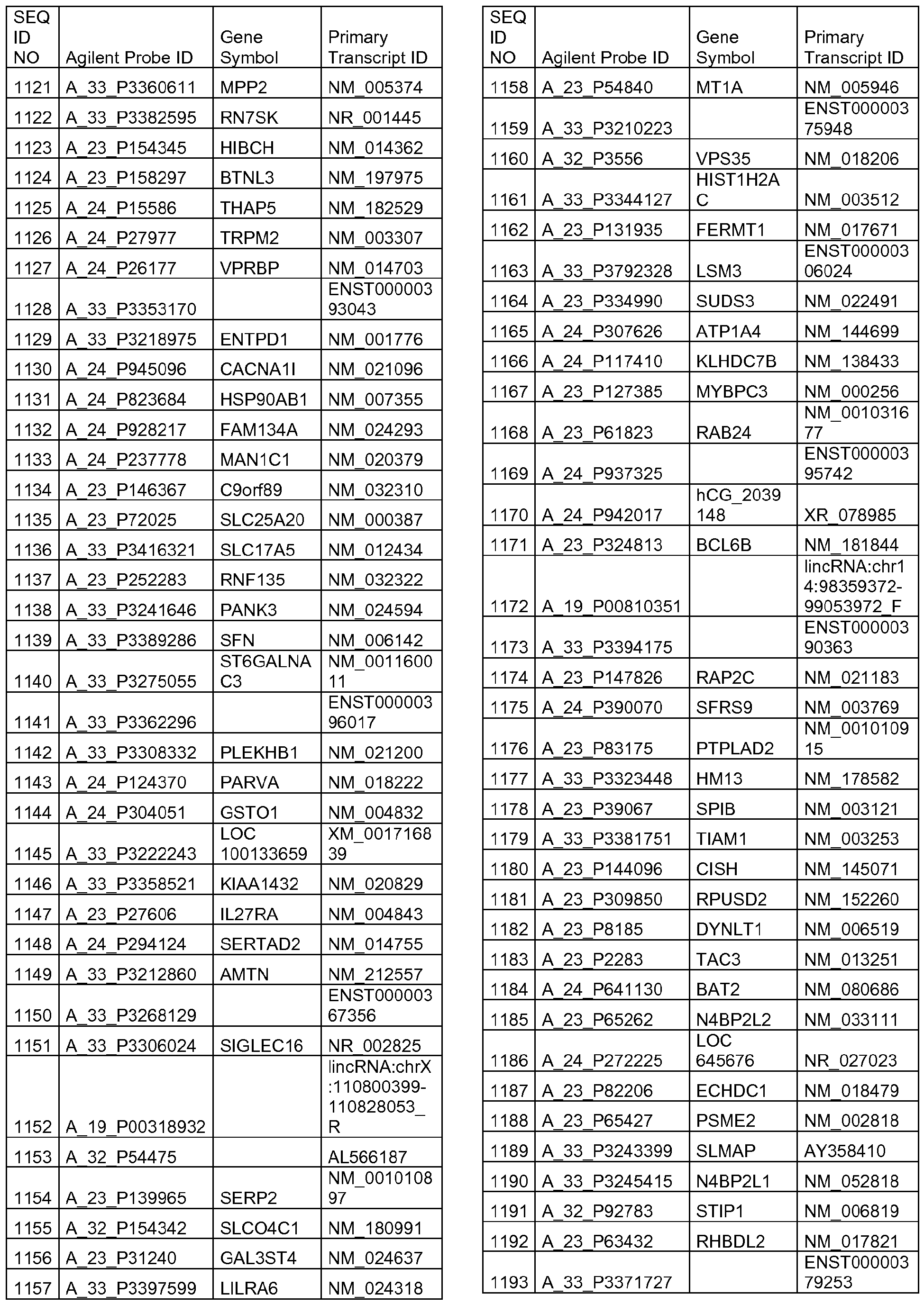

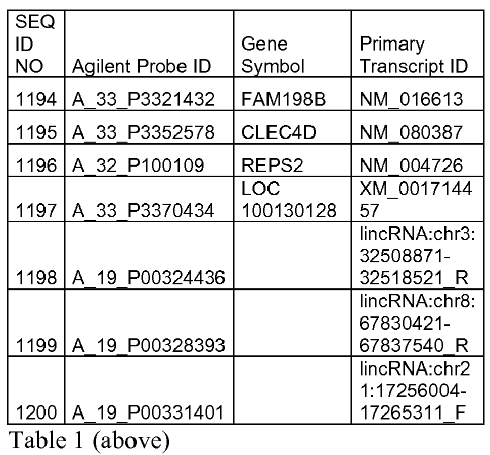

| SEQ ID NO | Log-2 Fold Change | Non-Log Fold Change | | SEQ ID NO | Log-2 Fold Change | Non-Log Fold Change | | SEQ ID NO | Log-2 Fold Change | Non-Log Fold Change |
|---|---|---|---|---|---|---|---|---|---|---|
| 1 | 2.02 | 406% | | 41 | -0.19 | 88% | | 81 | 0.52 | 143% |
| 2 | -0.36 | 78% | | 42 | -0.73 | 60% | | 82 | -0.21 | 86% |
| 3 | -0.32 | 80% | | 43 | 0.51 | 143% | | 83 | 0.31 | 124% |
| 4 | 0.54 | 146% | | 44 | -0.09 | 94% | | 84 | 0.37 | 129% |
| 5 | 0.74 | 168% | | 45 | -0.51 | 70% | | 85 | 0.95 | 193% |
| 6 | -0.33 | 80% | | 46 | -0.40 | 76% | | 86 | 0.43 | 134% |
| 7 | 0.40 | 132% | | 47 | 0.73 | 166% | | 87 | 0.31 | 124% |
| 8 | -0.50 | 71% | | 48 | -0.22 | 86% | | 88 | 0.48 | 139% |
| 9 | 0.50 | 142% | | 49 | -0.37 | 77% | | 89 | 0.30 | 123% |
| 10 | -0.27 | 83% | | 50 | 0.28 | 122% | | 90 | 0.48 | 139% |
| 11 | -0.63 | 65% | | 51 | 0.51 | 142% | | 91 | 0.27 | 121% |
| 12 | 0.34 | 126% | | 52 | 0.22 | 116% | | 92 | -0.44 | 74% |
| 13 | -0.40 | 76% | | 53 | -0.74 | 60% | | 93 | 0.43 | 135% |
| 14 | 0.58 | 150% | | 54 | -0.50 | 71% | | 94 | 1.66 | 317% |
| 15 | 0.50 | 141% | | 55 | 0.42 | 134% | | 95 | -0.23 | 85% |
| 16 | 0.49 | 141% | | 56 | -0.62 | 65% | | 96 | 0.13 | 109% |
| 17 | 0.51 | 142% | | 57 | -0.43 | 74% | | 97 | 0.32 | 125% |
| 18 | -0.31 | 81% | | 58 | 0.28 | 121% | | 98 | 0.32 | 125% |
| 19 | 0.47 | 139% | | 59 | 0.31 | 124% | | 99 | 0.21 | 115% |
| 20 | 0.50 | 142% | | 60 | -0.09 | 94% | | 100 | -0.52 | 70% |
| 21 | 0.47 | 139% | | 61 | 0.72 | 165% | | 101 | -0.28 | 82% |
| 22 | -0.27 | 83% | | 62 | -0.27 | 83% | | 102 | -0.25 | 84% |
| 23 | -0.30 | 81% | | 63 | -0.65 | 64% | | 103 | 0.86 | 181% |
| 24 | 1.40 | 263% | | 64 | -0.34 | 79% | | 104 | 0.72 | 165% |
| 25 | 0.61 | 153% | | 65 | 0.58 | 149% | | 105 | 0.40 | 132% |
| 26 | 0.21 | 115% | | 66 | -0.61 | 66% | | 106 | 0.43 | 135% |
| 27 | 0.43 | 135% | | 67 | 0.37 | 129% | | 107 | -0.27 | 83% |
| 28 | 1.02 | 203% | | 68 | 0.48 | 140% | | 108 | -0.57 | 67% |
| 29 | -0.47 | 72% | | 69 | 0.28 | 122% | | 109 | 0.36 | 129% |
| 30 | -0.48 | 72% | | 70 | -0.19 | 88% | | 110 | 0.24 | 118% |
| 31 | 0.27 | 121% | | 71 | 1.10 | 215% | | 111 | 0.49 | 140% |
| 32 | 0.54 | 146% | | 72 | 0.37 | 129% | | 112 | 0.77 | 170% |
| 33 | 0.32 | 125% | | 73 | 0.33 | 125% | | 113 | 0.66 | 158% |
| 34 | -0.41 | 75% | | 74 | -0.47 | 72% | | 114 | -0.28 | 82% |
| 35 | 0.67 | 159% | | 75 | -0.50 | 71% | | 115 | -0.42 | 75% |
| 36 | 0.54 | 146% | | 76 | 0.45 | 136% | | 116 | -0.64 | 64% |
| 37 | 0.43 | 135% | | 77 | 0.31 | 124% | | 117 | 0.29 | 122% |
| 38 | -0.34 | 79% | | 78 | -0.10 | 94% | | 118 | 0.20 | 115% |
| 39 | -0.85 | 56% | | 79 | 0.31 | 124% | | 119 | 0.60 | 151% |
| 40 | -0.32 | 80% | | 80 | -0.45 | 73% | | 120 | 0.30 | 123% |
| 121 | -0.34 | 79% | | 161 | -0.39 | 76% | | 201 | 0.45 | 136% |
| 122 | 0.89 | 185% | | 162 | 0.41 | 133% | | 202 | -0.37 | 77% |
| 123 | -0.33 | 80% | | 163 | -0.47 | 72% | | 203 | -0.50 | 71% |
| 124 | -0.21 | 87% | | 164 | -0.58 | 67% | | 204 | 0.42 | 134% |
| 125 | 0.94 | 191% | | 165 | -0.54 | 69% | | 205 | -1.78 | 29% |
| 126 | 0.24 | 118% | | 166 | 0.46 | 137% | | 206 | 1.79 | 346% |
| 127 | -0.56 | 68% | | 167 | 0.49 | 141% | | 207 | 0.70 | 162% |
| 128 | 0.37 | 129% | | 168 | 0.40 | 132% | | 208 | -0.19 | 88% |
| 129 | 0.72 | 165% | | 169 | 0.27 | 121% | | 209 | -0.12 | 92% |
| 130 | 0.32 | 125% | | 170 | -2.33 | 20% | | 210 | 0.49 | 140% |
| 131 | -0.18 | 88% | | 171 | -0.51 | 70% | | 211 | -0.54 | 69% |
| 132 | 0.31 | 124% | | 172 | 0.69 | 161% | | 212 | 1.17 | 226% |
| 133 | 0.56 | 147% | | 173 | 0.44 | 135% | | 213 | 0.92 | 190% |
| 134 | -0.27 | 83% | | 174 | 0.47 | 139% | | 214 | -0.28 | 83% |
| 135 | -0.48 | 72% | | 175 | 0.44 | 135% | | 215 | 0.39 | 131% |
| 136 | 0.23 | 118% | | 176 | 0.30 | 123% | | 216 | 0.33 | 126% |
| 137 | 0.73 | 166% | | 177 | 0.52 | 144% | | 217 | 0.28 | 122% |
| 138 | -0.29 | 82% | | 178 | 0.80 | 174% | | 218 | -0.29 | 82% |
| 139 | 0.28 | 121% | | 179 | 0.47 | 139% | | 219 | 0.34 | 126% |
| 140 | 0.97 | 195% | | 180 | 0.34 | 127% | | 220 | 0.51 | 142% |
| 141 | 0.28 | 121% | | 181 | -0.33 | 80% | | 221 | -0.34 | 79% |
| 142 | 0.46 | 138% | | 182 | 0.40 | 132% | | 222 | -0.16 | 89% |
| 143 | 0.30 | 123% | | 183 | 1.59 | 301% | | 223 | -0.39 | 76% |
| 144 | 0.69 | 162% | | 184 | -0.31 | 81% | | 224 | 0.38 | 130% |
| 145 | 0.53 | 144% | | 185 | -0.43 | 74% | | 225 | -0.25 | 84% |
| 146 | 0.56 | 147% | | 186 | 0.78 | 171% | | 226 | 0.22 | 117% |
| 147 | 2.98 | 791% | | 187 | 0.44 | 136% | | 227 | 1.16 | 223% |
| 148 | -0.25 | 84% | | 188 | -0.83 | 56% | | 228 | 0.24 | 118% |
| 149 | -0.46 | 73% | | 189 | 0.70 | 163% | | 229 | -0.64 | 64% |
| 150 | 0.41 | 133% | | 190 | 1.02 | 203% | | 230 | 0.27 | 121% |
| 151 | -0.42 | 75% | | 191 | 0.62 | 153% | | 231 | 0.70 | 163% |
| 152 | 0.34 | 126% | | 192 | 0.48 | 139% | | 232 | 0.41 | 133% |
| 153 | -0.23 | 85% | | 193 | 0.77 | 171% | | 233 | 0.63 | 155% |
| 154 | 0.30 | 123% | | 194 | 0.53 | 144% | | 234 | -0.14 | 91% |
| 155 | 1.06 | 209% | | 195 | 0.52 | 143% | | 235 | 0.49 | 140% |
| 156 | 0.72 | 165% | | 196 | -0.29 | 82% | | 236 | 0.36 | 128% |
| 157 | 0.44 | 136% | | 197 | 0.37 | 129% | | 237 | 0.48 | 140% |
| 158 | 0.47 | 139% | | 198 | 0.79 | 173% | | 238 | 0.43 | 135% |
| 159 | 0.37 | 129% | | 199 | -0.25 | 84% | | 239 | -0.43 | 74% |
| 160 | -0.44 | 74% | | 200 | 0.31 | 124% | | 240 | 0.26 | 120% |
| 241 | 0.23 | 118% | | 281 | -0.53 | 69% | | 321 | 0.80 | 174% |
| 242 | -0.28 | 82% | | 282 | -1.01 | 50% | | 322 | 0.23 | 117% |
| 243 | 0.34 | 127% | | 283 | -0.24 | 85% | | 323 | -0.51 | 70% |
| 244 | 1.59 | 300% | | 284 | -0.29 | 82% | | 324 | 0.62 | 154% |
| 245 | -0.40 | 76% | | 285 | -0.24 | 85% | | 325 | -0.38 | 77% |
| 246 | -0.32 | 80% | | 286 | 0.38 | 130% | | 326 | 0.43 | 135% |
| 247 | -0.37 | 77% | | 287 | 0.62 | 153% | | 327 | 0.53 | 144% |
| 248 | -0.35 | 78% | | 288 | -0.30 | 81% | | 328 | 0.61 | 153% |
| 249 | 0.38 | 130% | | 289 | 0.89 | 186% | | 329 | 0.30 | 123% |
| 250 | 0.28 | 122% | | 290 | -0.49 | 71% | | 330 | -0.33 | 80% |
| 251 | 0.52 | 144% | | 291 | -0.75 | 59% | | 331 | 0.23 | 117% |
| 252 | 0.48 | 140% | | 292 | 0.24 | 118% | | 332 | 0.35 | 127% |
| 253 | -0.29 | 82% | | 293 | -0.30 | 81% | | 333 | 0.36 | 129% |
| 254 | -0.71 | 61% | | 294 | -0.25 | 84% | | 334 | -0.34 | 79% |
| 255 | 0.86 | 181% | | 295 | 0.38 | 130% | | 335 | 0.45 | 137% |
| 256 | 0.53 | 145% | | 296 | 0.77 | 171% | | 336 | -0.35 | 78% |
| 257 | 0.07 | 105% | | 297 | -0.45 | 73% | | 337 | -0.42 | 75% |
| 258 | -0.28 | 82% | | 298 | 0.42 | 134% | | 338 | -0.30 | 81% |
| 259 | -0.37 | 77% | | 299 | -0.66 | 63% | | 339 | 0.19 | 114% |
| 260 | 0.57 | 148% | | 300 | 0.30 | 123% | | 340 | 0.45 | 137% |
| 261 | 0.42 | 133% | | 301 | 0.24 | 118% | | 341 | -0.38 | 77% |
| 262 | -0.27 | 83% | | 302 | 1.32 | 249% | | 342 | -0.26 | 84% |
| 263 | 0.29 | 122% | | 303 | -0.55 | 68% | | 343 | 0.43 | 135% |
| 264 | -0.55 | 68% | | 304 | 0.46 | 137% | | 344 | 0.38 | 130% |
| 265 | 0.48 | 140% | | 305 | 0.43 | 135% | | 345 | 0.23 | 117% |
| 266 | -0.31 | 81% | | 306 | 0.57 | 148% | | 346 | -0.40 | 76% |
| 267 | 0.55 | 146% | | 307 | 0.53 | 145% | | 347 | 0.76 | 169% |
| 268 | 0.66 | 158% | | 308 | 0.35 | 127% | | 348 | 0.57 | 149% |
| 269 | 0.78 | 171% | | 309 | -0.38 | 77% | | 349 | 0.83 | 177% |
| 270 | -0.23 | 85% | | 310 | 0.48 | 139% | | 350 | 0.64 | 156% |
| 271 | 0.84 | 178% | | 311 | -0.18 | 88% | | 351 | 0.46 | 138% |
| 272 | 0.63 | 155% | | 312 | -0.30 | 81% | | 352 | -0.43 | 74% |
| 273 | 0.09 | 106% | | 313 | 0.46 | 138% | | 353 | 0.44 | 136% |
| 274 | 0.01 | 101% | | 314 | -0.49 | 71% | | 354 | 0.86 | 182% |
| 275 | 0.29 | 122% | | 315 | 0.68 | 161% | | 355 | -0.76 | 59% |
| 276 | 0.41 | 133% | | 316 | -0.28 | 82% | | 356 | -0.31 | 81% |
| 277 | -0.15 | 90% | | 317 | -0.50 | 71% | | 357 | -0.55 | 68% |
| 278 | 0.51 | 143% | | 318 | 0.38 | 130% | | 358 | -0.51 | 70% |
| 279 | -0.28 | 82% | | 319 | 0.60 | 152% | | 359 | 0.30 | 123% |
| 280 | 0.27 | 120% | | 320 | 0.43 | 135% | | 360 | -0.24 | 85% |
| 361 | 0.21 | 116% | | 401 | -0.38 | 77% | | 441 | 0.54 | 146% |
| 362 | 0.36 | 129% | | 402 | 0.69 | 161% | | 442 | 0.29 | 122% |
| 363 | 0.66 | 158% | | 403 | 0.48 | 139% | | 443 | -0.26 | 84% |
| 364 | 0.20 | 115% | | 404 | -0.54 | 69% | | 444 | -0.25 | 84% |
| 365 | 0.30 | 123% | | 405 | 0.23 | 117% | | 445 | 0.30 | 123% |
| 366 | 1.73 | 333% | | 406 | -0.25 | 84% | | 446 | 0.75 | 168% |
| 367 | 0.57 | 149% | | 407 | 0.17 | 113% | | 447 | -0.20 | 87% |
| 368 | 0.34 | 127% | | 408 | 0.46 | 138% | | 448 | 0.32 | 125% |
| 369 | 0.39 | 131% | | 409 | -0.52 | 70% | | 449 | -0.23 | 85% |
| 370 | 0.21 | 116% | | 410 | 0.60 | 152% | | 450 | 0.63 | 155% |
| 371 | 0.33 | 125% | | 411 | 0.27 | 121% | | 451 | -0.40 | 76% |
| 372 | 0.12 | 108% | | 412 | -1.06 | 48% | | 452 | 0.64 | 156% |
| 373 | 0.60 | 151% | | 413 | 0.54 | 145% | | 453 | 0.49 | 140% |
| 374 | -0.38 | 77% | | 414 | 3.50 | 1128% | | 454 | 0.14 | 110% |
| 375 | 0.40 | 132% | | 415 | 0.31 | 124% | | 455 | -0.25 | 84% |
| 376 | -0.42 | 75% | | 416 | 0.44 | 135% | | 456 | 0.30 | 123% |
| 377 | 0.36 | 128% | | 417 | -0.48 | 72% | | 457 | 0.45 | 137% |
| 378 | -0.31 | 81% | | 418 | -0.45 | 73% | | 458 | 0.82 | 176% |
| 379 | -0.62 | 65% | | 419 | 0.93 | 191% | | 459 | 0.39 | 131% |
| 380 | 0.68 | 160% | | 420 | 0.39 | 131% | | 460 | 0.54 | 146% |
| 381 | 0.69 | 162% | | 421 | 0.28 | 122% | | 461 | -0.61 | 66% |
| 382 | 0.35 | 127% | | 422 | 0.24 | 118% | | 462 | 0.19 | 114% |
| 383 | 0.53 | 144% | | 423 | 0.42 | 133% | | 463 | -0.61 | 66% |
| 384 | 0.08 | 105% | | 424 | -0.53 | 69% | | 464 | 0.45 | 137% |
| 385 | 0.37 | 129% | | 425 | -0.28 | 82% | | 465 | -0.46 | 73% |
| 386 | -0.27 | 83% | | 426 | -0.42 | 75% | | 466 | 0.52 | 143% |
| 387 | 0.43 | 134% | | 427 | -0.34 | 79% | | 467 | -0.22 | 86% |
| 388 | 0.47 | 139% | | 428 | 0.25 | 119% | | 468 | 0.25 | 119% |
| 389 | -0.35 | 78% | | 429 | 0.23 | 117% | | 469 | 1.06 | 209% |
| 390 | 0.62 | 154% | | 430 | 0.35 | 127% | | 470 | 0.35 | 127% |
| 391 | 0.46 | 137% | | 431 | 0.49 | 141% | | 471 | 0.37 | 129% |
| 392 | -0.27 | 83% | | 432 | 0.50 | 142% | | 472 | 0.64 | 155% |
| 393 | 0.47 | 138% | | 433 | 0.63 | 155% | | 473 | 0.40 | 132% |
| 394 | 0.73 | 166% | | 434 | 0.49 | 140% | | 474 | -0.45 | 73% |
| 395 | 0.58 | 150% | | 435 | -0.33 | 79% | | 475 | -0.62 | 65% |
| 396 | 0.74 | 167% | | 436 | -0.33 | 80% | | 476 | 0.30 | 123% |
| 397 | 0.40 | 132% | | 437 | -0.42 | 74% | | 477 | -0.32 | 80% |
| 398 | -0.54 | 69% | | 438 | -0.29 | 82% | | 478 | -0.31 | 81% |
| 399 | 0.36 | 128% | | 439 | -0.48 | 71% | | 479 | 0.81 | 176% |
| 400 | 0.50 | 141% | | 440 | 0.70 | 163% | | 480 | 0.05 | 103% |
| 481 | -0.26 | 83% | | 521 | 0.30 | 123% | | 561 | 0.70 | 162% |
| 482 | 0.31 | 124% | | 522 | 0.25 | 119% | | 562 | 0.38 | 130% |
| 483 | 0.36 | 129% | | 523 | -0.41 | 75% | | 563 | -0.21 | 86% |
| 484 | -0.30 | 81% | | 524 | -0.62 | 65% | | 564 | -0.46 | 72% |
| 485 | -0.24 | 85% | | 525 | 0.45 | 137% | | 565 | 0.55 | 147% |
| 486 | 0.64 | 156% | | 526 | 0.50 | 141% | | 566 | -0.36 | 78% |
| 487 | 0.39 | 131% | | 527 | -0.49 | 71% | | 567 | 0.38 | 131% |
| 488 | 0.51 | 143% | | 528 | 0.24 | 118% | | 568 | -0.35 | 78% |
| 489 | 0.62 | 154% | | 529 | 0.68 | 161% | | 569 | 0.39 | 131% |
| 490 | 0.46 | 137% | | 530 | 0.64 | 156% | | 570 | -0.37 | 78% |
| 491 | 0.69 | 161% | | 531 | 0.19 | 114% | | 571 | -0.45 | 73% |
| 492 | 0.54 | 146% | | 532 | 0.47 | 138% | | 572 | -0.70 | 62% |
| 493 | 0.64 | 156% | | 533 | -0.41 | 75% | | 573 | 0.29 | 122% |
| 494 | -0.26 | 83% | | 534 | 1.31 | 247% | | 574 | 0.26 | 120% |
| 495 | 0.26 | 119% | | 535 | 0.11 | 108% | | 575 | -0.21 | 87% |
| 496 | 0.17 | 113% | | 536 | 0.32 | 124% | | 576 | 1.65 | 315% |
| 497 | 0.61 | 152% | | 537 | 0.42 | 134% | | 577 | 0.39 | 131% |
| 498 | 0.38 | 131% | | 538 | 0.55 | 147% | | 578 | 0.95 | 193% |
| 499 | 0.78 | 172% | | 539 | 0.36 | 128% | | 579 | -0.41 | 75% |
| 500 | 0.36 | 128% | | 540 | 0.52 | 144% | | 580 | 0.59 | 151% |
| 501 | 0.36 | 128% | | 541 | 0.99 | 199% | | 581 | -0.33 | 79% |
| 502 | -0.56 | 68% | | 542 | 0.85 | 181% | | 582 | 0.33 | 126% |
| 503 | -0.22 | 86% | | 543 | -0.47 | 72% | | 583 | -0.23 | 86% |
| 504 | 0.35 | 128% | | 544 | 0.31 | 124% | | 584 | 0.38 | 130% |
| 505 | 0.24 | 118% | | 545 | -0.33 | 80% | | 585 | 0.34 | 127% |
| 506 | -0.14 | 91% | | 546 | -0.45 | 73% | | 586 | 0.51 | 143% |
| 507 | 0.52 | 143% | | 547 | 0.43 | 135% | | 587 | -0.30 | 81% |
| 508 | 0.51 | 142% | | 548 | 1.09 | 212% | | 588 | -0.65 | 64% |
| 509 | 0.35 | 127% | | 549 | -0.43 | 74% | | 589 | 0.65 | 157% |
| 510 | -0.25 | 84% | | 550 | -0.33 | 79% | | 590 | -0.20 | 87% |
| 511 | 0.23 | 118% | | 551 | 0.38 | 130% | | 591 | -0.32 | 80% |
| 512 | 0.62 | 154% | | 552 | -0.57 | 67% | | 592 | -0.57 | 68% |
| 513 | 0.44 | 135% | | 553 | 0.29 | 122% | | 593 | 1.20 | 230% |
| 514 | -0.29 | 82% | | 554 | 0.52 | 144% | | 594 | -0.14 | 91% |
| 515 | 0.68 | 160% | | 555 | 0.33 | 125% | | 595 | -0.23 | 85% |
| 516 | 0.53 | 144% | | 556 | 5.66 | 5043% | | 596 | -1.52 | 35% |
| 517 | 0.06 | 104% | | 557 | -0.62 | 65% | | 597 | 0.67 | 159% |
| 518 | -0.38 | 77% | | 558 | 0.51 | 143% | | 598 | 0.33 | 126% |
| 519 | 0.52 | 144% | | 559 | -0.46 | 72% | | 599 | -0.23 | 85% |
| 520 | 0.71 | 163% | | 560 | -0.50 | 71% | | 600 | 0.62 | 154% |
| 601 | 0.41 | 133% | | 641 | 0.24 | 118% | | 681 | 0.33 | 125% |
| 602 | 0.36 | 128% | | 642 | 0.32 | 125% | | 682 | 0.28 | 122% |
| 603 | 0.60 | 151% | | 643 | 0.22 | 116% | | 683 | -0.37 | 78% |
| 604 | 0.48 | 140% | | 644 | 0.28 | 122% | | 684 | 0.54 | 145% |
| 605 | -0.24 | 85% | | 645 | 0.97 | 196% | | 685 | 0.27 | 121% |
| 606 | -0.69 | 62% | | 646 | 0.43 | 135% | | 686 | 0.65 | 157% |
| 607 | 0.40 | 132% | | 647 | 0.46 | 138% | | 687 | -0.27 | 83% |
| 608 | -0.44 | 74% | | 648 | -0.45 | 73% | | 688 | 1.12 | 217% |
| 609 | -0.59 | 66% | | 649 | 0.44 | 135% | | 689 | -1.03 | 49% |
| 610 | 0.38 | 130% | | 650 | 0.21 | 116% | | 690 | 0.37 | 129% |
| 611 | -0.34 | 79% | | 651 | 0.24 | 118% | | 691 | 0.79 | 173% |
| 612 | 0.35 | 128% | | 652 | 0.35 | 127% | | 692 | -0.39 | 76% |
| 613 | -0.32 | 80% | | 653 | 0.53 | 144% | | 693 | -0.24 | 85% |
| 614 | -0.27 | 83% | | 654 | 0.61 | 153% | | 694 | -0.47 | 72% |
| 615 | -0.32 | 80% | | 655 | 0.80 | 174% | | 695 | 0.44 | 135% |
| 616 | -0.43 | 74% | | 656 | 0.54 | 146% | | 696 | -0.30 | 81% |
| 617 | 1.21 | 232% | | 657 | -0.40 | 76% | | 697 | 0.93 | 190% |
| 618 | -0.25 | 84% | | 658 | 0.20 | 115% | | 698 | 0.52 | 143% |
| 619 | 0.62 | 154% | | 659 | 0.22 | 117% | | 699 | -0.28 | 82% |
| 620 | -0.33 | 79% | | 660 | 0.60 | 151% | | 700 | -0.43 | 74% |
| 621 | 0.34 | 126% | | 661 | 0.92 | 189% | | 701 | -0.31 | 80% |
| 622 | 0.31 | 124% | | 662 | -0.31 | 81% | | 702 | -0.30 | 81% |
| 623 | -0.36 | 78% | | 663 | 0.42 | 134% | | 703 | -0.29 | 82% |
| 624 | 0.72 | 165% | | 664 | 0.29 | 122% | | 704 | -0.33 | 79% |
| 625 | 0.47 | 138% | | 665 | 0.50 | 141% | | 705 | 0.57 | 148% |
| 626 | 1.11 | 216% | | 666 | -0.40 | 76% | | 706 | 0.40 | 132% |
| 627 | 0.93 | 190% | | 667 | 0.25 | 119% | | 707 | -0.34 | 79% |
| 628 | 0.39 | 131% | | 668 | 0.32 | 125% | | 708 | 0.31 | 124% |
| 629 | -0.21 | 87% | | 669 | 0.28 | 122% | | 709 | 1.03 | 204% |
| 630 | 1.20 | 229% | | 670 | 0.38 | 130% | | 710 | -0.65 | 64% |
| 631 | 0.40 | 132% | | 671 | -0.41 | 75% | | 711 | 0.73 | 166% |
| 632 | 0.36 | 128% | | 672 | -0.40 | 76% | | 712 | -0.12 | 92% |
| 633 | 0.31 | 124% | | 673 | 0.46 | 137% | | 713 | 0.74 | 167% |
| 634 | 0.56 | 147% | | 674 | -0.43 | 74% | | 714 | 0.74 | 167% |
| 635 | 0.44 | 136% | | 675 | -0.28 | 82% | | 715 | 0.47 | 138% |
| 636 | 0.32 | 125% | | 676 | -0.38 | 77% | | 716 | 0.71 | 164% |
| 637 | 1.60 | 304% | | 677 | 0.53 | 144% | | 717 | -0.54 | 69% |
| 638 | 0.40 | 132% | | 678 | -0.39 | 76% | | 718 | -0.34 | 79% |
| 639 | -0.38 | 77% | | 679 | 0.91 | 188% | | 719 | -0.35 | 79% |
| 640 | 0.63 | 155% | | 680 | 0.27 | 121% | | 720 | -0.70 | 62% |
| 721 | 0.47 | 139% | | 761 | 0.37 | 129% | | 801 | -0.45 | 73% |
| 722 | 0.55 | 147% | | 762 | -0.46 | 73% | | 802 | 0.54 | 145% |
| 723 | 0.33 | 126% | | 763 | 0.21 | 116% | | 803 | -0.36 | 78% |
| 724 | 0.52 | 143% | | 764 | -0.31 | 80% | | 804 | 0.53 | 145% |
| 725 | 0.29 | 122% | | 765 | -0.38 | 77% | | 805 | 0.82 | 176% |
| 726 | 0.50 | 142% | | 766 | -0.17 | 89% | | 806 | -0.52 | 70% |
| 727 | 0.41 | 133% | | 767 | -0.41 | 75% | | 807 | 0.39 | 131% |
| 728 | 0.65 | 157% | | 768 | -0.34 | 79% | | 808 | 0.73 | 165% |
| 729 | -0.33 | 80% | | 769 | -0.36 | 78% | | 809 | 0.63 | 155% |
| 730 | -0.34 | 79% | | 770 | -0.11 | 93% | | 810 | 0.47 | 138% |
| 731 | 1.57 | 296% | | 771 | -0.42 | 75% | | 811 | -0.77 | 59% |
| 732 | 0.48 | 139% | | 772 | 0.47 | 138% | | 812 | 0.28 | 122% |
| 733 | -0.14 | 91% | | 773 | 0.53 | 144% | | 813 | 0.55 | 146% |
| 734 | -0.17 | 89% | | 774 | 0.54 | 145% | | 814 | -0.35 | 78% |
| 735 | 1.59 | 302% | | 775 | -0.32 | 80% | | 815 | -0.27 | 83% |
| 736 | -1.50 | 35% | | 776 | 0.72 | 165% | | 816 | 0.57 | 149% |
| 737 | 0.24 | 118% | | 777 | -0.61 | 65% | | 817 | 0.98 | 198% |
| 738 | -0.51 | 70% | | 778 | -0.35 | 79% | | 818 | 1.20 | 229% |
| 739 | 0.79 | 173% | | 779 | -0.37 | 77% | | 819 | 0.26 | 120% |
| 740 | 0.50 | 141% | | 780 | 0.59 | 151% | | 820 | -0.31 | 81% |
| 741 | -0.18 | 88% | | 781 | 0.23 | 118% | | 821 | 0.58 | 149% |
| 742 | 0.53 | 144% | | 782 | 0.40 | 132% | | 822 | -0.26 | 83% |
| 743 | -0.40 | 76% | | 783 | 0.08 | 106% | | 823 | 0.63 | 155% |
| 744 | 0.83 | 178% | | 784 | 0.42 | 134% | | 824 | -0.60 | 66% |
| 745 | 0.35 | 127% | | 785 | 0.52 | 143% | | 825 | 0.47 | 138% |
| 746 | 0.71 | 164% | | 786 | 0.49 | 141% | | 826 | 0.61 | 153% |
| 747 | -0.31 | 81% | | 787 | -0.27 | 83% | | 827 | 0.73 | 166% |
| 748 | 0.29 | 122% | | 788 | 0.43 | 135% | | 828 | 0.66 | 158% |
| 749 | -0.35 | 78% | | 789 | -0.30 | 81% | | 829 | -0.18 | 88% |
| 750 | -0.31 | 80% | | 790 | 0.26 | 119% | | 830 | 0.74 | 167% |
| 751 | 1.28 | 242% | | 791 | -0.49 | 71% | | 831 | -0.33 | 80% |
| 752 | -0.22 | 86% | | 792 | -0.42 | 75% | | 832 | 0.48 | 139% |
| 753 | 0.29 | 123% | | 793 | -0.70 | 61% | | 833 | 0.22 | 117% |
| 754 | 0.42 | 133% | | 794 | -0.42 | 75% | | 834 | 1.19 | 227% |
| 755 | 0.86 | 182% | | 795 | 0.62 | 154% | | 835 | 0.53 | 145% |
| 756 | -0.17 | 89% | | 796 | 0.57 | 148% | | 836 | 0.67 | 159% |
| 757 | -0.53 | 69% | | 797 | 0.43 | 135% | | 837 | 0.60 | 152% |
| 758 | 1.34 | 253% | | 798 | 0.66 | 158% | | 838 | 0.74 | 167% |
| 759 | 0.39 | 131% | | 799 | 0.50 | 141% | | 839 | -0.85 | 56% |
| 760 | 0.24 | 118% | | 800 | 0.75 | 168% | | 840 | 0.35 | 128% |
| 841 | -0.42 | 75% | | 881 | -0.31 | 81% | | 921 | 0.48 | 140% |
| 842 | -0.12 | 92% | | 882 | 0.38 | 130% | | 922 | 0.47 | 139% |
| 843 | 0.58 | 149% | | 883 | -0.19 | 88% | | 923 | -0.63 | 65% |
| 844 | 0.05 | 104% | | 884 | -0.29 | 82% | | 924 | 0.32 | 125% |
| 845 | 0.34 | 127% | | 885 | 1.08 | 212% | | 925 | 0.32 | 125% |
| 846 | -0.29 | 82% | | 886 | 0.47 | 139% | | 926 | 0.40 | 132% |
| 847 | -0.48 | 71% | | 887 | 0.47 | 138% | | 927 | 0.31 | 124% |
| 848 | 0.59 | 150% | | 888 | -0.20 | 87% | | 928 | 0.29 | 122% |
| 849 | 1.15 | 222% | | 889 | 0.33 | 126% | | 929 | 0.44 | 135% |
| 850 | 0.35 | 127% | | 890 | 0.68 | 161% | | 930 | 2.07 | 419% |
| 851 | -0.36 | 78% | | 891 | 0.27 | 120% | | 931 | -0.33 | 79% |
| 852 | 0.45 | 137% | | 892 | 0.27 | 120% | | 932 | 0.56 | 148% |
| 853 | 0.22 | 117% | | 893 | -0.53 | 69% | | 933 | -0.24 | 85% |
| 854 | -0.64 | 64% | | 894 | 0.56 | 147% | | 934 | -0.26 | 84% |
| 855 | 0.52 | 143% | | 895 | 0.42 | 134% | | 935 | -0.35 | 78% |
| 856 | -0.64 | 64% | | 896 | -0.54 | 69% | | 936 | 0.43 | 135% |
| 857 | -0.45 | 73% | | 897 | 0.43 | 135% | | 937 | 0.97 | 195% |
| 858 | -0.38 | 77% | | 898 | 0.59 | 151% | | 938 | 0.26 | 120% |
| 859 | -0.32 | 80% | | 899 | 0.94 | 192% | | 939 | 0.40 | 132% |
| 860 | 0.27 | 121% | | 900 | -0.30 | 81% | | 940 | -0.42 | 75% |
| 861 | -0.32 | 80% | | 901 | 0.47 | 138% | | 941 | 0.59 | 151% |
| 862 | 0.83 | 177% | | 902 | 0.38 | 130% | | 942 | 0.45 | 136% |
| 863 | -0.11 | 93% | | 903 | -0.37 | 77% | | 943 | -0.26 | 83% |
| 864 | 0.21 | 116% | | 904 | 0.41 | 133% | | 944 | 0.28 | 121% |
| 865 | -0.20 | 87% | | 905 | -0.24 | 85% | | 945 | -0.44 | 74% |
| 866 | 0.68 | 160% | | 906 | 0.07 | 105% | | 946 | 0.37 | 129% |
| 867 | 0.49 | 141% | | 907 | 1.19 | 228% | | 947 | 0.31 | 124% |
| 868 | 0.39 | 131% | | 908 | -0.40 | 76% | | 948 | -0.54 | 69% |
| 869 | 0.78 | 172% | | 909 | 0.45 | 137% | | 949 | 0.51 | 143% |
| 870 | -0.36 | 78% | | 910 | 0.27 | 121% | | 950 | 0.43 | 135% |
| 871 | 0.37 | 129% | | 911 | -0.29 | 82% | | 951 | 0.32 | 125% |
| 872 | -0.37 | 78% | | 912 | 0.52 | 143% | | 952 | 0.23 | 117% |
| 873 | 0.46 | 137% | | 913 | 0.34 | 127% | | 953 | 0.60 | 152% |
| 874 | -0.34 | 79% | | 914 | 0.38 | 131% | | 954 | -0.42 | 75% |
| 875 | -0.14 | 91% | | 915 | -0.37 | 78% | | 955 | 0.30 | 123% |
| 876 | 0.41 | 133% | | 916 | 0.51 | 143% | | 956 | 0.46 | 137% |
| 877 | 0.76 | 169% | | 917 | 0.27 | 121% | | 957 | 1.20 | 230% |
| 878 | 0.58 | 149% | | 918 | -0.16 | 89% | | 958 | -0.22 | 86% |
| 879 | -0.46 | 73% | | 919 | -0.66 | 63% | | 959 | -0.35 | 78% |
| 880 | 0.49 | 140% | | 920 | 0.30 | 123% | | 960 | 0.87 | 183% |
| 961 | 0.22 | 117% | | 1001 | 0.54 | 145% | | 1041 | 0.22 | 117% |
| 962 | 0.14 | 110% | | 1002 | 0.21 | 116% | | 1042 | -0.41 | 75% |
| 963 | 0.55 | 146% | | 1003 | -0.51 | 70% | | 1043 | -0.84 | 56% |
| 964 | -0.39 | 76% | | 1004 | -0.54 | 69% | | 1044 | 0.37 | 129% |
| 965 | -0.63 | 65% | | 1005 | 0.39 | 131% | | 1045 | 0.58 | 149% |
| 966 | -0.48 | 72% | | 1006 | 0.48 | 139% | | 1046 | 0.07 | 105% |
| 967 | 0.53 | 144% | | 1007 | 0.71 | 163% | | 1047 | -0.31 | 81% |
| 968 | 0.42 | 134% | | 1008 | -0.49 | 71% | | 1048 | -0.30 | 81% |
| 969 | 0.29 | 122% | | 1009 | -0.76 | 59% | | 1049 | 0.06 | 104% |
| 970 | 0.35 | 127% | | 1010 | 0.26 | 119% | | 1050 | 0.57 | 149% |
| 971 | -0.30 | 81% | | 1011 | -0.29 | 82% | | 1051 | 0.36 | 128% |
| 972 | 0.79 | 172% | | 1012 | -0.38 | 77% | | 1052 | -0.30 | 81% |
| 973 | -0.15 | 90% | | 1013 | -0.39 | 76% | | 1053 | -0.33 | 80% |
| 974 | 0.14 | 110% | | 1014 | 0.45 | 136% | | 1054 | -0.52 | 70% |
| 975 | 0.59 | 150% | | 1015 | -0.50 | 71% | | 1055 | 0.67 | 159% |
| 976 | 0.64 | 155% | | 1016 | -0.46 | 73% | | 1056 | -0.59 | 66% |
| 977 | 0.44 | 136% | | 1017 | 0.48 | 139% | | 1057 | -0.27 | 83% |
| 978 | 0.21 | 116% | | 1018 | 0.39 | 131% | | 1058 | -0.41 | 75% |
| 979 | 0.37 | 129% | | 1019 | -0.18 | 88% | | 1059 | 0.45 | 137% |
| 980 | 0.25 | 119% | | 1020 | 0.04 | 103% | | 1060 | -0.49 | 71% |
| 981 | -0.36 | 78% | | 1021 | 0.35 | 128% | | 1061 | 0.55 | 147% |
| 982 | 0.45 | 137% | | 1022 | 0.43 | 135% | | 1062 | -0.39 | 76% |
| 983 | 0.58 | 149% | | 1023 | -0.42 | 75% | | 1063 | -0.42 | 75% |
| 984 | -0.27 | 83% | | 1024 | -0.35 | 79% | | 1064 | -0.37 | 77% |
| 985 | -0.14 | 90% | | 1025 | 0.81 | 176% | | 1065 | 0.71 | 163% |
| 986 | -0.57 | 67% | | 1026 | -0.17 | 89% | | 1066 | 0.23 | 117% |
| 987 | 0.83 | 178% | | 1027 | -0.21 | 87% | | 1067 | 0.62 | 154% |
| 988 | 0.41 | 133% | | 1028 | 0.63 | 155% | | 1068 | -0.13 | 91% |
| 989 | 0.44 | 135% | | 1029 | 0.40 | 132% | | 1069 | -0.45 | 73% |
| 990 | 0.27 | 121% | | 1030 | 0.53 | 144% | | 1070 | -0.24 | 85% |
| 991 | 0.36 | 128% | | 1031 | 0.28 | 121% | | 1071 | 0.31 | 124% |
| 992 | 0.52 | 144% | | 1032 | 1.06 | 208% | | 1072 | 0.34 | 127% |
| 993 | 1.15 | 222% | | 1033 | 0.31 | 124% | | 1073 | 0.48 | 140% |
| 994 | -0.29 | 82% | | 1034 | 0.38 | 130% | | 1074 | -0.23 | 85% |
| 995 | -0.18 | 88% | | 1035 | 0.68 | 160% | | 1075 | 0.60 | 152% |
| 996 | 0.66 | 158% | | 1036 | -0.30 | 81% | | 1076 | 0.32 | 124% |
| 997 | 0.52 | 143% | | 1037 | -0.27 | 83% | | 1077 | -0.37 | 77% |
| 998 | 0.73 | 166% | | 1038 | -0.71 | 61% | | 1078 | -0.51 | 70% |
| 999 | -0.21 | 86% | | 1039 | 0.41 | 133% | | 1079 | 0.28 | 122% |
| 1000 | 0.23 | 117% | | 1040 | 0.17 | 112% | | 1080 | 0.51 | 142% |
| 1081 | 0.50 | 141% | | 1121 | 2.67 | 638% | | 1161 | 0.46 | 138% |
| 1082 | 0.32 | 125% | | 1122 | 0.36 | 129% | | 1162 | 0.21 | 116% |
| 1083 | 0.68 | 160% | | 1123 | 0.18 | 113% | | 1163 | -0.28 | 82% |
| 1084 | 1.64 | 311% | | 1124 | 1.43 | 269% | | 1164 | 0.27 | 121% |
| 1085 | 1.95 | 387% | | 1125 | -0.22 | 86% | | 1165 | -0.37 | 78% |
| 1086 | 0.37 | 129% | | 1126 | 0.46 | 138% | | 1166 | 0.66 | 159% |
| 1087 | 0.19 | 114% | | 1127 | -0.38 | 77% | | 1167 | 0.36 | 129% |
| 1088 | -0.32 | 80% | | 1128 | 0.63 | 155% | | 1168 | 0.34 | 127% |
| 1089 | 0.41 | 133% | | 1129 | 0.46 | 138% | | 1169 | 0.70 | 163% |
| 1090 | 0.45 | 137% | | 1130 | -0.51 | 70% | | 1170 | 0.37 | 130% |
| 1091 | 0.88 | 184% | | 1131 | -0.39 | 76% | | 1171 | 0.36 | 128% |
| 1092 | -0.43 | 74% | | 1132 | 0.21 | 116% | | 1172 | -0.39 | 77% |
| 1093 | 0.27 | 121% | | 1133 | -0.58 | 67% | | 1173 | -0.64 | 64% |
| 1094 | -0.30 | 81% | | 1134 | 0.31 | 124% | | 1174 | 0.51 | 142% |
| 1095 | 0.70 | 163% | | 1135 | 0.38 | 130% | | 1175 | 0.19 | 114% |
| 1096 | 0.39 | 131% | | 1136 | 0.28 | 121% | | 1176 | 0.43 | 135% |
| 1097 | -0.61 | 65% | | 1137 | 0.25 | 119% | | 1177 | 0.23 | 117% |
| 1098 | -0.38 | 77% | | 1138 | 0.50 | 141% | | 1178 | -0.53 | 69% |
| 1099 | 0.38 | 130% | | 1139 | 0.37 | 129% | | 1179 | -0.36 | 78% |
| 1100 | 0.35 | 127% | | 1140 | 0.80 | 174% | | 1180 | -0.36 | 78% |
| 1101 | 0.45 | 137% | | 1141 | -0.65 | 64% | | 1181 | -0.35 | 78% |
| 1102 | -0.23 | 85% | | 1142 | -0.62 | 65% | | 1182 | 0.48 | 139% |
| 1103 | -0.26 | 83% | | 1143 | -0.05 | 96% | | 1183 | -0.77 | 59% |
| 1104 | 0.46 | 137% | | 1144 | 0.31 | 124% | | 1184 | -0.31 | 81% |
| 1105 | 0.46 | 138% | | 1145 | -0.29 | 82% | | 1185 | 0.57 | 148% |
| 1106 | -0.46 | 73% | | 1146 | 0.27 | 121% | | 1186 | 0.29 | 123% |
| 1107 | -0.29 | 82% | | 1147 | -0.40 | 76% | | 1187 | 0.38 | 130% |
| 1108 | 0.36 | 128% | | 1148 | -0.26 | 83% | | 1188 | 0.33 | 126% |
| 1109 | 0.45 | 137% | | 1149 | -0.28 | 82% | | 1189 | 0.48 | 139% |
| 1110 | -0.17 | 89% | | 1150 | 0.30 | 123% | | 1190 | 0.32 | 125% |
| 1111 | 0.46 | 138% | | 1151 | 0.21 | 115% | | 1191 | -0.34 | 79% |
| 1112 | -0.44 | 73% | | 1152 | -0.33 | 80% | | 1192 | 0.39 | 131% |
| 1113 | 0.38 | 130% | | 1153 | -0.29 | 82% | | 1193 | 0.71 | 163% |
| 1114 | 0.37 | 130% | | 1154 | -0.59 | 67% | | 1194 | 0.77 | 170% |
| 1115 | -0.48 | 72% | | 1155 | 0.51 | 142% | | 1195 | 1.22 | 233% |
| 1116 | 0.62 | 154% | | 1156 | -0.43 | 74% | | 1196 | 0.62 | 153% |
| 1117 | 1.09 | 213% | | 1157 | 0.52 | 143% | | 1197 | -0.53 | 69% |
| 1118 | 0.23 | 117% | | 1158 | 0.28 | 121% | | 1198 | 0.35 | 127% |
| 1119 | 0.71 | 164% | | 1159 | 0.65 | 157% | | 1199 | -0.19 | 88% |
| 1120 | 0.37 | 129% | | 1160 | -0.16 | 89% | | 1200 | 0.31 | 124% |

| SEQ ID Nos for Examples 1-3 of length 300 | | | | SEQ ID Nos for Examples 1-3 of length 300 | | | | SEQ ID Nos for Examples 1-3 of length 300 | | |
|---|---|---|---|---|---|---|---|---|---|---|
| Example 1 | Example 2 | Example 3 | | Example 1 | Example 2 | Example 3 | | Example 1 | Example 2 | Example 3 |
| 1 | 5 | 4 | | 121 | 142 | 142 | | 244 | 278 | 304 |
| 5 | 6 | 5 | | 133 | 144 | 144 | | 247 | 283 | 305 |
| 7 | 14 | 6 | | 134 | 146 | 146 | | 256 | 286 | 306 |
| 9 | 15 | 7 | | 135 | 152 | 153 | | 260 | 287 | 317 |
| 10 | 18 | 9 | | 142 | 153 | 157 | | 263 | 289 | 318 |
| 13 | 21 | 10 | | 144 | 157 | 166 | | 275 | 294 | 320 |
| 14 | 23 | 14 | | 146 | 166 | 167 | | 278 | 296 | 324 |
| 17 | 29 | 20 | | 150 | 167 | 171 | | 280 | 304 | 327 |
| 18 | 30 | 21 | | 153 | 168 | 172 | | 286 | 306 | 335 |
| 21 | 36 | 24 | | 154 | 171 | 181 | | 300 | 310 | 336 |
| 24 | 38 | 25 | | 157 | 176 | 184 | | 301 | 318 | 340 |
| 28 | 47 | 29 | | 171 | 179 | 185 | | 305 | 324 | 347 |
| 30 | 51 | 32 | | 172 | 181 | 188 | | 307 | 330 | 349 |
| 32 | 54 | 36 | | 177 | 184 | 193 | | 309 | 348 | 350 |
| 37 | 57 | 38 | | 178 | 185 | 195 | | 320 | 349 | 359 |
| 38 | 64 | 40 | | 182 | 188 | 198 | | 322 | 350 | 362 |
| 39 | 66 | 48 | | 183 | 190 | 200 | | 324 | 354 | 363 |
| 40 | 68 | 51 | | 184 | 191 | 214 | | 331 | 358 | 365 |
| 51 | 74 | 57 | | 185 | 192 | 215 | | 335 | 362 | 369 |
| 52 | 77 | 66 | | 186 | 193 | 224 | | 336 | 363 | 373 |
| 57 | 78 | 70 | | 188 | 194 | 235 | | 340 | 368 | 377 |
| 67 | 81 | 74 | | 189 | 195 | 238 | | 347 | 371 | 381 |
| 68 | 83 | 77 | | 198 | 206 | 242 | | 349 | 375 | 383 |
| 69 | 87 | 81 | | 199 | 210 | 251 | | 350 | 377 | 386 |
| 70 | 90 | 87 | | 200 | 215 | 252 | | 359 | 382 | 387 |
| 71 | 100 | 95 | | 201 | 219 | 256 | | 360 | 386 | 390 |
| 72 | 103 | 103 | | 202 | 224 | 260 | | 363 | 389 | 392 |
| 80 | 108 | 104 | | 207 | 225 | 268 | | 374 | 392 | 393 |
| 82 | 109 | 108 | | 214 | 235 | 271 | | 377 | 398 | 402 |
| 87 | 112 | 112 | | 215 | 248 | 275 | | 381 | 400 | 406 |
| 88 | 115 | 115 | | 218 | 249 | 280 | | 383 | 402 | 414 |
| 103 | 126 | 117 | | 221 | 251 | 282 | | 385 | 414 | 416 |
| 109 | 133 | 121 | | 225 | 252 | 286 | | 386 | 417 | 417 |
| 113 | 135 | 126 | | 228 | 256 | 289 | | 387 | 425 | 427 |
| 115 | 137 | 133 | | 233 | 268 | 294 | | 390 | 427 | 431 |
| 117 | 139 | 137 | | 242 | 275 | 296 | | 399 | 431 | 432 |
| 402 | 432 | 433 | | 524 | 570 | 620 | | 714 | 714 | 749 |
| 403 | 437 | 451 | | 529 | 571 | 621 | | 715 | 715 | 761 |
| 404 | 439 | 452 | | 530 | 572 | 624 | | 717 | 717 | 764 |
| 405 | 440 | 453 | | 534 | 581 | 625 | | 720 | 721 | 772 |
| 409 | 442 | 457 | | 540 | 589 | 626 | | 725 | 722 | 774 |
| 413 | 451 | 458 | | 541 | 601 | 630 | | 726 | 726 | 776 |
| 417 | 452 | 459 | | 566 | 603 | 632 | | 731 | 728 | 785 |
| 418 | 453 | 461 | | 570 | 607 | 634 | | 739 | 740 | 791 |
| 431 | 459 | 463 | | 571 | 613 | 642 | | 741 | 743 | 794 |
| 432 | 463 | 470 | | 586 | 616 | 644 | | 745 | 749 | 802 |
| 433 | 470 | 471 | | 608 | 621 | 652 | | 755 | 751 | 806 |
| 444 | 476 | 476 | | 619 | 624 | 655 | | 763 | 756 | 808 |
| 446 | 480 | 483 | | 621 | 625 | 657 | | 764 | 761 | 809 |
| 449 | 483 | 485 | | 623 | 627 | 666 | | 774 | 785 | 810 |
| 451 | 486 | 486 | | 624 | 632 | 671 | | 781 | 787 | 813 |
| 453 | 488 | 492 | | 626 | 640 | 672 | | 784 | 793 | 817 |
| 458 | 499 | 499 | | 628 | 642 | 673 | | 794 | 802 | 825 |
| 459 | 500 | 500 | | 632 | 644 | 675 | | 799 | 805 | 826 |
| 461 | 504 | 505 | | 634 | 655 | 676 | | 802 | 807 | 827 |
| 470 | 508 | 507 | | 635 | 656 | 686 | | 804 | 808 | 828 |
| 471 | 509 | 522 | | 637 | 657 | 690 | | 813 | 809 | 834 |
| 472 | 511 | 528 | | 641 | 668 | 694 | | 817 | 819 | 835 |
| 475 | 518 | 529 | | 646 | 669 | 695 | | 825 | 825 | 837 |
| 476 | 527 | 530 | | 657 | 672 | 697 | | 826 | 826 | 838 |
| 478 | 529 | 533 | | 669 | 673 | 698 | | 827 | 827 | 839 |
| 482 | 533 | 543 | | 670 | 679 | 706 | | 834 | 828 | 846 |
| 483 | 534 | 547 | | 672 | 683 | 713 | | 835 | 836 | 849 |
| 485 | 540 | 550 | | 675 | 686 | 714 | | 837 | 837 | 852 |
| 486 | 541 | 554 | | 676 | 690 | 717 | | 839 | 839 | 855 |
| 492 | 543 | 566 | | 679 | 693 | 722 | | 848 | 840 | 857 |
| 499 | 550 | 568 | | 686 | 696 | 726 | | 849 | 843 | 858 |
| 500 | 554 | 572 | | 697 | 697 | 728 | | 850 | 847 | 866 |
| 501 | 559 | 589 | | 699 | 698 | 732 | | 857 | 849 | 867 |
| 502 | 562 | 603 | | 704 | 706 | 739 | | 858 | 851 | 868 |
| 503 | 563 | 604 | | 706 | 709 | 740 | | 859 | 853 | 873 |
| 511 | 564 | 616 | | 710 | 712 | 743 | | 864 | 864 | 874 |
| 866 | 866 | 877 | | 1011 | 1022 | 1013 | | 1133 | 1147 | 1134 |
| 869 | 867 | 890 | | 1013 | 1023 | 1014 | | 1134 | 1152 | 1138 |
| 873 | 868 | 892 | | 1021 | 1025 | 1021 | | 1138 | 1161 | 1142 |
| 874 | 873 | 893 | | 1025 | 1029 | 1022 | | 1139 | 1168 | 1158 |
| 879 | 874 | 895 | | 1028 | 1030 | 1023 | | 1140 | 1169 | 1174 |
| 892 | 875 | 896 | | 1029 | 1032 | 1025 | | 1158 | 1174 | 1176 |
| 893 | 877 | 899 | | 1032 | 1033 | 1028 | | 1175 | 1181 | 1181 |
| 895 | 880 | 907 | | 1033 | 1037 | 1029 | | 1177 | 1185 | 1183 |
| 896 | 889 | 909 | | 1039 | 1038 | 1030 | | 1183 | 1186 | 1186 |
| 899 | 893 | 911 | | 1041 | 1042 | 1032 | | 1187 | 1187 | 1187 |
| 900 | 895 | 914 | | 1042 | 1048 | 1037 | | 1190 | 1188 | 1193 |
| 903 | 896 | 916 | | 1047 | 1051 | 1039 | | 1193 | 1195 | 1195 |
| 909 | 909 | 919 | | 1048 | 1052 | 1042 | | Table 2 (above) | | |
| 911 | 911 | 920 | | 1052 | 1061 | 1052 | | | | |
| 914 | 912 | 921 | | 1060 | 1065 | 1061 | | | | |
| 923 | 914 | 923 | | 1063 | 1066 | 1063 | | | | |
| 931 | 916 | 926 | | 1065 | 1073 | 1065 | | | | |
| 934 | 931 | 930 | | 1072 | 1075 | 1070 | | | | |
| 936 | 937 | 937 | | 1076 | 1078 | 1071 | | | | |
| 937 | 941 | 941 | | 1078 | 1081 | 1073 | | | | |
| 938 | 942 | 943 | | 1081 | 1083 | 1076 | | | | |
| 941 | 943 | 949 | | 1083 | 1086 | 1078 | | | | |
| 946 | 948 | 950 | | 1085 | 1089 | 1081 | | | | |
| 947 | 949 | 954 | | 1087 | 1090 | 1087 | | | | |
| 959 | 954 | 959 | | 1088 | 1092 | 1091 | | | | |
| 960 | 960 | 960 | | 1096 | 1095 | 1092 | | | | |
| 962 | 970 | 963 | | 1098 | 1097 | 1095 | | | | |
| 965 | 971 | 970 | | 1100 | 1098 | 1100 | | | | |
| 970 | 976 | 976 | | 1101 | 1105 | 1104 | | | | |
| 973 | 977 | 977 | | 1105 | 1109 | 1105 | | | | |
| 974 | 981 | 991 | | 1106 | 1110 | 1107 | | | | |
| 976 | 988 | 992 | | 1107 | 1114 | 1111 | | | | |
| 978 | 998 | 996 | | 1111 | 1128 | 1117 | | | | |
| 985 | 1010 | 998 | | 1119 | 1134 | 1120 | | | | |
| 988 | 1013 | 1001 | | 1123 | 1138 | 1124 | | | | |
| 1001 | 1014 | 1010 | | 1124 | 1146 | 1128 | | | | |

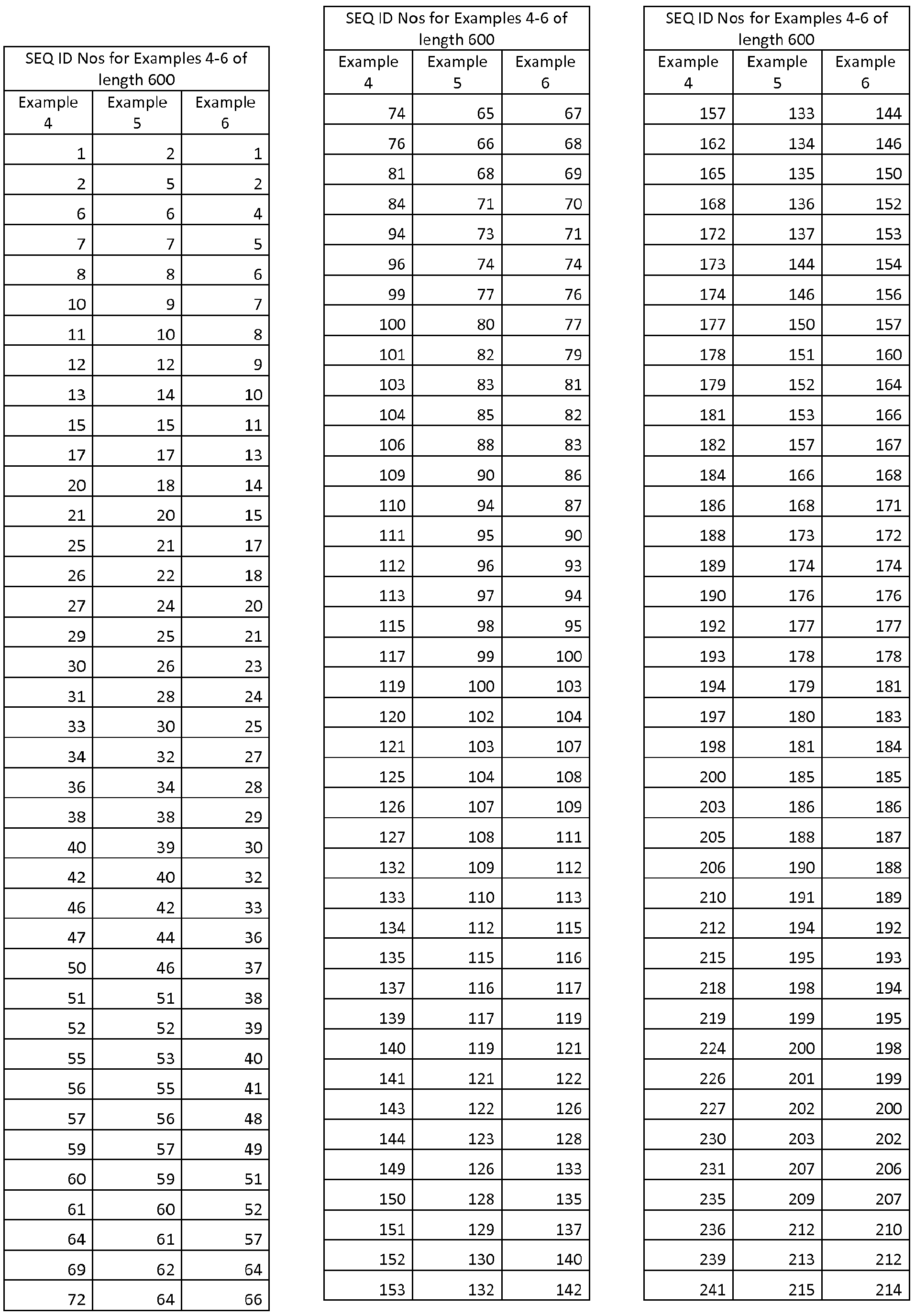

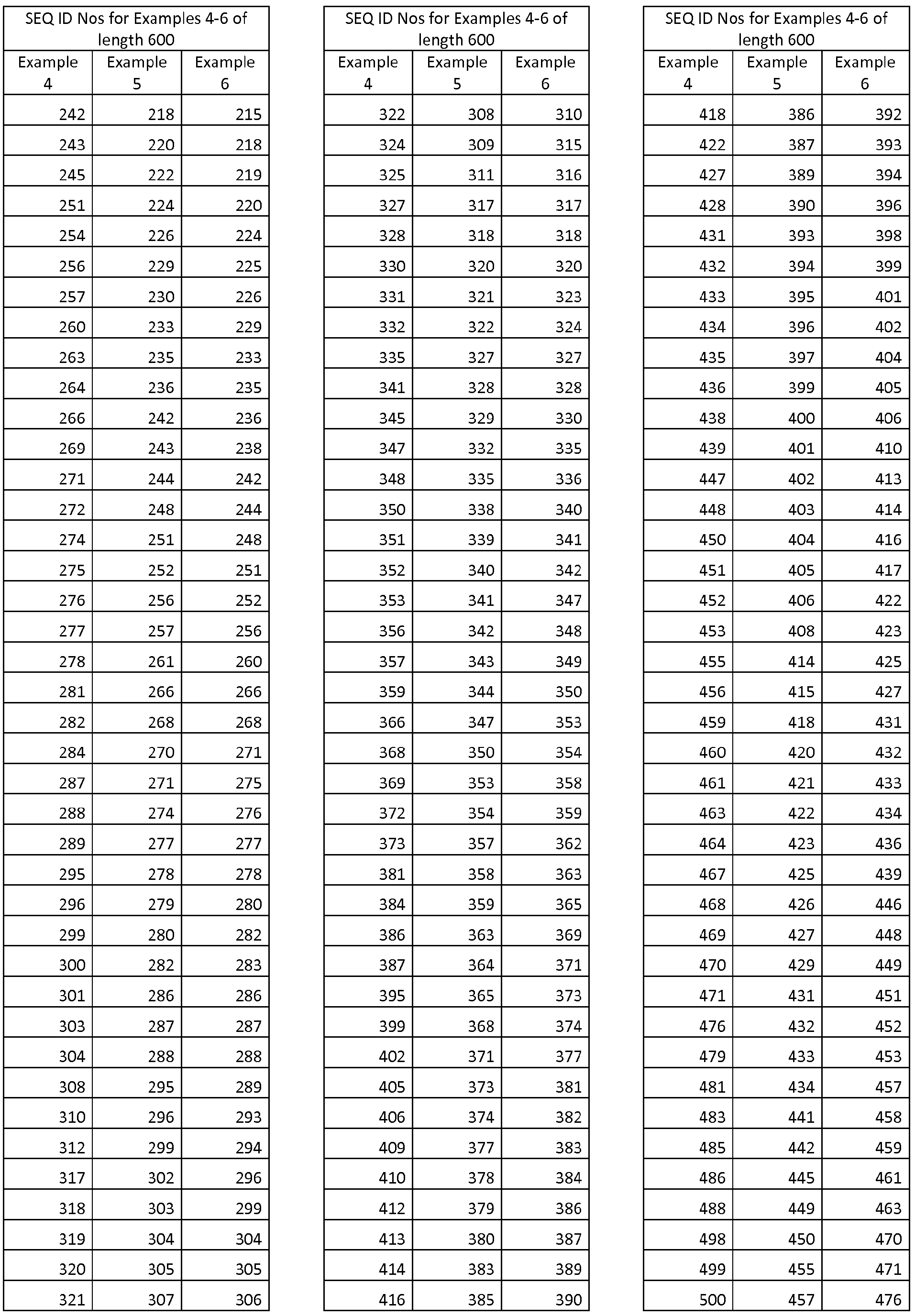

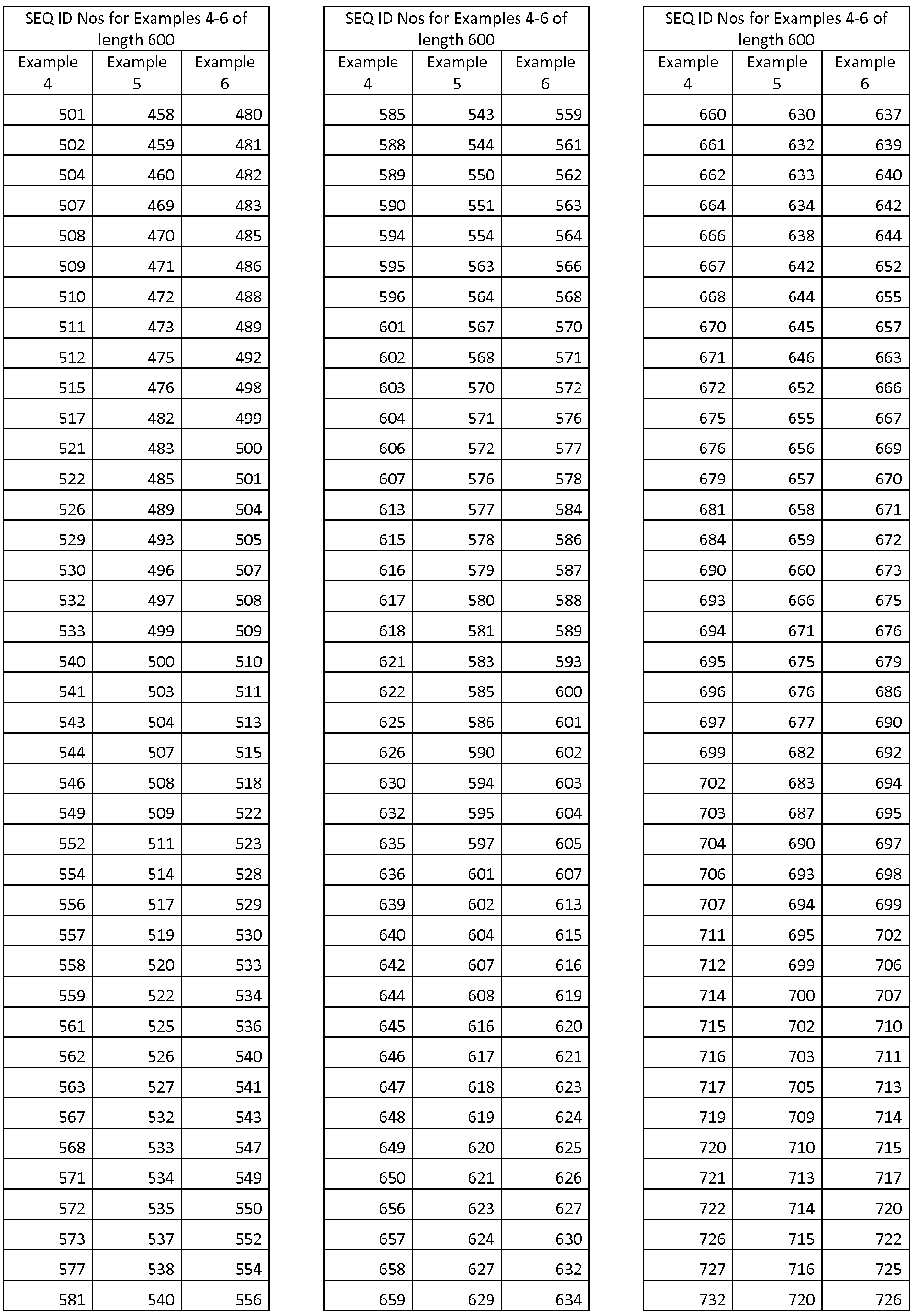

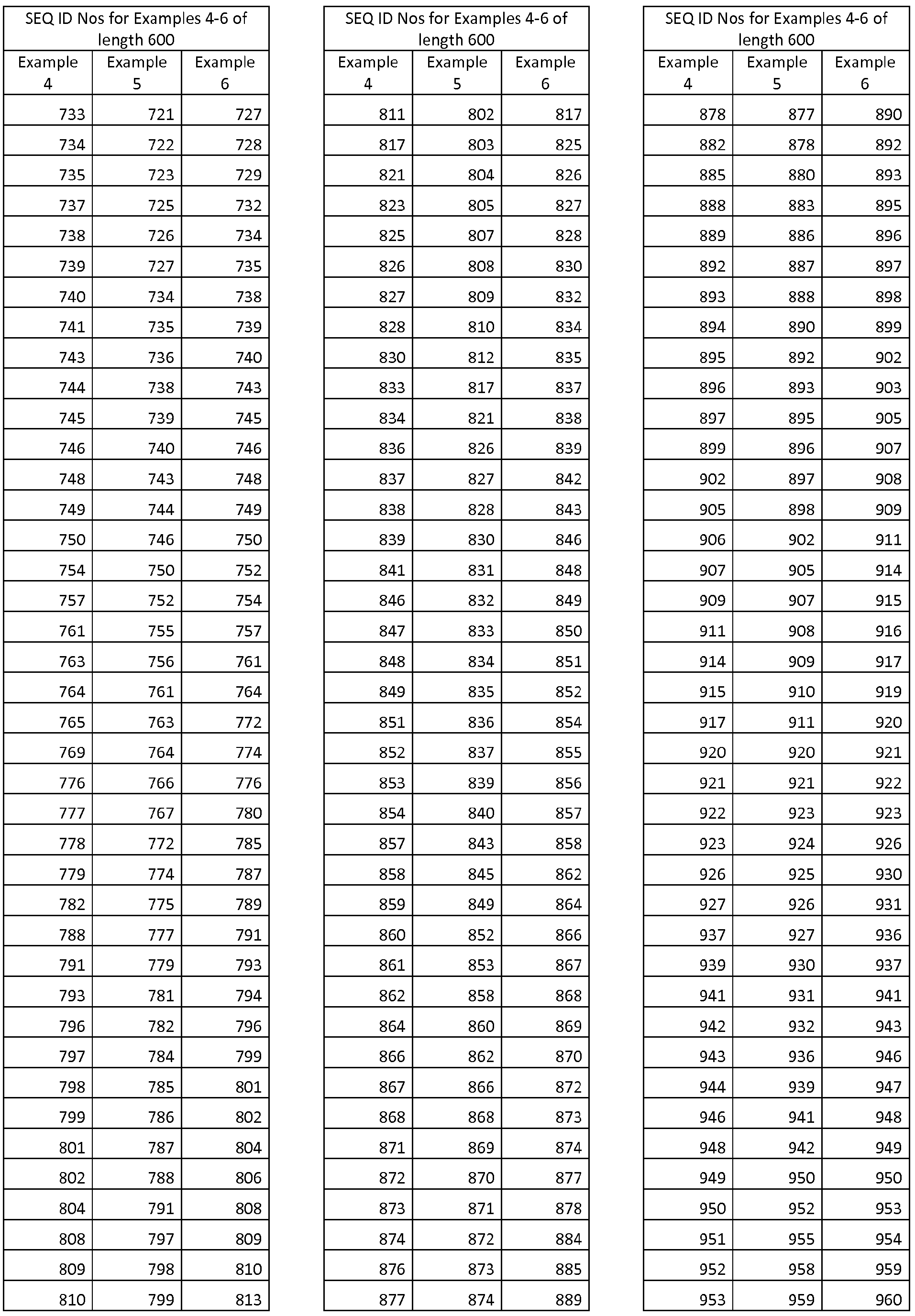

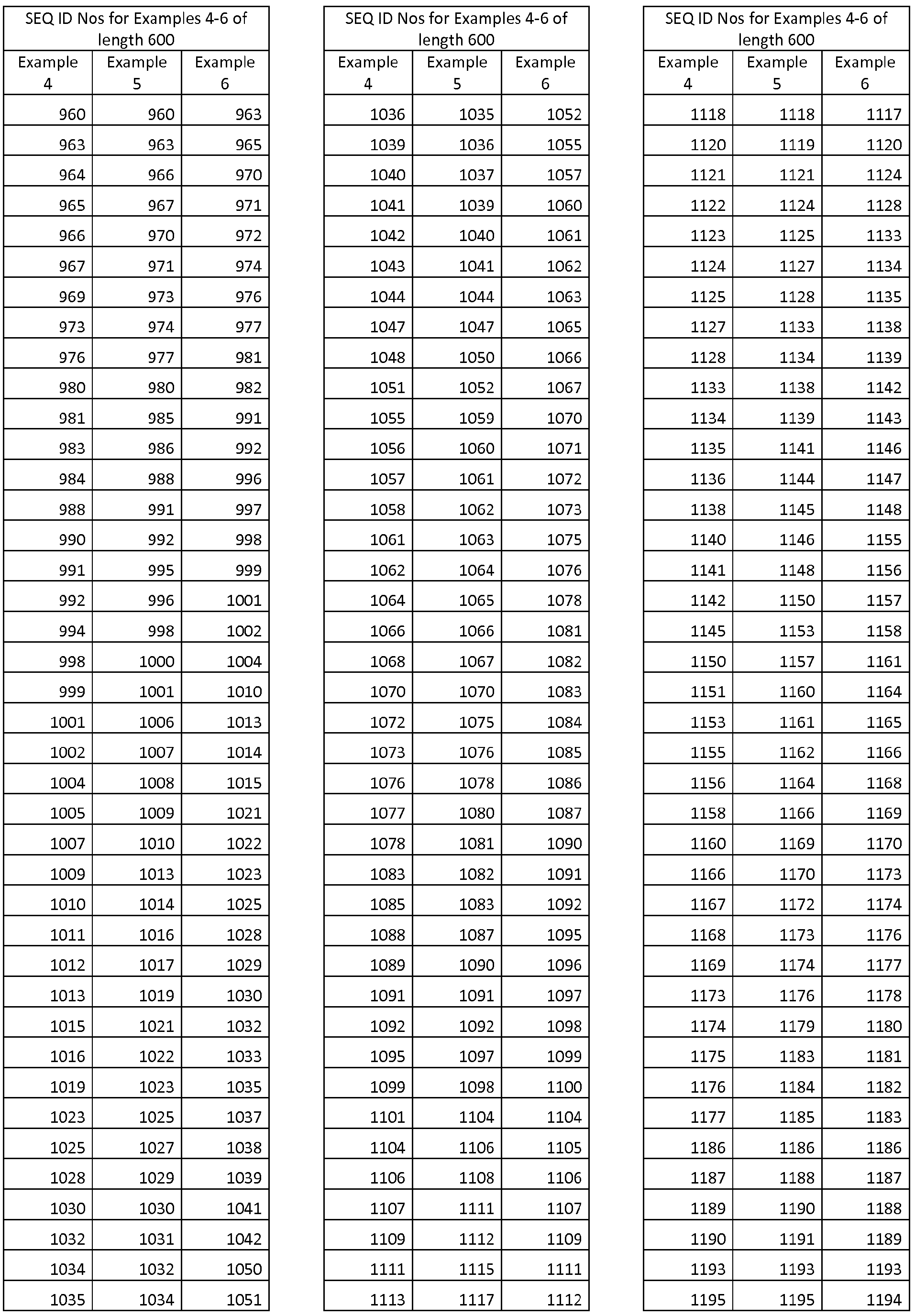

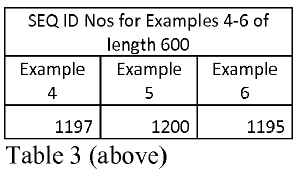

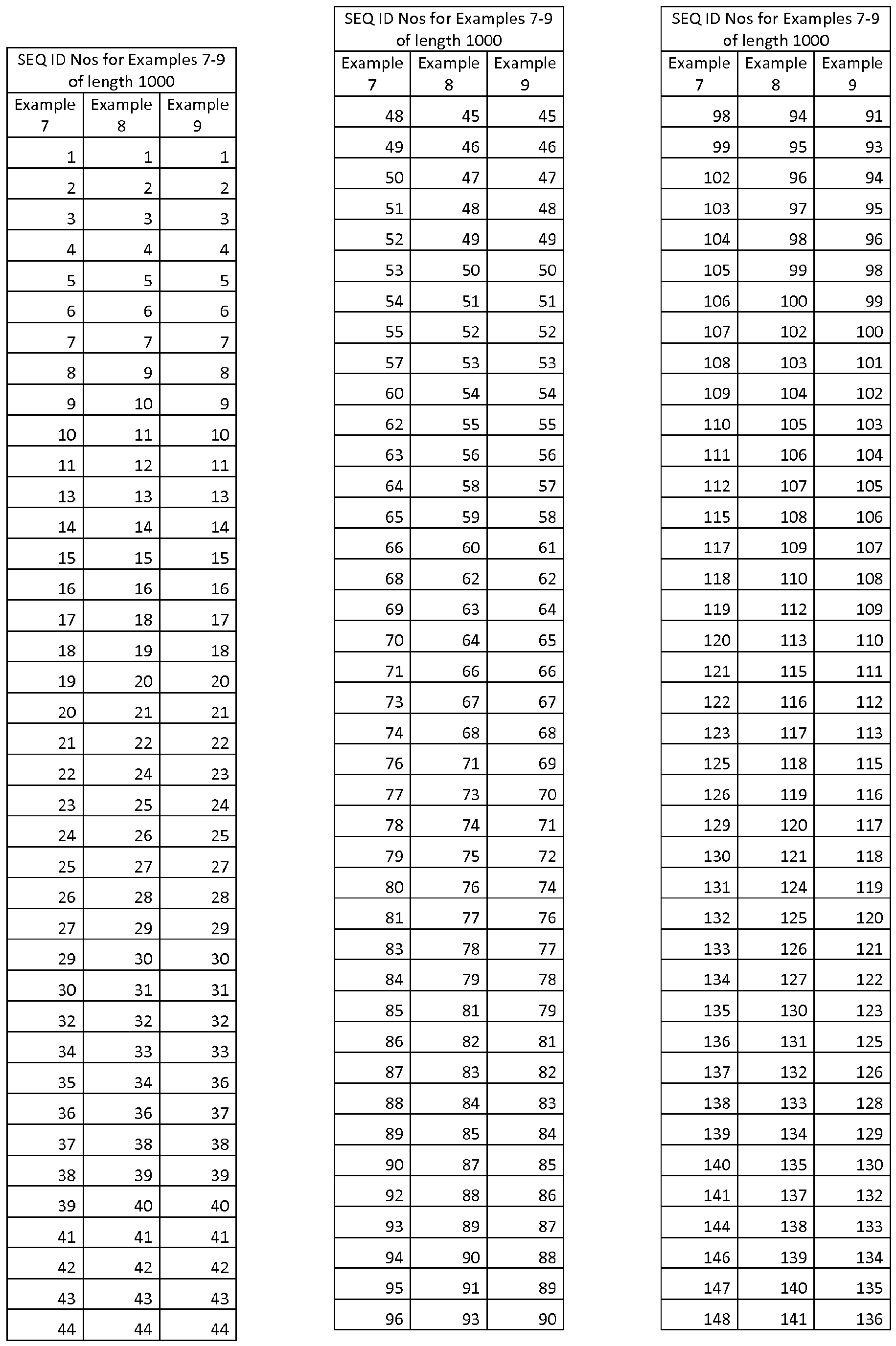

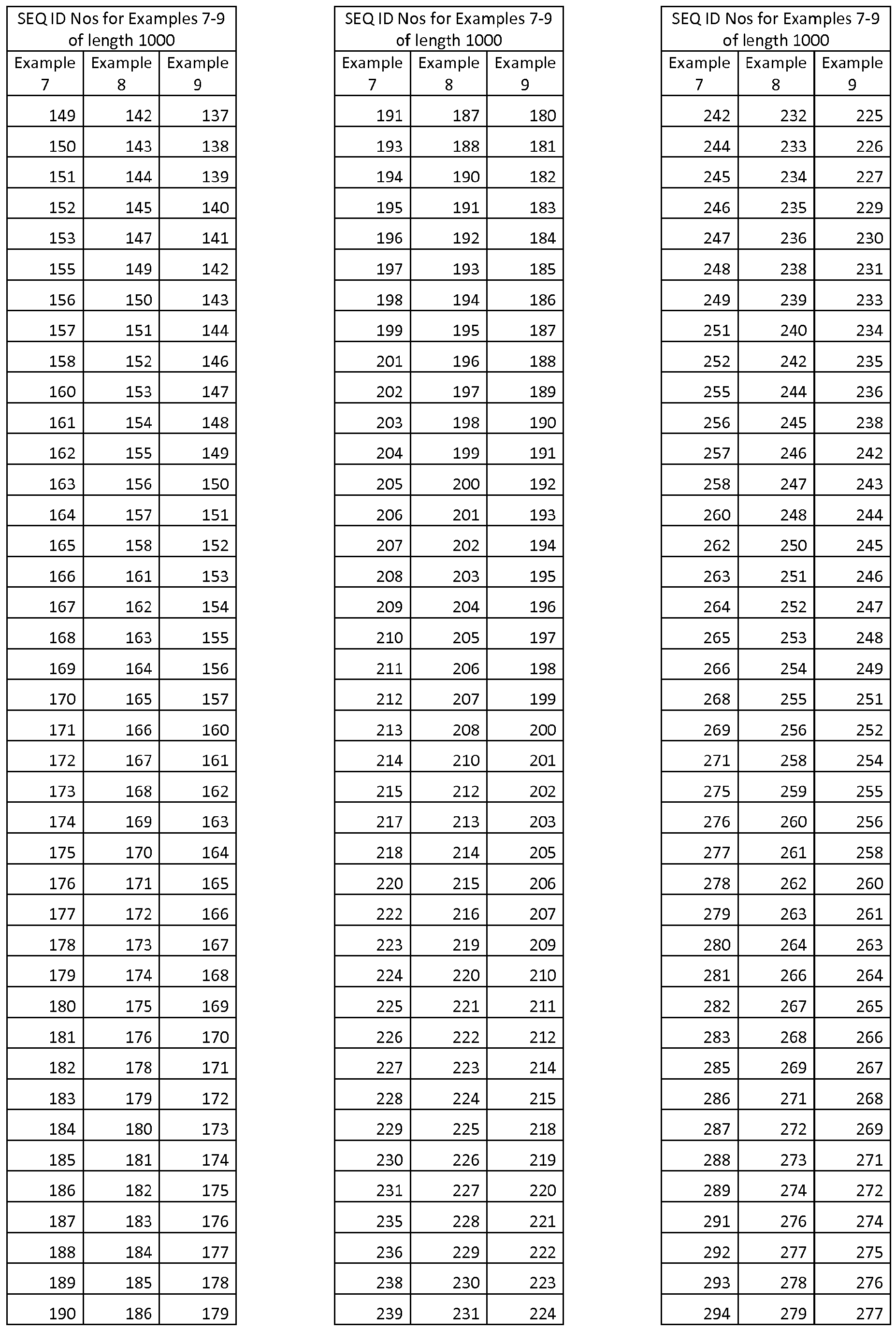

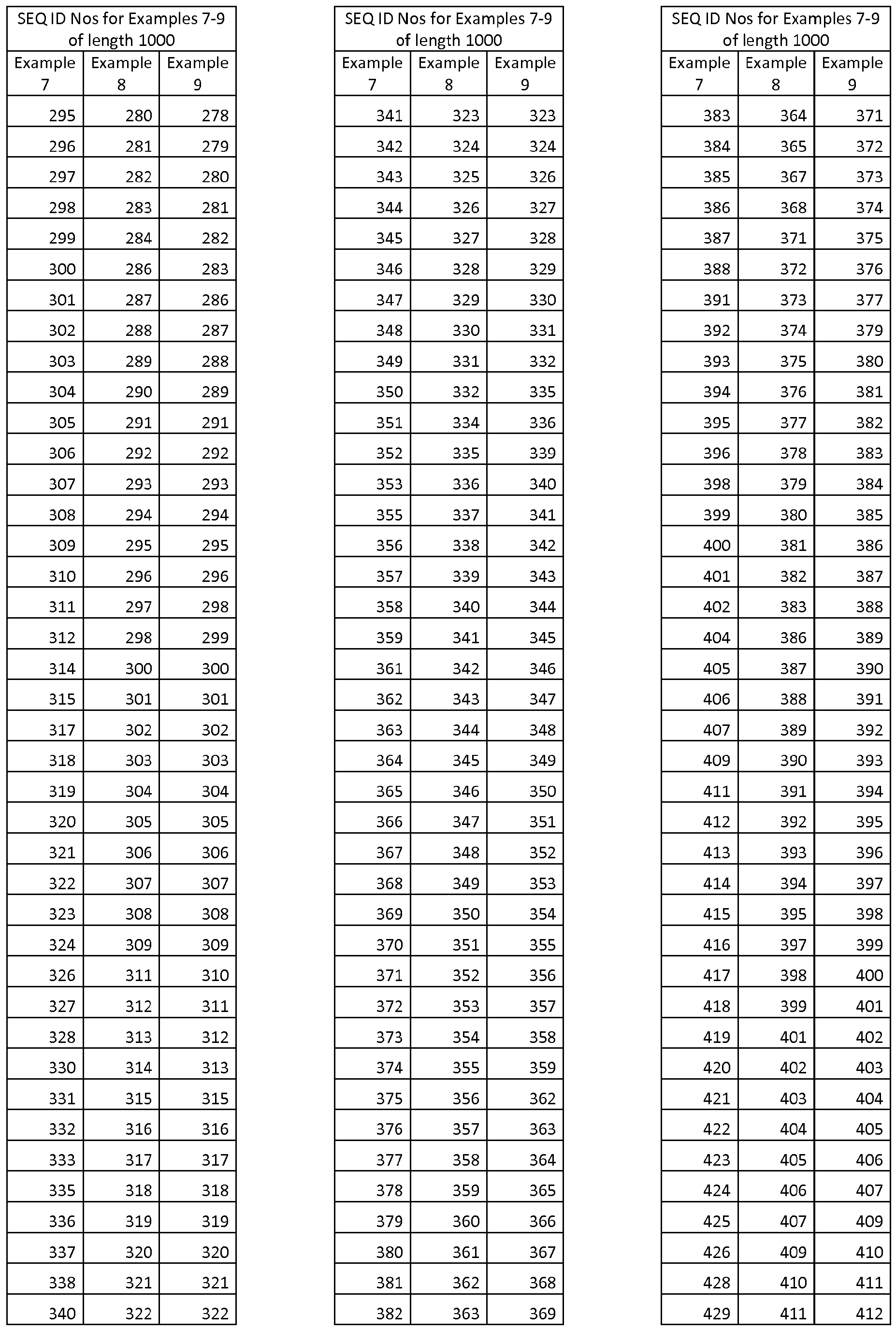

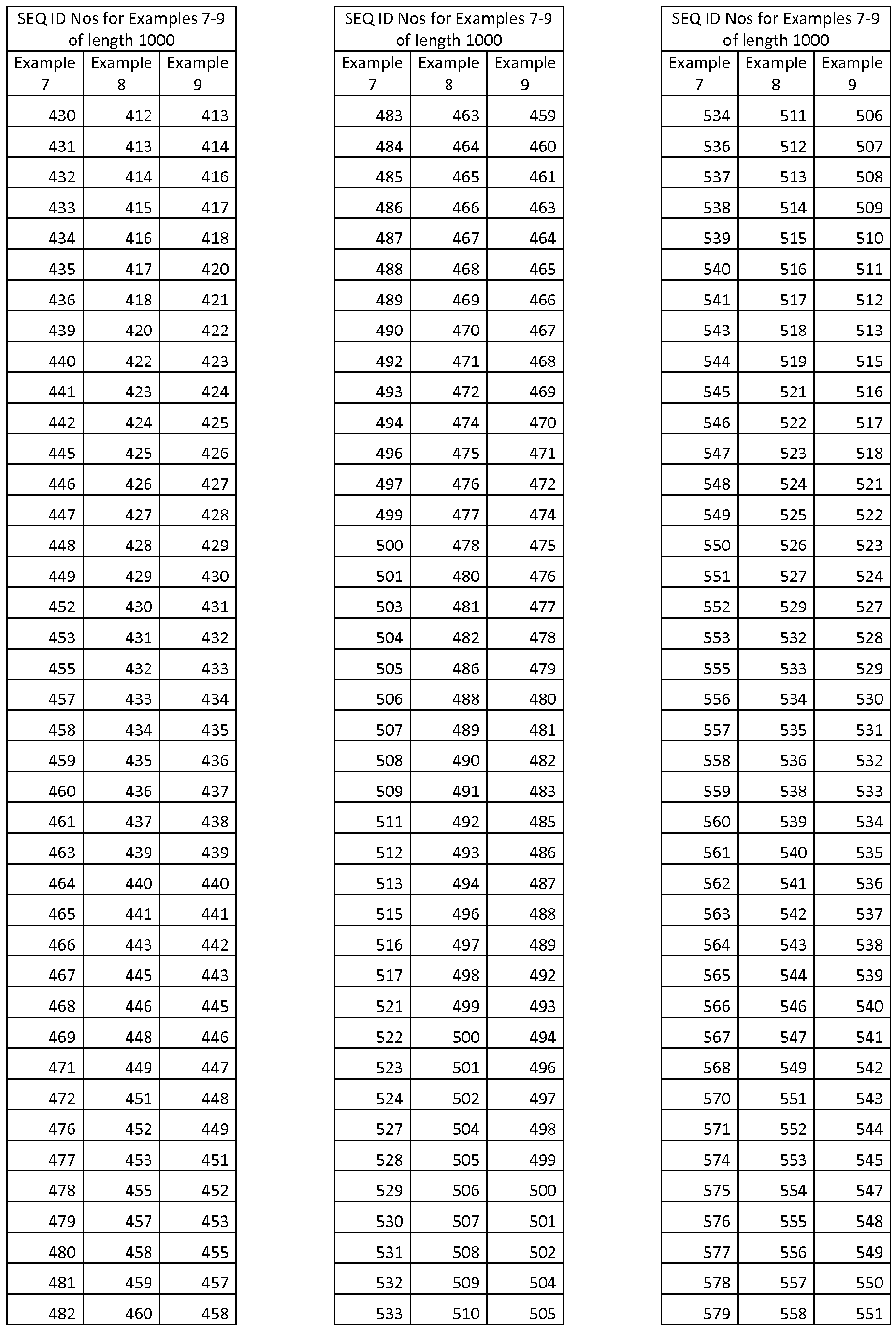

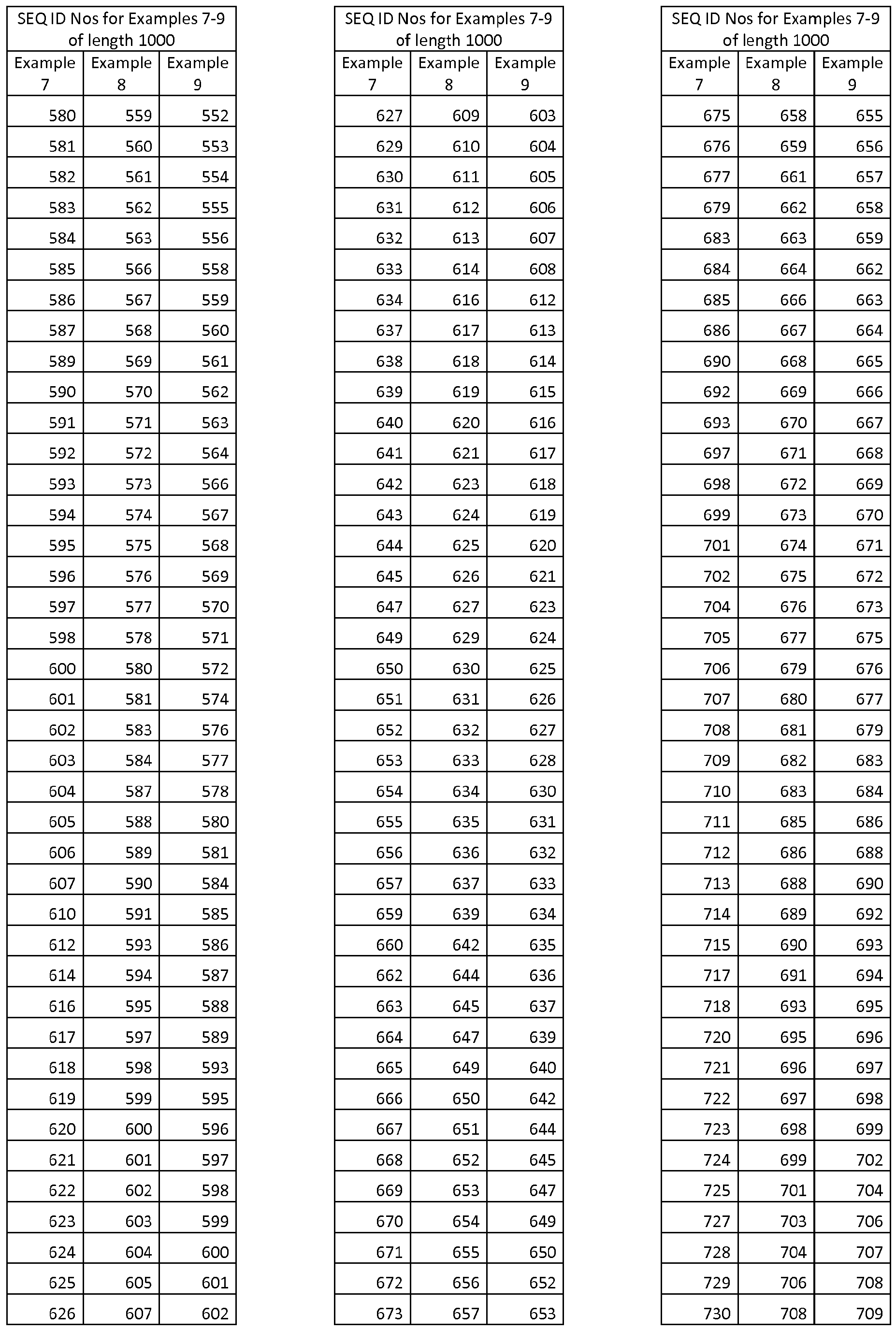

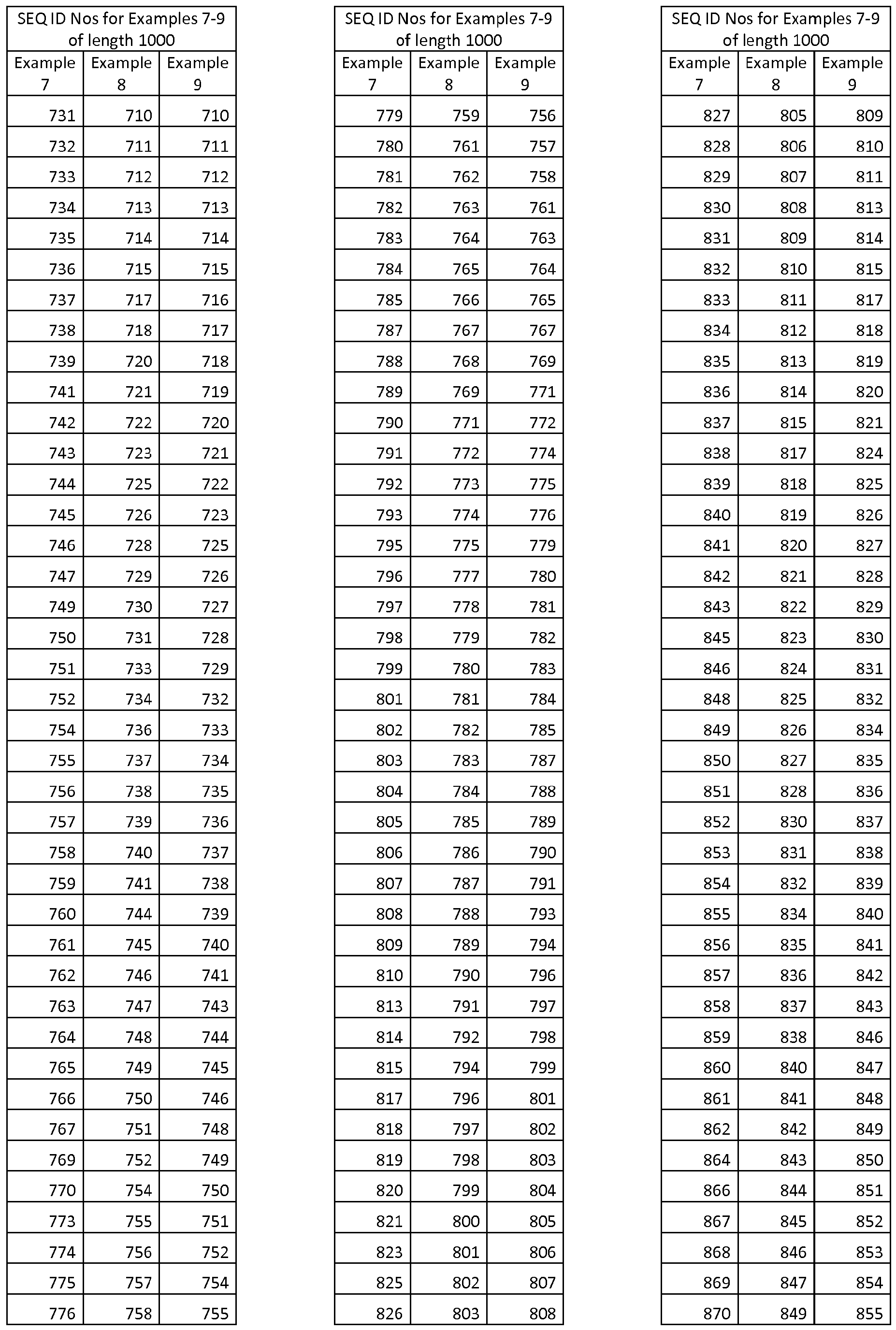

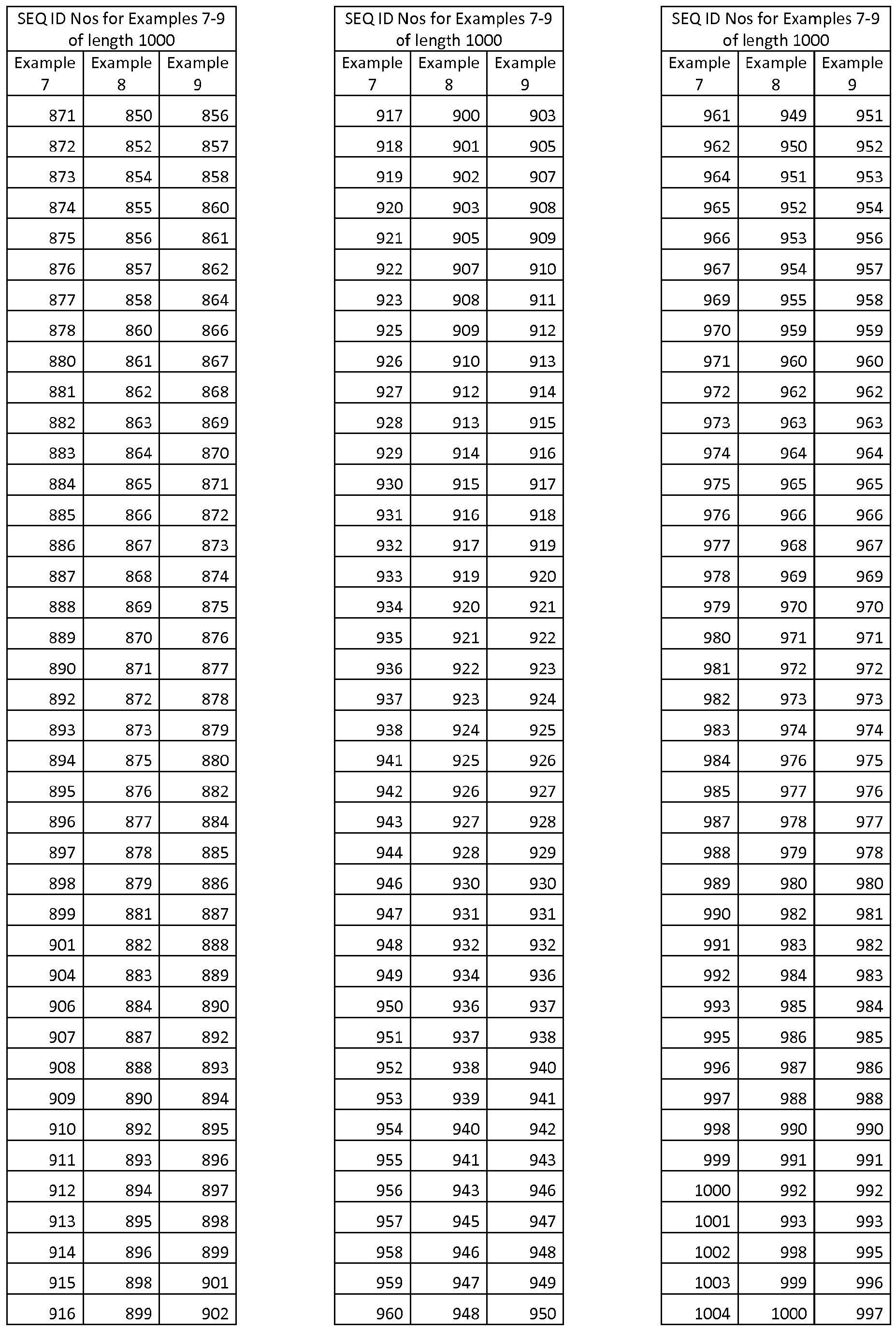

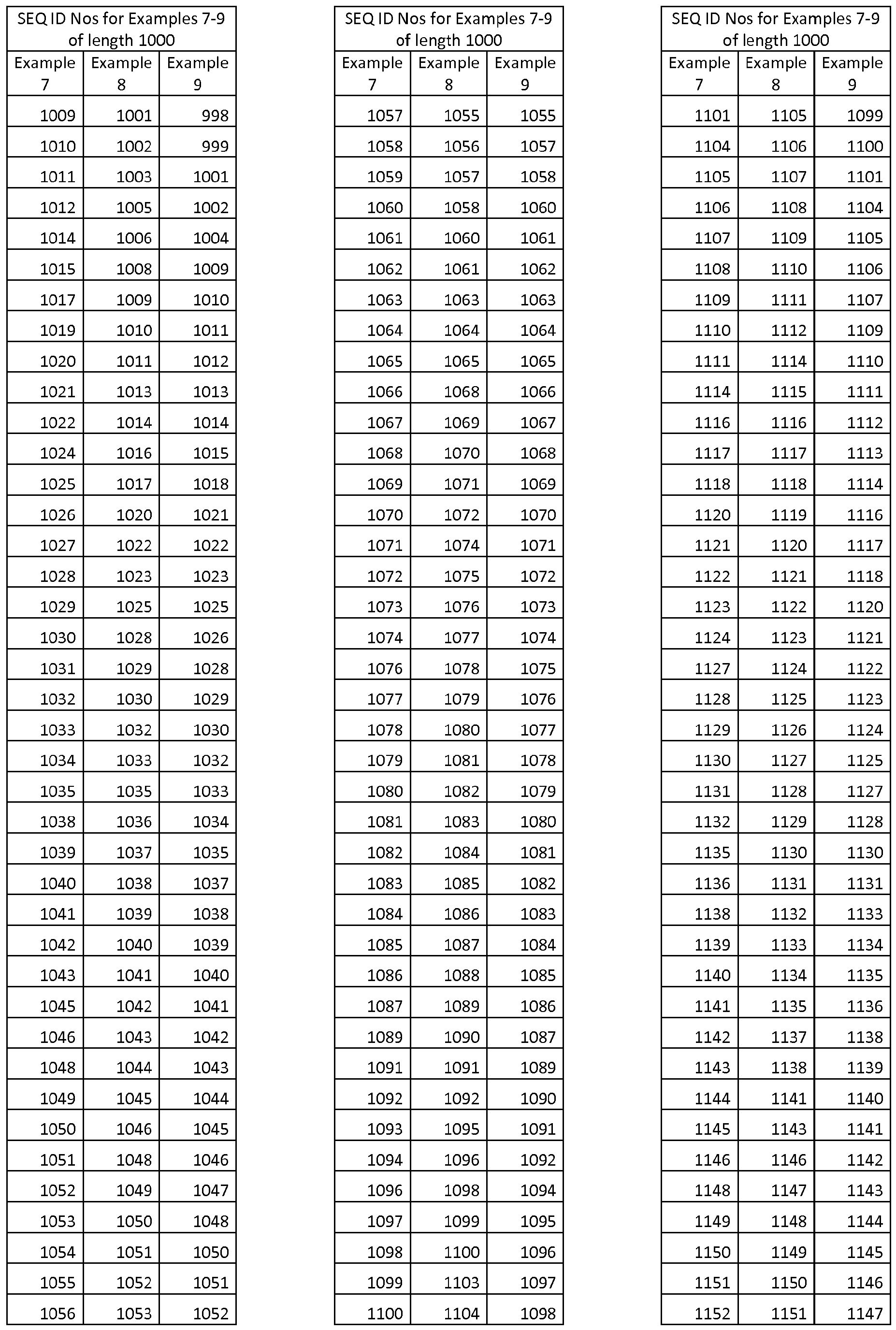

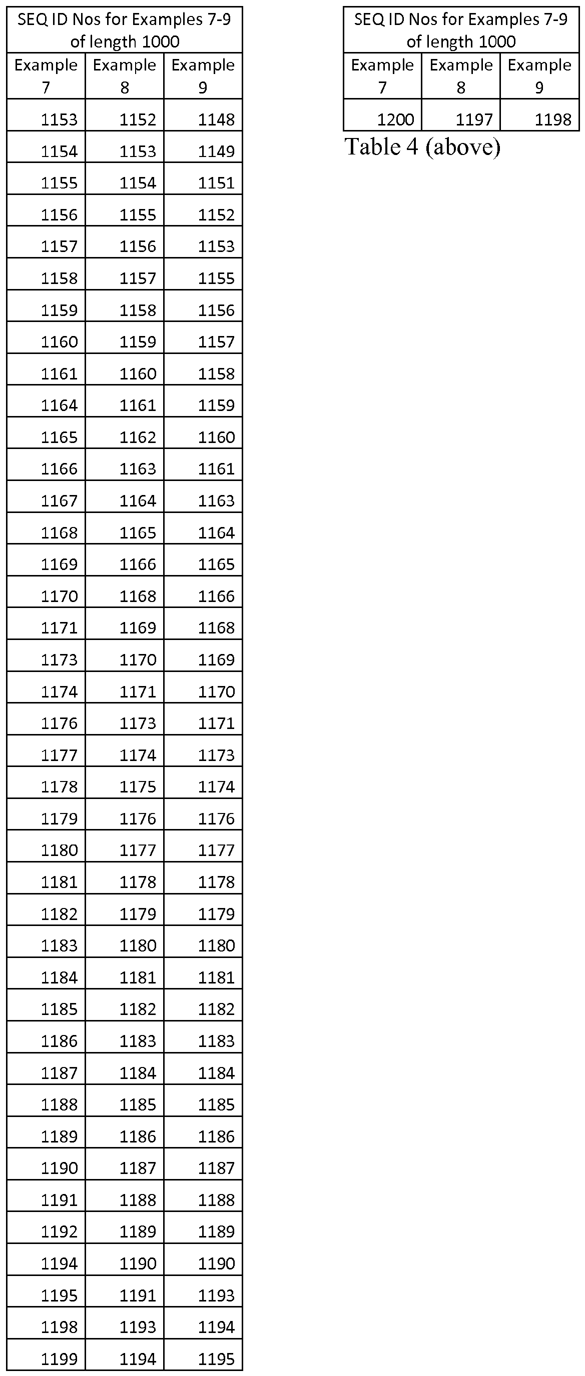

## Claims

1. A method for the detection of colorectal cancer in a human subject based on RNA from a blood sample obtained from said subject, comprising:
- measuring the abundance of at least 300 RNAs in the sample, that are chosen from the RNAs listed in table 1, and
- concluding based on the measured abundance whether the subject has colorectal cancer.

2. The method of claim 1, wherein the abundance of at least 600 RNAs, of at least 1000 RNAs, of at least 1200 RNAs that are chosen from the RNAs listed in table 1 is measured.

3. The method of claim 1, wherein the abundance of 300 RNAs as listed in table 2 is measured.

4. The method of claim 2, wherein the abundance of 600 RNAs as listed in table 3 is measured.

5. The method of claim 2, wherein the abundance of 1000 RNAs as listed in table 4 is measured.

6. The method of claims 1 to 5, wherein the measuring of RNA abundance is performed using a microarray, a real-time polymerase chain reaction or sequencing.

7. The method of claims 1 to 6, wherein the decision whether the subject has colorectal cancer comprises the step of training a classification algorithm on a training set of cases and controls, and applying it to measured RNA abundance.

8. The method of claims 1 to 7, wherein the classification method is a random forest method, a support vector machine (SVM), or a K-nearest neighbor method (K-NN), such as a 3-nearest neighbor method (3-NN).

9. The method of claims 1 to 8, wherein the RNA is mRNA, cDNA, micro RNA, small nuclear RNA, unspliced RNA, or its fragments.

10. Use of a method of claims 1 to 9 for detection of colorectal cancer in a human subject based on RNA from a blood sample.

11. A microarray, comprising a solid support and a set of oligonucleotide probes, the set containing from 300 to about 100,000 probes, and including at least 300 probes selected from table 1 or 2.

12. Use of a microarray for detection of colorectal cancer in a human subject based on RNA from a blood sample, comprising measuring the abundance of at least 300 RNAs listed in table 1, wherein the microarray comprises at least 1 probe for measuring the abundance of each of at least 300 RNAs.

13. A kit for the detection of colorectal cancer in a human subject based on RNA obtained from a blood sample, comprising means for measuring the abundance of at least 300 RNAs that are chosen from the RNAs listed in table 1, preferably comprising means for exclusively measuring the abundance of RNAs that are chosen from table 1.

14. Use of a kit of claim 13 for the detection of colorectal cancer in a human subject based on RNA from a blood sample, comprising means for measuring the abundance of at least 300 RNAs that are chosen from the RNAs listed in table 1, comprising
- measuring the abundance of at least 300 RNAs in a blood sample from a human subject, wherein the at least 300 RNAs are chosen from the RNAs listed in table 1, and
- concluding based on the measured abundance whether the subject has colorectal cancer.

15. A method for preparing an RNA expression profile that is indicative of the presence or absence of colorectal cancer in a subject, comprising:
- isolating RNA from a blood sample obtained from the subject, and
- determining the abundance of from 300 to about 3000 RNAs, including at least 300 RNAs selected from table 1.
